# EUROPEAN PATENT SPECIFICATION

(11) **EP 3 103 523 B1**
(45) Date of publication and mention of the grant of the patent: **29.10.2025**
(21) Application number: 15171784.0
(22) Date of filing: 12.06.2015
(51) Int. Cl.: A61Q 13/00, A61K 8/00, A61K 8/34, A61K 8/37, A61K 8/60, A61K 8/87, A61K 8/02

(54) **ABSORBENT ARTICLE COMPRISING FRAGRANCE COMPOSITION**
SAUGFÄHIGER ARTIKEL MIT DUFTSTOFFZUSAMMENSETZUNG
ARTICLE ABSORBANT COMPRENANT UNE COMPOSITION DE PARFUM

(43) Date of publication of application: 14.12.2016
(73) Proprietor: The Procter & Gamble Company, Cincinnati, OH 45202 (US)
(72) Inventor: Holland, Lynette Anne, Egham, Surrey TW209NW (GB); Bonnet, Christelle Marie Sandrine, 27120 Caillouet-Orgeville (FR); Pastor, Fabienne NMN, 95630 Meriel (FR); Velazquez Mendoza, José Maria, Egham, Surrey TW209NW (GB); Stonehouse, Jonathan Richard, Egham, Surrey TW209NW (GB); Staite, William Eoghan, Egham, Surrey TW209NW (GB); Stanton, David Thomas, Cincinnati, OH Ohio 45202 (US); Todini, Oreste NMN, 1853 Brussels (BE); Schofield, Stephen Robert, Egham, Surrey TW209NW (GB); Nyakana, Sarah Kyakyo Kanyunyuzi, Egham, Surrey TW209NW (GB); Fernando Prieto, Susana NMN, 1853 Brussels (BE); Smets, Johan NMN, 1853 Brussels (BE); Scheibel, Jeffrey John, Cincinnati, OH Ohio 45202 (US); Guimet, Isabelle NMN, 1853 Brussels (BE)
(74) Representative: P&G Patent Germany

(56) References cited:
- WO-A1-2015/089246
- WO-A2-2013/060691
- WO-A2-2013/064412
- US-A1- 2014 352 090
- RAMSBOTHAM J: "FRAGRANCES", TENSIDE, SURFACTANTS, DETERGENTS, CARL HANSER VERLAG, MUNCHEN, DE, vol. 23, no. 6, 1 November 1986 (1986-11-01), pages 325 - 332, XP000196119, ISSN: 0932-3414

## Description

### FIELD OF THE INVENTION

The present invention relates to the field of absorbent articles. In particular, it provides absorbent articles comprising fragrance compositions wherein these compositions comprise fragrance materials in a diamond construction and at least one substantially non-odorous fragrance modulator for improving or enhancing the fidelity and/or longevity of the fragrance profile.

### BACKGROUND OF THE INVENTION

Absorbent articles according to the present invention are articles which can be used to absorb any type of fluid. These articles include absorbent hygienic articles (like for example sanitary napkins, pantyliners, tampons, inter labial articles, adult incontinence articles such as adult incontinence pads, pants and diapers, baby pants and diapers, breast pads and hemorrhoid pads). Other absorbent articles according to the present invention can be for example absorbent paper towels, wipes, toilet paper, or facial tissues as well as absorbent articles used in the medical field such as wound dressings and surgical articles and absorbent articles used in food technology and conservation (such as fluid pads for meat, fish and so on). Absorbent articles according to the present invention include also absorbent materials used industrially to absorb fluids, for example to contain spillage of chemicals in fluid form.

Absorbent articles are commonly used to absorb and in some cases retain bodily fluids and other exudates excreted by the human or animal body, such as urine, menses, blood, fecal materials or mucus or chemicals or any type of fluid waste. Paper towels, wipes, facial tissues and toilet paper may be used also to absorb kitchen and food residues and/or any kind of dirt or waste. In many cases the absorbed materials, can be malodourant or can generate malodors with time while the article is still being used or after it has been disposed or thrown in the trash.

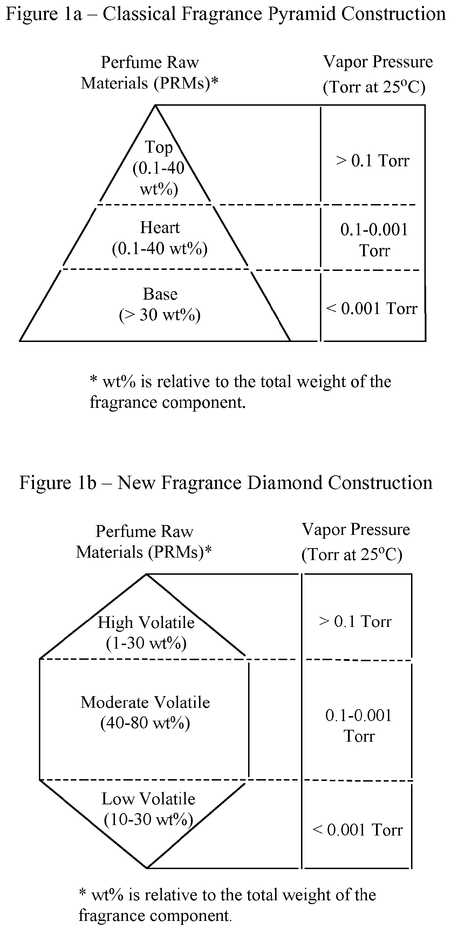

Materials for controlling and reducing malodors in absorbent articles have been identified in the art. Among them conventional perfumes and fragrance composition have been commonly used. However conventional perfuming composition are typically not completely satisfactory in absorbent articles where it is necessary that the perfume express and maintains a defined perfume character for a long time. Absorbent hygienic articles are in fact worn for many hours and also absorbent article after disposal may still have an unpleasant odor so that a perfume should continue to be perceived even after disposal of the absorbent article. In fact conventional perfuming compositions have a fragrance profile characterized by the classical fragrance pyramid three-tiered structure, which contains a higher amount of the base notes, a medium amount of the heart notes, and a lower amount of the top notes (see Fig. 1a). The conventional pyramid structure is used because higher levels of the base notes are relied upon to provide the intensity of the overall fragrance profile over time and replace the hearts notes when those are declining. Simply increasing the levels of heart and top notes does not provide the required longevity because of their fast-evaporation.

Perfumers typically classify fragrance materials as a base, heart or top note according to their characteristic aromas. For instance, the fragrance material "Hedione^{®}" (or also known as "methyl dihydrojasmonate") is commonly classified as a heart note based on its perceived floral aroma. However, due to the somewhat subjective nature of aromas, there has been no universal convention for objectively classifying fragrance materials. As a result of the subjective classification approach, fragrance formulation has been inconsistent. For example, two compositions having the exact same classification of fragrance materials constructed according to the classical fragrance pyramid structure could have two different, possibly very different, fragrance profiles.

With the classical fragrance pyramid structure, "base notes" make up from greater than 30 wt%, typically greater than 40 wt% or typically greater than 50 wt%, relative to the total weight of the perfume formulation. Base notes are characterized by providing animalic, woody, sweet, amber or musky aromas, and not being very volatile. The "heart or middle notes", make up from about 0.1 wt% to about 40 wt% relative to the total weight of the perfume formulation and have an intermediate volatility. Heart notes are associated with desirable aromas such as floral aromas (e.g., jasmin, rose), fruity, marine, aromatic or spicy aromas. The "top or head notes" provide citrusy, green, light, or fresh aromas, and make up from about 0.1 wt% to about 40 wt% relative to the total weight of the perfume formulation. Top notes tend to evaporate quickly due to their high volatility.

There are at least one of several drawbacks to the above described classical formulation approach. Firstly, classification of fragrance materials by their aromas is subjective and therefore results in inconsistency with the construction of the fragrance profile under classical fragrance pyramid construction rules. Secondly, the perceived intensity of the fragrance profile of the conventional perfume compositions, particularly those aromas attributable to the more volatile fragrance materials, decreases rapidly over time due to their quick evaporation. Accordingly, conventional perfume compositions will typically change their overall fragrance profile over time. This is a problem because it is desirable to maintain "fragrance profile fidelity" over time. In other words, it is desirable to maintain the same or substantively similar fragrance profile for a commercial fragrance over time, particularly over long periods of time (at least 4 hrs, 6 hrs, or even 8 hours after application). Thirdly, with the classical fragrance pyramid construction, the possible types of fragrance profiles have been somewhat limited. The consequence of using base notes at high levels is that many fragrance dry-downs appear repetitive, boring, non-memorable and un-interesting to consumers. However, if base notes are reduced or excluded then the fragrance intensity weakens over time and does not last for a sufficient duration. Lastly, it is generally accepted that some consumers desire prolonged intensity of select aromas, particularly the floral, spicy or aromatic aromas derived from the heart notes. Unfortunately, the consequence of using high levels of base notes is that they may impart particularly undesirable aroma characters, such as for example, musky, woody, ambery, warm and sweet, which overpower and dominate the more desirable fragrance characters over time, particular over long periods of time. Thus, the unique challenge remains of selectively extending the more desirable aromas attributable from the heart and/or top notes, particularly the heart notes, and even more particularly extending these desirable aromas over long periods of time,

Previous attempts to overcome these problems have been through the use of various "fixatives" or "modulators" to retard the evaporation of the more volatile fragrance ingredients present in fragrance compositions. For instance, U.S. Patent No. 6,737,396B2 (Firmenich) describes a perfume composition formed by mixing 2-30%, relative to the weight of the composition, of a fixative, (1-ethoxyethoxy)cyclododecane, to fix or exalt the musky or aromatic-type notes. U.S. Patent No. 6,440,400B1 (Takasago Perfumery) describes a composition using trimethylcyclohexane derivatives as perfuming-holding agents and melanin-formation inhibitors. U.S. Patent No. 4,313,855 (Dragoco) describes the use in cosmetic compositions of 1-(2,6,6-trimethylcyclohexyl)-hexane-3-ol as an odourless fixative for increasing the perfume's intensity. U.S. Patent No. 6,147,049 (Givaudan) discloses a perfume fixative derived from tera-hydronaphthalenese for use in a wide range of fragrance compositions. WO85/04803 (Diagnostica) describes the use of hyaluronic acid/ hyaluronate as fixatives (*via* molecular encapsulation) in fragrance products to improve persistent of the fragrance. JP Patent No. 61-083114 (Kanebo) describes ether derivatives as aroma-preserving agent for fine perfume composition. JP Patent No. 61-063612 (Kanebo) discloses diethylene glycol ether derivatives as fragrance adjusting agent showing effects as a fixative and a solubilizer. JP Patent No. 62-084010 (Shiseido) describes hydroquinone glycoside as perfume fixatives applicable for all kinds of perfume and blended perfume. U.S. Patent No. 7,196,052 (Takasago Int. Corp.) describes fragrance compositions containing glycerol ether derivatives as fixatives or fragrance note-improving agent. EP Patent Publication No. 616800A2 (Givaudan) discloses odourant compositions containing panethenol ethyl ether having improved prolonged diffusion of the perfume materials from the skin, without notably modifying the olfactive note of the product. U.S. Patent No. 4,110,626 (Shiseido) describes the use of aliphatic dibasic acid diester as "perfume controlling agent" for improved fixing effect on fragrance component. PCT Publication WO2014/155019 (LVMH) describes aliphatic ether derivatives to increase the stability of alcoholic fragrance composition and more particularly to preserve the original olfactive notes. Further fragrance compositions are disclosed in WO2013/060691 and WO2013/064412.

These attempts have advocated the use of such fixatives or modulators indiscriminately without regard to the fragrance profile. Further, these attempts do not teach how to objectively classify the fragrance materials as low, moderate or high volatile fragrance materials. Further, the use of fixatives or modulators in these attempts often shows effects on single fragrance material, which are often not observed in a fragrance composition of a mixture of fragrance materials where a number of such fragrance materials are competing with each other to interact with said fixatives or modulators. They do not teach how to formulate with fixatives or modulators in fragrance mixtures, which is not trivial. As a result, these attempts, while disclosing compositions that retain the perfume by way of fixatives or modulators, neither teach the fragrance diamond construction in compositions nor the particular type or levels of fragrance materials to include for delivering the benefits of improved fidelity to the perceived fragrance profile over time, or improved longevity of the fragrance profile, preferably the aromas attributable from the moderate or high volatile fragrance materials, particularly the moderate volatile fragrance materials.

On the other hand, other attempts propose a selective approach aimed at the selection of specific fixatives or modulators and defined amounts of fragrance materials. For instance, U.S. Patent No. 7,538,081 (Takasago Perfumery) approaches the problem of fixing a perfume and/or extending the perfume release from a fragrance composition. More particularly, said document describes the use of L-menthoxy ether derivatives as fixatives in fragrance compositions comprising at least one note selected from: floral, citrus, fruity, green, mint, herb and marine. U.S. Patent Publication No. 2011/0104089A1 (Symrise) describes certain compositions containing neopentyl glycol diisononanoate as a fixative for top note perfume oils by increasing their adhereance to skin and hair. U.S. Patent Publication No. 2011/0091404 (Symrise) discloses the use of N-hexadecyl n-nonanoate and N-octadecyl n-nonanoate as fixatives of fragrance substances, particularly the readily volatile top notes, by lowering their vapor pressure to allow for a time-delayed release of the perfume oil components from a composition.

However, these attempts tend not to describe how to formulate with fixatives or modulators in complex mixtures of fragrance materials. For those references that do describe mixtures of fragrance materials, they have different fragrance design criteria and are directed to specific preferred fixatives or modulators.

Thus, it is an advantage of the present invention to provide new rules for objectively classifying fragrance materials according to their volatility using their their vapor pressures defined at suitable temperature, instead of their aromas. The new rules operate irrespective of the perfumers performing the classification. In particular, the new rules classify the fragrance materials into low, moderate or high volatile fragrance materials for formulating into fragrance mixtures, particularly ones having a diamond construction. It is a further advantage of the present invention to provide absorbent articles comprising fragrance compositions having improved fidelity to the perceived fragrance profile over time. It is yet a further advantage to provide a composition, wherein the aroma attributable to moderate and high volatile fragrance materials, particularly the moderate volatile fragrance materials, remains significantly consistent from its initial impression to the end. It is yet a further advantage to provide compositions having improved longevity of the perceived fragrance profile, preferably the aromas attributable from the moderate or high volatile fragrance materials, particularly the moderate volatile fragrance material. It is yet a further advantage to provide compositions having stable quality of end product (*e.g*., fragrance profile, visual appearance) substantially comparable to the classical fragrance pyramid three-tiered structure, preferably even after three months storage at 40 °C. It is yet a further advantage to be able to create new to the world fragrance profiles wherein one, or several, well-recognized moderate volatile fragrance material characters, are maintained over time, preferably for long periods of time (*e.g.,* > 4, 6, or even 8 hours).

US 2014/0352090 discloses absorbent articles with a variety of fragrance compositions that meet the needs of various different consumers.

### SUMMARY OF THE INVENTION

The inventors have discovered new rules for objectively classifying fragrance materials according to their vapor pressures into low, moderate and high volatile fragrance materials for formulating into fragrance mixtures, preferably complex mixtures having a diamond construction.

The invention is defined by the claims.

In a first aspect, the present invention is directed to an absorbent article comprising a fragrance composition wherein said fragrance composition comprises a diamond construction fragrance formulation (see Fig. 1b) and at least one substantially non-odorous fragrance modulator for delivering enhanced intensity of the perceived fragrance profile over time, preferably the components attributable from the moderate and high volatile fragrance materials. In particular, the present invention is directed to an absorbent article according to claim 1.

These and other features of the present invention will become apparent to one skilled in the art upon review of the following detailed description when taken in conjunction with the appended claims.

### BRIEF DESCRIPTION OF THE DRAWINGS

While the specification concludes with claims particularly pointing out and distinctly claiming the invention, it is believed that the invention will be better understood from the following description of the accompanying figures wherein:
Figure 1a is a diagram of the classical fragrance pyramid structure of the prior art.
Figure 1b is a diagram of a fragrance diamond construction according to an embodiment of the present invention.

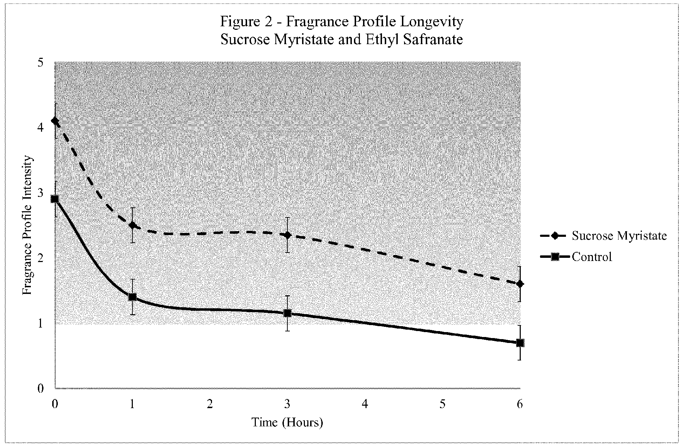

Figure 2 provides the panel test results of perceived fragrance profile, particularly improved fragrance profile longevity of Composition R comprising ethyl safranate fragrance material and Sucrose Myristate substantially non-odorous fragrance modulator as compared to Composition S, a control absent of a substantially non-odorous fragrance modulator (Sucrose Myristate), and as a function of time elapsed since application of the composition.

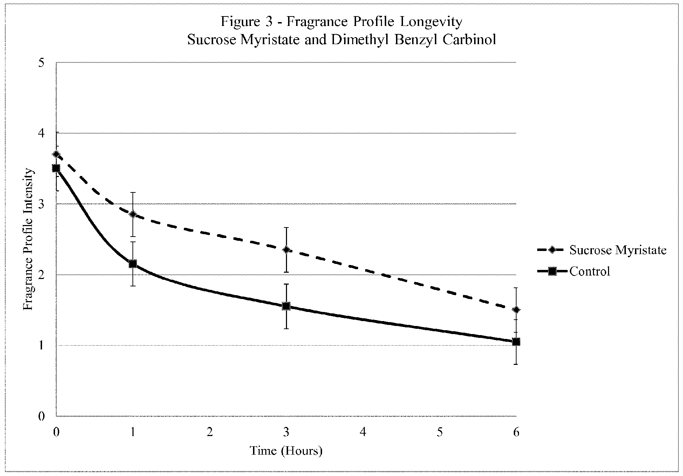

Figure 3 provides the panel test results of perceived fragrance profile, particularly improved fragrance profile longevity of Composition P comprising dimethylbenzyl carbinol fragrance material and Sucrose Myristate substantially non-odorous fragrance modulator as compared to Composition Q, a control absent of a substantially non-odorous fragrance modulator (Sucrose Myristate), and as a function of time elapsed since application of the composition.

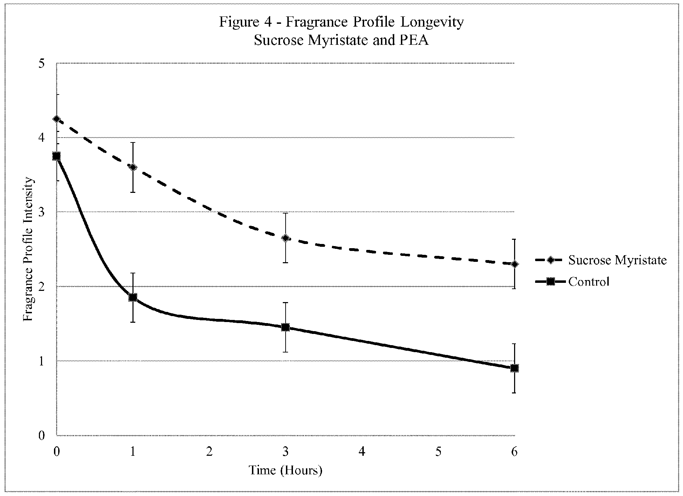

Figure 4 provides the panel test results of perceived fragrance profile, particularly improved fragrance profile longevity of Composition T comprising phenethyl alcohol and Sucrose Myristate substantially non-odorous fragrance modulator as compared to Composition U, a control absent of a substantially non-odorous fragrance modulator (Sucrose Myristate), and as a function of time elapsed since application of the composition.

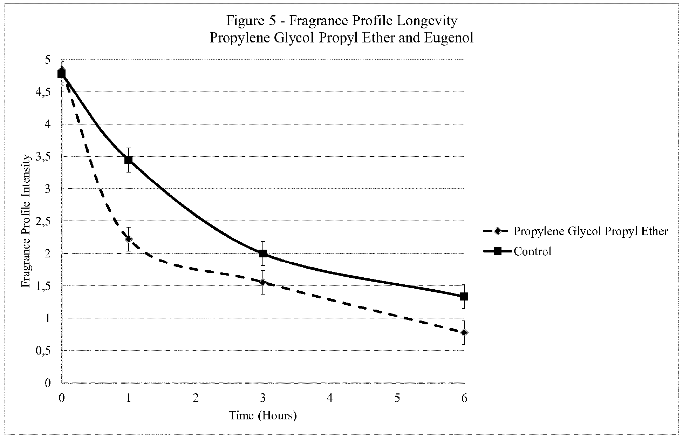

Figure 5 provides the panel test results of perceived fragrance profile, particularly improved fragrance profile longevity of Composition P comprising Eugenol fragrance material and Propylene Glycol Propyl Ether (PGPE) substantially non-odorous fragrance modulator as compared to Composition Q, a control absent of a substantially non-odorous fragrance modulator (PGPE), and as a function of time elapsed since application of the composition.

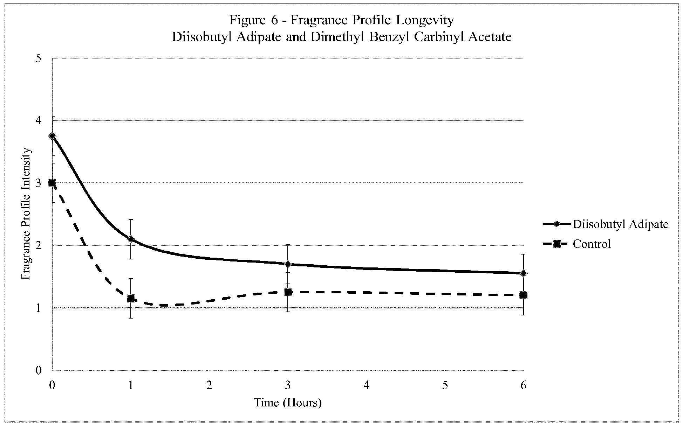

Figure 6 provides the panel test results of perceived fragrance profile, particularly improved fragrance profile longevity of Composition R comprising Dimethyl Benzyl Carbinyl Acetate fragrance material and Diisobutyl Adipate substantially non-odorous fragrance modulator as compared to Composition S, a control absent of a substantially non-odorous fragrance modulator (Diisobutyl Adipate), and as a function of time elapsed since application of the composition.

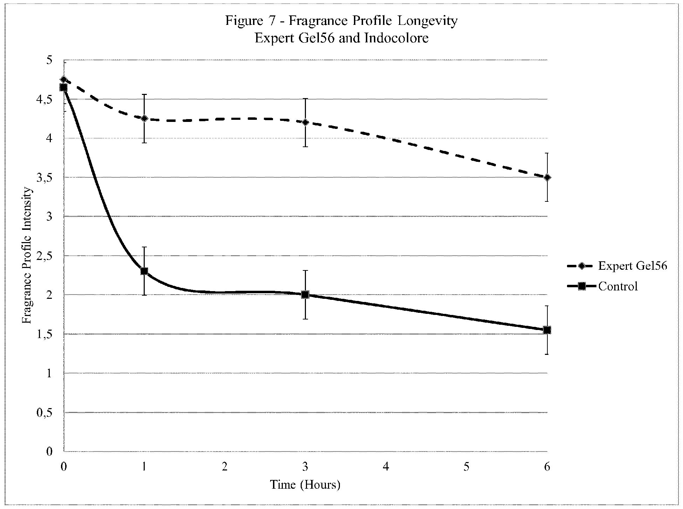

Figure 7 provides the panel test results of perceived fragrance profile, particularly improved fragrance profile longevity of Composition AAA comprising Indocolore fragrance material and Expert Gel56 substantially non-odorous fragrance modulator as compared to Composition BBB, a control absent of a substantially non-odorous fragrance modulator (Expert Gel56), and as a function of time elapsed since application of the composition.

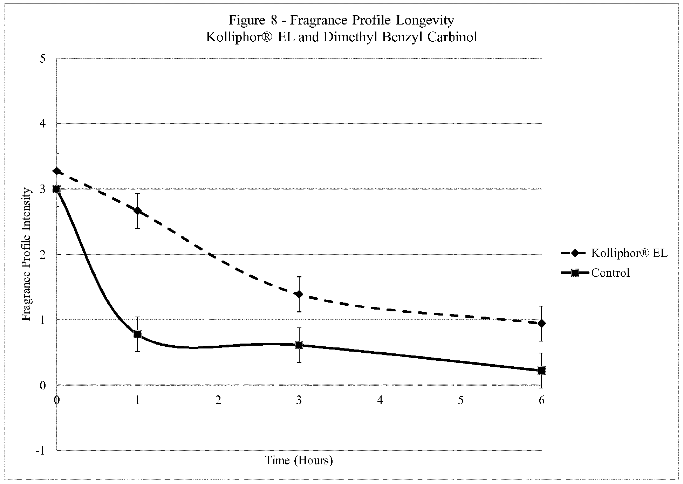

Figure 8 provides the panel test results of perceived fragrance profile, particularly improved fragrance profile longevity of Composition CCC comprising Dimethyl Benzyl Carbinol fragrance material and Kolliphor^{®} EL substantially non-odorous fragrance modulator as compared to Composition DDD, a control absent of a substantially non-odorous fragrance modulator (Kolliphor^{®} EL), and as a function of time elapsed since application of the composition.

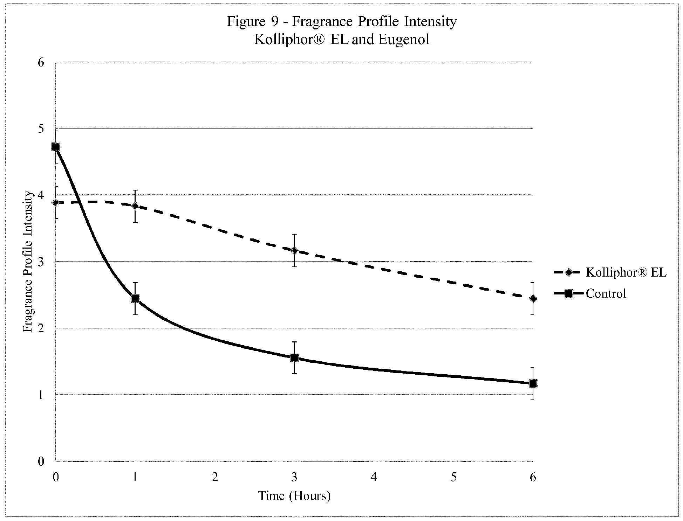

Figure 9 provides the panel test results of perceived fragrance profile, particularly improved fragrance profile longevity of Composition EEE comprising Eugenol fragrance material and Kolliphor^{®} EL substantially non-odorous fragrance modulator as compared to Composition FFF, a control absent of a substantially non-odorous fragrance modulator (Kolliphor^{®} EL), and as a function of time elapsed since application of the composition.

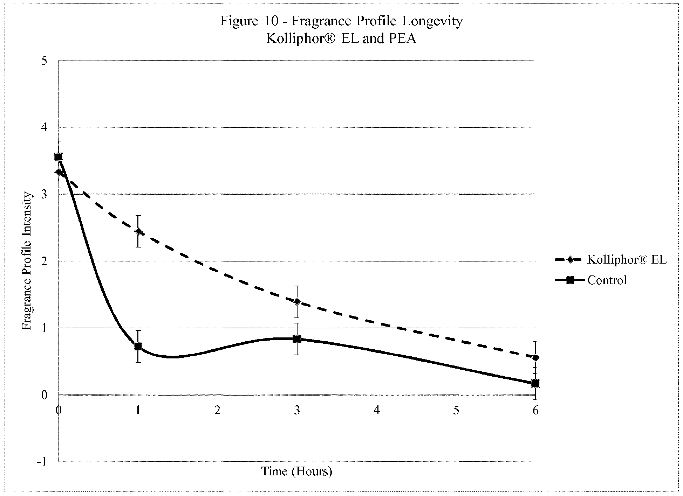

Figure 10 provides the panel test results of perceived fragrance profile, particularly improved fragrance profile longevity of Composition GGG comprising Phenethyl alcohol fragrance material and Kolliphor^{®} EL substantially non-odorous fragrance modulator as compared to Composition HHH, a control absent of a substantially non-odorous fragrance modulator (Kolliphor^{®} EL), and as a function of time elapsed since application of the composition.

### DETAILED DESCRIPTION OF THE INVENTION

### Definitions

"Absorbent article" refers to articles that absorb any type of fluid. These articles are typically disposable and include paper towels, wipes, toilet paper, facial tissue, absorbent articles used in the medical field such as wound dressings and surgical articles, absorbent articles used in food technology and conservation (such as fluid pads for meat, fish and the like), absorbent articles used industrially to absorb fluids, for example to contain spillage of chemicals in fluid form and absorbent hygienic articles. The term "absorbent hygienic articles" refers to devices that absorb and contain body exudates, such as urine, menses, blood and feces. The term "disposable" is used herein to describe absorbent articles which are not intended to be laundered or otherwise restored or reused as an absorbent article after a single use. Examples of absorbent hygienic articles include diapers, toddler training pants, adult incontinence pants, pads or diapers, and feminine hygiene garments such as sanitary napkins, pantiliners, tampons, interlabial articles, breast pads, hemorrhoid pads, and the like.

Absorbent hygienic articles and components thereof, including the topsheet, backsheet, absorbent core, and any individual layers of these components, can have a body-facing surface and a garment-facing surface. As used herein, "body-facing surface" means that surface of the article or component which is intended to be worn toward or adjacent to the body of the wearer, while the "garment-facing surface" is on the opposite side and is intended to be worn toward or placed adjacent to the wearer's undergarments when the disposable absorbent article is worn.

Most absorbent hygienic articles of the present invention (except those for internal use such as tampons) typically comprise a topsheet, a backsheet, and an absorbent core disposed between the topsheet and backsheet.

The topsheet of the absorbent hygienic article is preferably compliant, soft feeling, and non-irritating to the wearers skin and hair. Further, the topsheet is liquid pervious, permitting liquids (e.g., menses and/or urine) to readily penetrate through its thickness. A suitable topsheet may be manufactured from a wide range of materials such as woven and nonwoven materials (e.g., a nonwoven web of fibers), polymeric materials such as apertured formed thermoplastic films, apertured plastic films, and hydroformed thermoplastic films, porous foams, reticulated foams, reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers. When the topsheet comprises a nonwoven web, the web may be manufactured by a wide number of known techniques. For example, the web may be spunbonded, carded, wet-laid, melt-blown, hydroentangled, combinations of the above, or the like.

The backsheet can be impervious to liquids (e.g., menses and/or urine) and can be preferably manufactured from a thin plastic film, although other flexible materials may also be used such as nonwovens. As used herein, the term "flexible" refers to materials which are compliant and will readily conform to the general shape and contours of the human body. The backsheet can prevent the exudates absorbed and contained in the absorbent core from wetting articles which contact the absorbent article such as bedsheets, pants, pajamas and undergarments. The backsheet can also be vapor permeable ("breathable"), while remaining fluid impermeable. The backsheet may comprise a woven or nonwoven material, polymeric films such as thermoplastic films of polyethylene or polypropylene, or composite materials such as a film-coated nonwoven material.

The backsheet can comprise panty fastening means applied on its surface, particularly the surface facing outside the absorbent article in order to allow the article to stay in place when worn between the user's crotch and panties. Such panty fastening means can be for example a layer of adhesive or mechanical means such as Velcro^{®} or combination thereof. When an adhesive is present, typically a release paper is also present in order to protect the adhesive before use.

The backsheet and the topsheet can be positioned respectively adjacent the garment surface and the body surface of the absorbent core. The absorbent core can be joined with the topsheet, the backsheet, or both in any manner as is known by attachment means such as those well known in the art. Embodiments of the present invention are envisioned wherein portions of the entire absorbent core are unattached to either the topsheet, the backsheet, or both.

### Absorbent core

The absorbent core can be formed from any of the materials well known to those of ordinary skill in the art. Examples of such materials include multiple plies of creped cellulose wadding, fluffed cellulose fibers, wood pulp fibers also known as airfelt, textile fibers, a blend of fibers, a mass or batt of fibers, airlaid webs of fibers, a web of polymeric fibers, and a blend of polymeric fibers. Other suitable absorbent core materials include absorbent foams such as polyurethane foams or high internal phase emulsion ("HIPE") foams. Suitable HIPE foams are disclosed in US 5,550,167, US 5,387,207, US 5,352,711, and US 5,331,015.

For some absorbent articles, the absorbent core can be relatively thin, less than about 5 mm in thickness, or less than about 3 mm, or less than about 1 mm in thickness. Thickness can be determined by measuring the thickness at the midpoint along the longitudinal centerline of the pad by any means known in the art while under a uniform pressure of 1.72 kPa.

The absorbent core can comprise superabsorbent materials such as absorbent gelling materials (AGM), including AGM fibers, as is known in the art. The absorbent core can therefore constitute a layer comprising superabsorbent material.

The absorbent article can comprise other additional components, for example between the topsheet and absorbent core, such as a secondary topsheet or acquisition layer. The secondary topsheet or acquisition layer can comprise a tissue layer or a nonwoven, such as carded resin-bonded nonwovens, embossed carded resin-bonded nonwovens, high-loft carded resin-bonded nonwovens, carded through-air-bonded nonwovens, carded thermo-bonded nonwovens, spunbonded nonwovens, and the like. A variety of fibers can be used in the secondary topsheet or acquisition layer, including natural fibers, e.g. wood pulp, cotton, wool, and the like, as well as biodegradeable fibers, such as polylactic acid fibers, and synthetic fibers such as polyolefins (e.g., polyethylene and polypropylene), polyesters, polyamides, synthetic cellulosics (e.g., RAYON^{®}, Lyocell), cellulose acetate, bicomponent fibers, and blends thereof. The basis weight of the secondary topsheet or acquisition layer can vary depending upon the desired application.

The absorbent article can comprise further components such as side cuffs, typically found in diapers, or side wings or side flaps, typically found in sanitary napkins.

Absorbent catamenial tampons are absorbent articles for internal use in the vagina which are typically made by a pledget comprising absorbent fibers compressed to a cylindrical shape. Tampons can be "digital tampons" when they have a self sustaining shape and can be inserted with a finger or "applicator tampons" i.e. tampons which are introduced using an applicator. Tampons can also comprise an extraction cord so to facilitate extraction from the vagina.

The absorbent hygienic articles herein are preferably disposable after a single use.

Absorbent hygienic articles herein are often commercialized in packages containing a plurality of units, often the package is a plastic film or a carton box. Single units contained within the commercial package can be individually packaged or not.

As used herein, the term "composition" includes a fine fragrance composition intended for application to a body or product surface, such as for example, skin or hair, *i.e.,* to impart a pleasant odor thereto, or cover a malodour thereof. The fine fragrance compositions may be ethanol based compositions..

As used herein, the term "consumer" means both the user of the composition and the observer nearby or around the user.

As used herein, the term "diamond construction" means a fragrance formulation as shown in Fig. 1b. In particular, the diamond construction relates to the relative weight % of the fragrance materials classified according to their vapor pressure category (*i.e.,* low, moderate or high). A diamond constructed fragrance has a substantially greater amount of the perfume raw materials of a moderate volatility as comparied to the low and high volatile fragrance materials.

As used herein, the term "fragrance material" and "fragrance materials" relates to a perfume raw material ("PRM"), or a mixture of perfume raw materials ("PRMs"), that are used to impart an overall pleasant odour or fragrance profile to a composition. "Fragrance materials" can encompass any suitable perfume raw materials for fragrance uses, including materials such as, for example, alcohols, aldehydes, ketones, esters, ethers, acetates, nitriles, terpene hydrocarbons, nitrogenous or sulfurous heterocyclic compounds and essential oils. However, naturally occurring plant and animal oils and exudates comprising complex mixtures of various chemical components are also know for use as "fragrance materials". The individual perfume raw materials which comprise a known natural oil can be found by reference to Journals commonly used by those skilled in the art such as "Perfume and Flavourist" or "Journal of Essential Oil Research", or listed in reference texts such as the book by S. Arctander, Perfume and Flavor Chemicals, 1969, Montclair, New Jersey, USA and more recently re-publisehd by Allured Publishing Corporation Illinois (1994). Additionally, some perfume raw materials are supplied by the fragrance houses (Firmenich, International Flavors & Fragrances, Givaudan, Symrise) as mixtures in the form of proprietary speciality accords. Non-limiting examples of the fragrance materials useful herein include pro-fragrances such as acetal pro-fragrances, ketal pro-fragrances, ester pro-fragrances, hydrolyzable inorganic-organic pro-fragrances, and mixtures thereof. The fragrance materials may be released from the pro-fragrances in a number of ways. For example, the fragrance may be released as a result of simple hydrolysis, or by a shift in an equilibrium reaction, or by a pH-change, or by enzymatic release.

As used herein, the term "fragrance profile" means the description of how the fragrance is perceived by the human nose at any moment in time. The fragrance profile may change over time. It is a result of the combination of the low, moderate and high volatile fragrance materials, if present, of a fragrance. A fragrance profile is composed of 2 characteristics: 'intensity' and 'character'. The 'intensity' relates to the perceived strength whilst 'character' refers to the odour impression or quality of the perfume, *i.e*., fruity, floral, woody, *etc.*

As used herein, the terms "modulator", "fragrance modulator" and "fixative" are used interchangeably to designate an agent having the capacity to affect the fragrance profile, such as for example, by impacting the fragrance materials' evaporation rate. The modulator may mediate its effect by lowering the vapor pressure of the fragrance materials and increasing their adherence to the substrate (skin and/or hair) thus ensuring a longer-lasting impression of the fragrance. By incorporating the modulator, it is desired that the fragrance profile, preferably the fragrance components of the diamond construction attributable to the moderate and high volatile fragrance materials of the composition can be perceived by a consumer, over a longer period of time, as compared to the same perception in the absence of the fragrance diamond construction and the modulator. Suitable examples of the modulator are provided herein below. However, as discovered by the inventors, simply adding modulators to a traditionally constructed fragrance composition (*i.e.,* classical fragrance pyramid construction) will not ensure an improved or enhanced fidelity and/or longevity of the fragrance profile over time. Instead, it is only when the modulators are added in the presence of the fragrance diamond construction can the improved or enhanced fidelity and/or longevity of the fragrance profile, preferably attributable to the moderate and high volatile fragrance materials, be perceived as compared to control composition absent the fragrance diamond construction and modulators.

As used herein, the term "substantially non-odorous" means an agent that does not impart an odour of its own when added into a composition of the present invention. For example, a "substantially non-odorous fragrance modulator" does not impart a new odour that alters the character of the fragrance profile of the composition to which it is added. The term "substantially non-odorous" also encompasses an agent that may impart a minimal or slight odour of its own when added into a composition of the present invention. However, the odour imparted by the "substantially non-odorous fragrance modulator" is generally undetectable or tends to not substantively alter the character of the fragrance profile of the composition to which it is added initially or preferably over time. Furthermore, the term "substantially non-odorous" also includes materials that are perceivable only by a minority of people or those materials deemed "anosmic" to the majority of people.

As used herein, the term "vapor pressure" means the partial pressure in air at a defined temperature (*e.g*., 25 °C) and standard atmospheric pressure (101.325 kPa (760 mmHg)) for a given chemical species. It defines a chemical species' desire to be in the gas phase rather than the liquid or solid state. The higher the vapor pressure the greater the proportion of the material that will, at equilibrium, be found in a closed headspace. It is also related to the rate of evaporation of a fragrance material which is defined in an open environment where material is leaving the system. The vapor pressure is determined according to the reference program Advanced Chemistry Development (ACD/Labs) Software Version 14.02, or preferably the latest version update).

It is understood that the test methods that are disclosed in the Test Methods Section of the present application must be used to determine the respective values of the parameters of Applicants' inventions as described and claimed herein.

In all embodiments of the present invention, all percentages are by weight of the total composition, as evident by the context, unless specifically stated otherwise. All ratios are weight ratios, unless specifically stated otherwise, and all measurements are made at 25 °C, unless otherwise designated.

### Compositions

The inventors have surprisingly discovered a revolutionary new way of objectively classifying fragrance materials and then formulating those fragrance materials into complex fragrance mixtures having improved fragrance profile fidelity and longevity. Essentially, the solution is to formulate the fragrance materials into a diamond construction in the presence of a substantially non-odorous fragrance modulator to provide for improved or enhanced longevity and/or fidelity of the fragrance profile, particularly amongst aromas derived from the more volatile fragrance materials (*i.e.,* moderate and high vapor pressure range of the perfumer's palette). In fact, the inventors have discovered that in the complete absence of the low volatile fragrance materials or at very low levels of the low volatilie fragrance materials (< 10wt% relative to the total weight of the fragrance component) there is insufficient character complexity and roundness of the fragrance profile for consumer acceptance of the composition. Therefore the level of low volatile fragrance materials needs to be carefully chosen between 10 wt% and 30 wt%, relative to the total weight of the fragrance component, to balance consumer acceptance and the desired improved or enhanced longevity and/or fidelity of the fragrance profile, particularly amongst aromas attributable to the moderate and/or high volatile fragrance materials.

Unlike previous proposed classification of fragrance materials according to their characteristic aromas, which tends to be subjective, the inventors have established new rules to objectively classifying fragrance materials into low, moderate or high volatile fragrance materials according to their volatility using their vapor pressures defined at a suitable temperature. For example, Hedione^{®} which has been typically classified as a heart note under the traditional approach is now classified as a low volatile fragrance material because it has a vapor pressure of 0.0947 Pa (0.00071000 Torr) at 25 °C. This new classification better reflects Hedione^{®}'s technical properties of slow evaporation and long lasting properties.

Also unlike previous proposed use of modulators to enhance fragrance profile, the inventors have established that the improved aforementioned advantages are not tied to a particular modulator of specific nature/structure but can be reapplied broadly. In fact, what the inventors have established is a systematic approach for providing longer lasting fragrance profiles that is totally unexpected and advantageous contribution to the perfumery technology.

Specifically, in one aspect, the present invention provides for absorbent articles according to claim 1, comprising a fragrance composition comprising the fragrance component present in an amount of from about 0.04 wt% to 30 wt%, preferably 1 wt% to about 30 wt%, more preferably less than about 25 wt%, yet more preferably less than about 20 wt%, yet even more preferably less than about 15 wt%, yet even more preferably less than about 10 wt% or most preferably less than about 8 wt%, relative to the total weight of the composition. Alternatively, the fragrance component is present in an amount of from about 0.04 wt%, 0.3 wt%, 1 wt%, 8 wt% or 10 wt%, to about 15 wt%, 20 wt%, 25 wt% or 30 wt%, relative to the total weight of the composition.

### (i) Low Volatile Fragrance Materials

The fragrance component comprises at least one low volatile fragrance material having a vapor pressure < 0.133 Pa (0.001 Torr) at 25 °C. Preferably the composition according to the present invention comprises at least 3 low volatile fragrance materials, or at least 5 low volatile fragrance materials, or at least 7 low volatile fragrance materials. It is preferred that the composition of the present invention comprises low, preferably very low levels of the low volatile fragrance materials, lower than would traditionally be present in a fragrance pyramid three-tiered structure. As such, compositions of the present invention can comprise low levels of the low volatile fragrance material present in an amount of from about 10 wt% to about 30 wt%, preferably less than about 30 wt%, or preferably less than about 29 wt%, or preferably less than about 28 wt%, or preferably less than about 27 wt%, or preferably less than about 26 wt%, or preferably less than about 25 wt%, relative to the total weight of the fragrance component. Alternatively, the low volatile fragrance material is present in an amount of from about 10 wt%, 12 wt%, 15 wt%, 20 wt%, 25 wt% or 30 wt%, relative to the total weight of the fragrance component. If there is more than one low volatile fragrance materials, then the ranges provided hereinabove cover the total of all of the low volatile fragrance materials. Preferable examples of low volatile fragrances materials are provided in Table 1 below.

### (ii) Moderate Volatile Fragrance Materials

The fragrance component comprises at least one moderate volatile fragrance material having a vapor pressure in the range of 13.33 Pa (0.1 Torr) to 0.133 Pa (0.001 Torr) at 25 °C. Preferably the composition according to the present invention comprises at least 3 moderate volatile fragrance materials, or at least 5 moderate volatile fragrance materials, or at least 7 moderate
volatile fragrance materials. It is preferred that the composition of the present invention comprises high, preferably higher levels of the moderate volatile fragrance materials than would traditionally be present in a fragrance pyramid three-tiered structure. As such, compositions of the present invention can comprise high levels of the moderate volatile fragrance materials present in an amount of from about 40 wt% to about 80 wt%, preferably at least about 45 wt%, or preferably at least about 50 wt%, or preferably at least about 55 wt%, or preferably at least about 60 wt%, or preferably at least about 65 wt%, relative to the total weight of the fragrance component. Alternatively, the moderate volatile fragrance material is present in an amount less than about 75 wt%, or preferably less than 72 wt%, or preferably less than 70 wt%, relative to the total weight of the fragrance component. If there is more than one moderate volatile fragrance materials, then the ranges provided hereinabove cover the total of all of the moderate volatile fragrance materials. Preferable examples of modrate volatile fragrances materials are provided in Table 2 below.

### (iii) High Volatile Fragrance Materials

The fragrance component comprises at least one high volatile fragrance material having a vapor pressure > 13.33 Pa (0.1 Torr) at 25 °C. Preferably the composition according to the present invention comprises at least 3 high volatile fragrance materials, or at least 5 high volatile fragrance materials, or at least 7 high volatile fragrance materials. It is preferred that the composition of the present invention comprises high volatile fragrance materials present in an amount of from about 1 wt% to about 30 wt%, preferably less than about 25 wt%, or preferably less than about 22 wt%, or preferably less than about 20 wt%, relative to the total weight of the fragrance component. Alternatively, the low volatile fragrance material is present in an amount of from about 6 wt%, 8 wt%, 10 wt%, 12 wt %, 14 wt% or 16 wt% relative to the total weight of the fragrance component. If there is more than one high volatile fragrance materials, then the ranges provided hereinabove cover the total of all of the high volatile fragrance materials. Preferable examples of high volatile fragrances materials are provided in Table 3 below.

### (iv) Fragrance Modulators

The composition further comprises at least one substantially non-odorous fragrance modulator as described herein below. Preferable examples of the substantially non-odorous fragrance modulators are provided in Table 4 below.

Preferably, the substantially non-odorous fragrance modulator is present in an amount of from about 0.1 wt% to about 20 wt%, preferably from about 0.5 wt% to about 18 wt% or more preferably from about 2.5 wt% to about 15 wt% or combinations thereof, relative to the total weight of the composition. Alternatively, the substantially non-odorous fragrance modulator is present in an amount of from about 0.1 wt%, 0.5 wt% or 2.5 wt% to about 15 wt%, 18 wt% or 20 wt%, relative to the total weight of the composition. If there is more than one substantially non-odorous fragrance modulators, then the ranges provided hereinabove cover the total of all of the substantially non-odorous fragrance modulators.

The substantially non-odorous fragrance modulator of the present invention may be a liquid at temperatures lower than 100 °C, preferably at ambient temperature. The substantially non-odorous fragrance modulators may be fully miscible with the fragrance materials to form a single phase liquid. However, if the fragrance materials are not entirely miscible, or are immiscible, then co-solvents (*e.g*., dipropylene glycol (DPG), triethyl citrate, or others as well known to those skilled in the art) can be added to aid in the solubility of the fragrance materials.

Preferably, the composition according to the present invention, wherein the substantially non-odorous fragrance modulator does not comprise: (i) isocetyl alcohol, PPG-3 myristyl ether, neopentyl glycol diethylhexanoate or their mixtures; and (ii) n-hexadecyl n-nonanoate, n-octadecyl n-nonanoate or their mixtures.

Preferably, the composition according to the present invention, wherein the substantially non-odorous fragrance modulator and fragrance component are present in a weight ratio from about 3:1 to about 1:3.

The inventors have surprisingly discovered that by formulating the fragrance component into a diamond construction in a composition, the effect of the substantially non-odorous fragrance modulator on the fragrance profile, particularly the aromas of the fragrance profile which is attributable to the moderate and high volatile fragrance materials, preferably the moderate volatile fragrace materials, can be improved. By "improved" it is meant that the fragrance profile of the composition, particular the components contributed by the moderate and high volatile fragrance materials, can be perceived by the consumer at later time points such as, for example, 2 hours, 3 hours, 4 hours, 5 hours, 6 hours, 7 hours, 8 hours, 10 hours, and possibly all the way up to 24 hrs after application as compared to controls, i.e., compositions containing the classical fragrance pyramid three-tiered structure and the substantially non-odorous fragrance modulator or compositions containing the classical fragrance pyramid three-tiered structure and no substantially non-odorous fragrance modulator.

Alternatively, by "improved" it can mean that the perception, by the consumer, of the fidelity of the fragrance profile contributed by the moderate and high volatile fragrance materials is markedly increased or enhanced as compared to the controls. "Increased" or "enhanced" means that the consumer perceives the fragrance profile, preferably the aromas attributable to the moderate and/or high volatile fragrance materials, of a composition as not changing from its initial impression or the changes are minimal from when the composition was first applied to when it dissipates. In otherwords, the fidelity of the perceived fragrance profile of the composition is maintained over time.

Typically, it has been very difficult to formulate fragrance profile, particularly a floral or spicy aroma of the moderate volatile fragrance materials, which can last for very long periods, especially throughout the life of the composition after its application, without giving way to the stronger aromas of the low volatile fragrance materials. The present invention of the diamond construction of fragrance materials with the substantially non-odorous fragrance modulators allows perfumers to increase the olfactive perception of the moderate and high volatile fragrance materials, particularly the moderate volatile fragrance materials, to create new characters and address a re-occurring consumer issue that particular fragrance profiles, particularly those having floral or aromatic and spicy aromas, do not last long enough.

Such a solution as presented herein provides enhanced or improved fidelity and/or longevity of the fragrance profile, particularly amongst those composition formulated from volatile fragrance materials having moderate to high vapor pressure ranges (≥ 0.133 Pa (0.001 Torr) at 25 °C), without having to rely on the presence or significant amounts of the low volatile fragrance materials, which has a tendency to overpower and alter the overall fragrance profile, particularly over time. As a result, the present invention provides the perfumer options to formulate compositions having new fragrance profiles not possible before.

### Volatile Solvents

The present invention provides the solution to the problem of extending the longevity of the fragrance profile of compositions, particularly fine fragrance and cosmetic compositions, preferably fine fragrance compositions, which commonly contain high levels of a volatile solvent. Preferably, the composition according to the present invention, further comprising a volatile solvent present in the amount of from about 50 wt% to about 80 wt%, or preferably from about 55 wt% to about 75 wt%, relative to the total weight of the composition, and wherein the solvent is a branch or unbranched C₁ to C₁₀ alkanyl, alkenyl or alkynyl having at least one alcohol moiety, preferably ethanol, or isopropanol, or other alcohols (*e.g*., methanol, propanol, isopropanol, butanol, and mixtures thereof) commonly found in commercial fine fragrance products.

Accordingly, ethanol may be present in any of the compositions of the present invention, and more specifically, it will form from about 10 wt% to about 80 wt%, or even from about 25 wt% to about 75 wt% of the composition, or combinations thereof, relative to the total weight of the composition. Alternatively, ethanol may be present in an amount of from about 10 wt% or 25 wt% to about 75 wt% or 80 wt%, relative to the total weight of the composition. Any acceptable quality of ethanol, compatible and safe for the specific intended use of the composition such as, for example, topical applications of fine fragrance or cosmetic compositions, and is convenient for use in the compositions according to the present invention.

### Water

In yet another aspect, water may be present in any of the compositions of the present invention, and more specifically, it shall not exceed about 40 wt%, preferably about 20 wt% or less, or more preferably about 10 wt% or less, relative to the total weight of the composition. Alternatively, water may be present in an amount of from about 10 wt% or about 20 wt% to about 40 wt%, relative to the total weight of the composition. When the composition is a cosmetic composition the level of water should not be so high that the product becomes cloudy thus negatively impacting the product aesthetics. It is understood that the amount of water present in the composition may be from the water present in the volatile solvent (*e.g*., ethanol) used in the composition, as the case may be.

### Non-Volatile Solvents

The composition may comprise a non-volatile solvent or a mixture of non-volatile solvents. Non-limiting examples of non-volatile solvents include benzyl benzoate, diethyl phthalate, isopropyl myristate, propylene glycol, dipropylene glycol, triethyl citrate, and mixtures thereof. These solvents often are introduced to the product via the perfume oil as many perfume raw materials may be purchased as a dilution in one of these solvents. Where non-volatile solvents are present, introduced either with the perfume materials or separately, then for the purposes of calculating the proportion of fragrance component having a vapor pressure of < 0.133 Pa (0.001 Torr) at 25 °C the total fragrance components does not include non-volatile solvents. In addition if present with cyclic oligosacchrides, the non-volatile solvent may be included at a weight ratio of the non-volatile solvent to the cyclic oligosaccharide of less than 1:1, less than 1:2, less than 1:10, or less than 1:100.

### Entrapment Materials

In yet another aspect, compositions of the present invention may comprise an entrapment material at a level such that the weight ratio of the entrapment material to the fragrance materials is in the range of from about 1:20 to about 20:1. Preferably, the composition may comprise an entrapment material present in the amount of from about 0.001 wt% to about 40 wt%, from about 0.1 wt% to about 25 wt%, from about 0.3 wt% to about 20 wt%, from about 0.5 wt% to about 10 wt%, or from about 0.75 wt% to about 5 wt%, relative to the total weight of the composition. The compositions disclosed herein may comprise from 0.001 wt% to 40%, from 0.1 wt% to 25 wt%, from 0.3 wt% to 20 wt%, from 0.5 wt% to 10 wt% or from 0.75 wt% to 5 wt%, relative to the total weight of the composition, of a cyclic oligosaccharide.

Suitable entrapment materials for use herein are selected from polymers; capsules, microcapsules and nanocapsules; liposomes, absorbents; cyclic oligosaccharides and mixtures thereof. Preferred are absorbents and cyclic oligosaccharides and mixtures thereof. Highly preferred are cyclic oligosaccharides (*see* PCT Publication Nos. WO2000/67721 (Procter & Gamble); and WO2000/67720 (Procter & Gamble); and U.S. Patent No. US 6,893,647 (Procter & Gamble)).

As used herein, the term "cyclic oligosaccharide" means a cyclic structure comprising six or more saccharide units. Preferred for use herein are cyclic oligosaccharides having six, seven or eight saccharide units and mixtures thereof, more preferably six or seven saccharide units and even more preferably seven saccharide units. It is common in the art to abbreviate six, seven and eight membered cyclic oligosaccharides to α, β and γ respectively.

The cyclic oligosaccharide of the compositions used for the present invention may comprise any suitable saccharide or mixtures of saccharides. Examples of suitable saccharides include, but are not limited to, glucose, fructose, mannose, galactose, maltose and mixtures thereof. However, preferred for use herein are cyclic oligosaccharides of glucose. The preferred cyclic oligosaccharides for use herein are α-cyclodextrins or β-cyclodextrins, or mixtures thereof, and the most preferred cyclic oligosaccharides for use herein are β-cyclodextrins.

The cyclic oligosaccharide, or mixture of cyclic oligosaccharides, for use herein may be substituted by any suitable substituent or mixture of substituents. Herein the use of the term "mixture of substituents" means that two or more different suitable substituents can be substituted onto one cyclic oligosaccharide. The derivatives of cyclodextrins consist mainly of molecules wherein some of the OH groups have been substituted. Suitable substituents include, but are not limited to, alkyl groups; hydroxyalkyl groups; dihydroxyalkyl groups; (hydroxyalkyl)alkylenyl bridging groups such as cyclodextrin glycerol ethers; aryl groups; maltosyl groups; allyl groups; benzyl groups; alkanoyl groups; cationic cyclodextrins such as those containing 2-hydroxy-3-(dimethylamino) propyl ether; quaternary ammonium groups; anionic cyclodextrins such as carboxyalkyl groups, sulphobutylether groups, sulphate groups, and succinylates; amphoteric cyclodextrins; and mixtures thereof.

The substituents may be saturated or unsaturated, straight or branched chain. Preferred substituents include saturated and straight chain alkyl groups, hydroxyalkyl groups and mixtures thereof. Preferred alkyl and hydroxyalkyl substituents are selected from C₁-C₈ alkyl or hydroxyalkyl groups or mixtures thereof, more preferred alkyl and hydroxyalkyl substituents are selected from C₁-C₆ alkyl or hydroxyalkyl groups or mixtures thereof, even more preferred alkyl and hydroxyalkyl substituents are selected from C₁-C₄ alkyl or hydroxyalkyl groups and mixtures thereof. Especially preferred alkyl and hydroxyalkyl substituents are propyl, ethyl and methyl, more especially hydroxypropyl and methyl and even more preferably methyl.

Preferred cyclic oligosaccharides for use in the present invention are unsubstituted, or are substituted by only saturated straight chain alkyl, or hydroxyalkyl substituents. Therefore, preferred examples of cyclic oligosaccharides for use herein are α-cyclodextrin, β-cyclodextrin, methyl-α-cyclodextrin, methyl-β-cyclodextrin, hydroxypropyl-α-cyclodextrin and hydroxypropyl-β-cyclodextrin. Most preferred examples of cyclic oligosaccharides for use herein are methyl-α-cyclodextrin and methyl-β-cyclodextrin. These are available from Wacker-Chemie GmbH Hanns-Seidel-Platz 4, Munchen, DE under the tradename Alpha W6 M and Beta W7 M respectively. Especially preferred is methyl-β-cyclodextrin.

The cyclic oligosaccharides of the compositions used for the present invention are preferably soluble in water, ethanol, or both water and ethanol. As used herein "soluble" means at least about 0.1 g of solute dissolves in 100 mL of solvent, at 25 °C and 101.325 kPa (1 atm) of pressure. Preferably the cyclic oligosaccharides for use herein have a solubility of at least about 1 g/100 mL, at 25 °C and 101.325 kPa (1 atm) of pressure. Preferred is that cyclic oligosaccharides are only present at levels up to their solubility limits in a given composition at room temperature. A person skilled in the art will recognise that the levels of cyclic oligosaccharides used in the present invention will also be dependent on the components of the composition and their levels, for example the solvents used or the exact fragrance oils, or combination of fragrance oils, present in the composition. Therefore, although the limits stated for the entrapment material are preferred, they are not exhaustive.

### Antiperspirant Active

The compositions described herein may be free of, substantially free of, or may include an antiperspirant active (*i.e*., any substance, mixture, or other material having antiperspirant activity). Examples of antiperspirant actives include astringent metallic salts, like the inorganic and organic salts of aluminum, zirconium and zinc, as well as mixtures thereof. Such antiperspirant actives include, for example, the aluminum and zirconium salts, such as aluminum halides, aluminum hydroxyhalides, zirconyl oxyhalides, zirconyl hydroxyhalides, and mixtures thereof.

### Other Ingredients

In yet another aspect, the composition consists essentially of the recited ingredients but may contain small amounts (not more than about 10 wt%, preferably no more than 5 wt%, or preferably no more than 2 wt% thereof, relative to the total weight of the composition) of other ingredients that do not impact on the fragrance profile, particularly the evaporation rate and release of the fragrance materials. For example, a fine fragrance composition may comprise stabilizing or anti-oxidant agents, UV filters or quenchers, or colouring agents, commonly used in perfumery. There are a number of other examples of additional ingredients that are suitable for inclusion in the present compositions. These include, but are not limited to, alcohol denaturants such as denatonium benzoate; UV stabilisers such as benzophenone-2; antioxidants such as tocopheryl acetate; preservatives such as phenoxyethanol, benzyl alcohol, methyl paraben, and propyl paraben; dyes; pH adjusting agents such as lactic acid, citric acid, sodium citrate, succinic acid, phosphoric acid, sodium hydroxide, and sodium carbonate; deodorants and anti-microbials such as farnesol and zinc phenolsulphonate; humectants such as glycerine; oils; skin conditioning agents such as allantoin; cooling agents such as trimethyl isopropyl butanamide and menthol; hair conditioning ingredients such as panthenol, panthetine, pantotheine, panthenyl ethyl ether, and combinations thereof; silicones; solvents such as hexylene glycol; hair-hold polymers such as those described in PCT Publication WO94/08557 (Procter & Gamble); salts in general, such as potassium acetate and sodium chloride and mixtures thereof.

In yet another aspect, the composition of the present invention, depending on its intended use, is a mixture of fragrance materials possibly together with other ingredients such as, for example, perfume carriers. By the term "perfume carrier", it is meant to include materials which are practically neutral from a perfumery point of view, *i.e.*, which does not significantly alter the organoleptic properties of perfuming components. The perfume carrier may be a compatible liquid or solid fillers, diluents, and the like. The term "compatible", as used herein, means that the components of the compositions of this invention are capable of being combined with the primary actives of the present invention, and with each other, in a manner such that there is no interaction which would substantially reduce the efficacy of the composition under ordinary use situations. The type of carrier utilized in the present invention depends on the type of product desired and may comprise, but are not limited to, solutions, aerosols, emulsions (including oil-in-water or water-in-oil), gels, and liposomes. Preferably, the carrier is a liquid and will be a solvent such as, for example, dipropyleneglycol, diethyl phthalate, isopropyl myristate, benzyl benzoate, 2-(2-ethoxyethoxy)-l-ethanol, or ethyl citrate (triethyl citrate).

In yet another aspect, the compositions for use in the present invention may take any form suitable for use. These include, but are not limited to, vapor sprays, aerosols, emulsions, lotions, liquids, creams, gels, sticks, ointments, pastes, mousses, powders, granular products, substrates, cosmetics (*e.g.*, semi-solid or liquid makeup, including foundations) and the like. Preferably the compositions for use in the present invention take the form of aliquid. Compositions of the present invention can be further added as an ingredient to other compositions, preferably fine fragrance or cosmetic compositions, in which they are compatible. As such they can be used within solid composition or applied substrates etc.

Preferably, the absorbent articles of the present invention comprise a fragrance composition wherein said fragrance composition comprises:
(i) a fragrance component present in an amount of from about 0.04 wt% to about 30 wt%, relative to the total weight of the composition, and wherein the fragrance component comprises:
   (a) at least one low volatile fragrance material having a vapor pressure < 0.133 Pa (0.001 Torr) at 25 °C;
   (b) at least one moderate volatile fragrance material having a vapor pressure ≥ 0.133 Pa (0.001 Torr) to 13.33 Pa (0.1 Torr) at 25 °C; and
   (c) at least one high volatile fragrance material having a vapor pressure> 13.33 Pa (0.1 Torr) at 25 °C;
      wherein the weight ratio of (a), (b) and (c) is as defined in claim 1;
(ii) at least one substantially non-odorous fragrance modulator present in the amount of from about 0.1 wt% to about 20 wt%, relative to the total weight of the composition;
(iii) a volatile solvent present in an amount of from about 50 wt% to about 80 wt%, relative to the total weight of the composition; and
(iv) optionally water.

Preferably, the present invention uses compositions in the form of a liquid.
Therefore, it goes without saying that the compositions of the present invention encompasses any composition comprising any of the ingredients cited herein, in any embodiment wherein each such ingredient is independently present in any appropriate amount as defined herein. Many such compositions, than what is specifically set out herein, can be encompassed.

The composition of the present invention may be applied on any of the layers making up the absorbent article, for example in liquid form using a liquid or spray applicator. The composition can also be applied in any other form which is known in the art for applying conventional perfume compositions to absorbent articles.
The fragrance composition of the invention can be applied in a variety of ways, and in a variety of patterns, to the absorbent article. For example can be applied in dropplets or spray, or using conventional glue or liquid application equipment such as a slot applicator, which can be used for striped patterns, or air assisted applicators for patterned applications (like spray, spiral, serpentine, fibrils, omega^{®}, signature^{®} and the like) because this allows one to position the fragrance in a preferred way (i.e. in a fem care article the material might not be applied in correspondence with the vaginal opening if so desired). Also patterned applications are helpful because they allow a precise application so that it is easier to avoid contact with the glue which connects the various layers of the absorbent article and which can interact with perfume ingredients.

The fragrance composition can also be incorporated into a liquid or semi-solid carrier and applied as a lotion. The carrier can be for example of polysiloxane oil, mineral oil, petrolatum, polyethylene glycol, glycerin, PEG, PPG and the like, and mixtures thereof.
The frangrance compositions of the present invention can also be introduced in the absorbent articles also as complexed or encapsulated materials, e.g. capsules containg the composition can be introduced into the absorbent article in such a way that these capsules are broken or dissolved and their content is released when the absorbent articlke is used or comes into contanct with the malodorant liquid.
The fragrance composition are typically disposed in the absorbent article in an amount of from about 0.01 to about 1000 milligrams per absorbent article, in some embodiments from about 0.1 to about 100 milligrams per absorbent article, or from about 0.1 to about 500 milligrams per absorbent article.
An effective amount of the composition, typically from about 1 µL to about 10,000 µL, preferably from about 10 µL to about 1,000 µL, more preferably from about 25 µL to about 500 µL, or most preferably from about 50 µL to about 100 µL, or combinations thereof, is applied to the suitable substrate. Alternatively, an effective amount of the composition of the present invention is from about 1 µL, 10 µL, 25 µL or 50 µL to about 100 µL, 500 µL, 1,000 µL or 10,000 µL. The composition may be applied by hand or applied utilizing a delivery apparatus such as, for example, vaporizer or atomizer. Preferably, the composition is allowed to dry after its application to the substrate. The scope of the present invention should be considered to cover one or more distinct applications of the composition or the continuous release of a composition *via* a vaporizer or other type of atomizer.
In combination with the described fragrance compositions the absorbent articles of the invention may also comprise odor controlling materials as known in the art. For example the absorbent articles may comprise odor asorbers such as silica, zeolites, active carbon and the like. Alternatively or in combination absorbent articles mav comprise reactive odor control materials such as those described in EP1842564 and EP2512526. Reactive compounds can also be introduced int the absorbent articles as liquids, but preferable, due to their reactivity, as encapsulated materials as described in WO2014205047, or complexes such as cyclodextrin complexes as known in the art and described for example in EP2114331.

### Fragrance Materials

In order that the compositions can be developed with the appropriate fragrance profile for the present invention, the "fragrance materials" have been classified as low, moderate or high volatile fragrance materials according to their volatility by their vapor pressure. This method of classifying fragrance materials by their vapor pressure avoids the problem of different classifications for the same fragrance material according to the traditional approach that relies on their subjective characteristic aroma. For the purpose of clarity, when the fragrance materials refer to a single individual compound, its vapor pressure should be determined according to the reference program cited above. In the case that the fragrance materials are a natural oil, extract or absolute, which comprises a mixture of several compounds, the vapor pressure of the complete oil should be treated a mixture of the individual perfume raw material components using the reference program cited above. The individual components and their level, in any given natural oil or extract, can be determined by direct injection of the oil into a GC-MS column for analysis as known by one skilled in the art. In the scenario that the fragrance materials are a proprietary specialty accord, so called 'bases', the vapor pressure, using the reference program cited above, should preferably be obtained from the supplier. However, it is understood by one skilled in the art that they can physically analyze the composition of a full fragrance oil available commercially to identity the fragrance raw materials and their levels using standard GC-MS techniques. This would be irrespective of whether they had been added to the fragrance oil as individual chemicals, as components of naturals or from proprietary bases. Although proprietary bases and naturals are included in our examples, when analyzing a commercially available fragrance *via* GC-MS one could simply identify the components of the base or natural oil as part of the overall fragrance mixture and their levels, without being able to identify which proprietary base or natural oil the fragrance had come from.

The nature and type of fragrance materials in the compositions according to the present invention can be selected by the skilled person, on the basis of its general knowledge together with the teachings contained herein, with reference to the intended use or application of the composition and the desired fragrance profile effect. Examples of suitable fragrance materials are disclosed in U.S. Pat. No. 4,145,184, U.S. Pat. No. 4,209,417, U.S. Pat. No. 4,515,705, and U.S. Pat. No. 4,152,272.

### (i) Low Volatile Fragrance Materials

Preferable examples of fragrance materials having a vapor pressure < 0.133 Pa (0.001 Torr) at 25 °C are provided in Table 1 Low Volatile Fragrance Materials. Preferably, the low volatile fragrance material is selected from at least 1 material, or at least 2 materials, or at least 3 materials, or at least 5 materials, or at least 7 low volatile fragrance materials as disclosed in Table 1.

**Table 1 - Low Volatile Fragrance Materials**

| **No.** | **CAS Number** | **IUPAC Name** | **Common Name**** | **Vapor Pressure (Torr at 25°C)*** | **Vapor Pressure (Pa at 25 °C)** |
|---|---|---|---|---|---|
| 1. | 1211-29-6 | Cyclopentaneacetic acid, 3-oxo-2-(2*Z*)-2-penten-1-yl-, methyl ester, (1*R*,2*R*)- | Methyl jasmonate | 0.00096500 | 0.12866 |
| 2. | 28219-60-5 | 2-Buten-1-ol, 2-methyl-4-(2,2,3-trimethyl-3-cyclopenten-1-yl)- | Hindinol | 0.00096100 | 0.12812 |
| 3. | 93-08-3 | Ethanone, 1-(2-naphthalenyl)- | Methyl beta-naphthyl ketone | 0.00095700 | 0.12759 |
| 4. | 67633-95-8 | 3-Decanone, 1-hydroxy- | Methyl Lavender Ketone | 0.00095100 | 0.12679 |
| 5. | 198404-98-7 | Cyclopropanemethanol, 1-methyl-2-[(1,2,2-trimethylbicyclo[3.1.0]hex-3-yl)methyl]- | Javanol^{®} | 0.00090200 | 0.12026 |
| 6. | 121-32-4 | Benzaldehyde, 3-ethoxy-4-hydroxy- | Ethyl vanillin | 0.00088400 | 0.11786 |
| 7. | 72403-67-9 | 3-Cyclohexene-1-methanol, 4-(4-methyl-3-penten-1-yl)-, 1-acetate | Myraldylac etate | 0.00087900 | 0.11719 |
| 8. | 28940-11-6 | 2*H*-1,5-Benzodioxepin-3(4*H*)-one, 7-methyl- | Oxalone^{®} | 0.00083100 | 0.11079 |
| 9. | 139504-68-0 | 2-Butanol, 1-[[2-(1,1-dimethylethyl)cyclohexyl] oxy]- | Amber core | 0.00080300 | 0.10706 |
| 10. | 502847-01-0 | Spiro[5.5]undec-8-en-1-one, 2,2,7,9-tetramethyl- | Spiro[5.5] undec-8-en-1-one, 2,2,7,9-tetramethyl | 0.00073100 | 0.097459 |
| 11. | 2570-03-8 | Cyclopentaneacetic acid, 3-oxo-2-pentyl-, methyl ester, (1*R*,2*R*)-rel- | trans-Hedione | 0.00071000 | 0.094659 |
| 12. | 24851-98-7 | Cyclopentaneacetic acid, 3-oxo-2-pentyl-, methyl ester | Methyl dihydroj as monate or alternatives *1* | 0.00071000 | 0.094659 |
| 13. | 101-86-0 | Octanal, 2-(phenylmethylene)- | Hexyl cinnamic aldehyde | 0.00069700 | 0.092926 |
| 14. | 365411-50-3 | Indeno[4,5-d]-1,3-dioxin, 4,4a,5,6,7,8,9,9b-octahydro-7,7,8,9,9-pentamethyl- | Nebulone | 0.00069200 | 0.092259 |
| 15. | 37172-53-5 | Cyclopentanecarboxylic acid, 2-hexyl-3-oxo-, methyl ester | Dihydro Iso Jasmonate | 0.00067500 | 0.089993 |
| 16. | 65113-99-7 | 3-Cyclopentene-1-butanol, α,β,2,2,3-pentamethyl- | Sandalore^{®} | 0.00062500 | 0.083326 |
| 17. | 68133-79-9 | Cyclopentanone, 2-(3,7-dimethyl-2,6-octadien-1-yl)- | Apritone | 0.00062000 | 0.082660 |
| 18. | 7212-44-4 | 1,6,10-Dodecatrien-3-ol, 3, 7,11-trimethyl- | Nerolidol | 0.00061600 | 0.082127 |
| 19. | 53243-59-7 | 2-Pentenenitrile, 3-methyl-5-phenyl-, (2Z)- | Citronitril | 0.00061500 | 0.081993 |
| 20. | 134123-93-6 | Benzenepropanenitrile, 4-ethyl-α,α-dimethyl- | Fleuranil | 0.00057600 | 0.076794 |
| 21. | 77-53-2 | 1*H*-3a,7-Methanoazulen-6-ol, octahydro-3,6,8,8-tetramethyl-, (3*R*,3a*S*,6*R*, 7*R*,8a*S*)- | Cedrol Crude | 0.00056900 | 0.075860 |
| 22. | 68155-66-8 | Ethanone, 1-(1,2,3,5,6,7,8, 8a-octahydro-2,3,8,8-tetramethyl-2-naphthalenyl) | Iso Gamma Super | 0.00056500 | 0.075327 |
| 23. | 54464-57-2 | Ethanone, 1-(1,2,3,4,5,6,7, 8-octahydro-2,3,8,8-tetramethyl-2-naphthalenyl)- | Iso-E Super^{®} | 0.00053800 | 0.071727 |
| 24. | 774-55-0 | Ethanone, 1-(5,6,7,8-tetrahydro-2-naphthalenyl)- | Florantone | 0.00053000 | 0.070661 |
| 25. | 141-92-4 | 2-Octanol, 8,8-dimethoxy-2,6-dimethyl- | Hydroxycit ronellal Dimethyl Acetal | 0.00052000 | 0.069328 |
| 26. | 20665-85-4 | Propanoic acid, 2-methyl-, 4-formyl-2-methoxyphenyl ester | Vanillin isobutyrate | 0.00051200 | 0.068261 |
| 27. | 79-78-7 | 1,6-Heptadien-3-one, 1-(2, 6,6-trimethyl-2-cyclohexen-1-yl)- | Hexalon | 0.00049800 | 0.066395 |
| 28. | 6259-76-3 | Benzoic acid, 2-hydroxy-, hexyl ester | Hexyl Salicylate | 0.00049100 | 0.065461 |
| 29. | 93-99-2 | Benzoic acid, phenyl ester | Phenyl Benzoate | 0.00047900 | 0.063861 |
| 30. | 153859-23-5 | Cyclohexanepropanol, 2,2, 6-trimethyl-α-propyl-, (1*R,* 6*S*)- | Norlimban ol | 0.00046900 | 0.062528 |
| 31. | 70788-30-6 | Cyclohexanepropanol, 2,2, 6-trimethyl-α-propyl- | Timberol | 0.00046900 | 0.062528 |
| 32. | 68555-58-8 | Benzoic acid, 2-hydroxy-, 3-methyl-2-buten-1-yl ester | Prenyl Salicylate | 0.00045700 | 0.060928 |
| 33. | 950919-28-5 | 2H-1,5-Benzodioxepin-3(4*H*)-one, 7-(1-methylethyl)- | Cascalone | 0.00045500 | 0.060662 |
| 34. | 30168-23-1 | Butanal, 4-(octahydro-4,7-methano-5*H*-inden-5-ylidene)- | Dupical | 0.00044100 | 0.058795 |
| 35. | 1222-05-5 | Cyclopenta[g]-2-benzopyran, 1,3,4,6,7,8-hexahydro-4,6,6,7,8,8-hexamethyl- | Galaxolide ^{®} | 0.00041400 | 0.055195 |
| 36. | 4602-84-0 | 2,6,10-Dodecatrien-1-ol, 3, 7,11-trimethyl- | Farnesol | 0.00037000 | 0.049329 |
| 37. | 95962-14-4 | Cyclopentanone, 2-[2-(4-methyl-3-cyclohexen-1-yl) propyl]- | Nectaryl | 0.00036700 | 0.048929 |
| 38. | 4674-50-4 | 2(3*H*)-Naphthalenone, 4,4a, 5,6,7,8-hexahydro-4,4a-dimethyl-6-(1-methylethenyl)-, (4*R*,4a*S*, 6*R*)*-* | Nootkatone | 0.00035800 | 0.047729 |
| 39. | 3487-99-8 | 2-Propenoic acid, 3-phenyl-, pentyl ester | Amyl Cinnamate | 0.00035200 | 0.046929 |
| 40. | 10522-41-5 | 2-hydroxy-2-phenylethyl acetate | Styrolyl Acetate | 0.00033900 | 0.045196 |
| 41. | 118-71-8 | 4*H*-Pyran-4-one, 3-hydroxy-2-methyl- | Maltol | 0.00033700 | 0.044930 |
| 42. | 128119-70-0 | 1-Propanol, 2-methyl-3-[(1, 7,7-trimethylbicyclo[2.2.1] hept-2-yl)oxy]- | Bornafix | 0.00033400 | 0.044530 |
| 43. | 103614-86-4 | 1-Naphthalenol, 1,2,3,4,4a, 5,8,8a-octahydro-2,2,6,8-tetramethyl- | Octalynol | 0.00033200 | 0.044263 |
| 44. | 7785-33-3 | 2-Butenoic acid, 2-methyl-, (2*E*)-3,7-dimethyl-2,6-octadien-1-yl ester, (2*E*)- | Geranyl Tiglate | 0.00033200 | 0.044263 |
| 45. | 117933-89-8 | 1,3-Dioxane, 2-(2,4-dimethyl-3-cyclohexen-1-yl)-5-methyl-5-(1-methylpropyl)- | Karanal | 0.00033100 | 0.044130 |
| 46. | 629-92-5 | Nonadecane | Nonadecane | 0.00032500 | 0.043330 |
| 47. | 67801-20-1 | 4-Penten-2-ol, 3-methyl-5-(2,2,3-trimethyl-3-cyclopenten-1-yl)- | Ebanol | 0.00028100 | 0.037464 |
| 48. | 65416-14-0 | Propanoic acid, 2-methyl-, 2-methyl-4-oxo-4*H*-pyran-3-yl ester | Maltol Isobutyrate | 0.00028000 | 0.037330 |
| 49. | 28219-61-6 | 2-Buten-1-ol, 2-ethyl-4-(2, 2,3-trimethyl-3-cyclopenten-1-yl)- | Laevo Trisandol | 0.00028000 | 0.037330 |
| 50. | 5986-55-0 | 1,6-Methanonaphthalen-1(2*H*)-ol, octahydro-4,8a,9, 9-tetramethyl-, (1*R,*4*S,*4*aS,* 6*R*,8a*S*)- | Healingwo od | 0.00027800 | 0.037064 |
| 51. | 195251-91-3 | 2*H*-1,5-Benzodioxepin-3(4*H*)-one, 7-(1,1-dimethylethyl)- | Transluzon e | 0.00026500 | 0.035330 |
| 52. | 120-51-4 | Benzoic acid, phenylmethyl ester | Benzyl Benzoate | 0.00025400 | 0.033864 |
| 53. | 3100-36-5 | 8-Cyclohexadecen-1-one | Cyclohexa decenone | 0.00025300 | 0.033731 |
| 54. | 65405-77-8 | Benzoic acid, 2-hydroxy-, (3*Z*)-3-hexen-1-yl ester | cis-3-Hexenyl salicylate | 0.00024600 | 0.032797 |
| 55. | 4940-11-8 | 4*H*-Pyran-4-one, 2-ethyl-3-hydroxy- | Ethyl Maltol | 0.00022800 | 0.030398 |
| 56. | 541-91-3 | Cyclopentadecanone, 3-methyl- | Muskone | 0.00017600 | 0.023465 |
| 57. | 118-58-1 | Benzoic acid, 2-hydroxy-, phenylmethyl ester | Benzyl salicylate | 0.00017500 | 0.023331 |
| 58. | 81783-01-9 | 6,8-Nonadien-3-one, 2,4,4, 7-tetramethyl-, oxime | Labienoxi me | 0.00017300 | 0.023065 |
| 59. | 25485-88-5 | Benzoic acid, 2-hydroxy-, cyclohexyl ester | Cyclohexyl Salicylate | 0.00017300 | 0.023065 |
| 60. | 91-87-2 | Benzene, [2-(dimethoxymethyl)-1-hepten-1-yl]- | Amyl Cinnamic Aldehyde Dimethyl Acetal | 0.00016300 | 0.021732 |
| 61. | 104864-90-6 | 3-Cyclopentene-1-butanol, β,2,2,3-tetramethyl-δ-methylene- | Firsantol | 0.00016000 | 0.021332 |
| 62. | 224031-70-3 | 4-Penten-1-one, 1-spiro[4.5]dec-7-en-7-yl- | Spirogalba none | 0.00015300 | 0.020398 |
| 63. | 134-28-1 | 5-Azulenemethanol, 1,2,3,4,5,6,7,8-octahydro-α,α,3,8-tetramethyl-, 5-acetate, (3*S*,5*R*,8*S*)- | Guaiyl Acetate | 0.00013400 | 0.017865 |
| 64. | 236391-76-7 | Acetic acid, 2-(1-oxopropoxy)-, 1-(3,3-dimethylcyclohexyl)ethyl ester | Romandoli de^{®} | 0.00012400 | 0.016532 |
| 65. | 115-71-9 | 2-Penten-1-ol, 5-[(1*R*,3*R,*6*S*)-2,3-dimethyltricyclo[2.2.1.02,6 ]hept-3-yl]-2-methyl-, (2*Z*)- | cis-alpha-Santalol | 0.00011800 | 0.015732 |
| 66. | 107898-54-4 | 4-Penten-2-ol, 3,3-dimethyl-5-(2,2,3-trimethyl-3-cyclopenten-1-yl)- | Polysantol^{®} | 0.00011700 | 0.015599 |
| 67. | 69486-14-2 | 5,8-Methano-2*H*-1-benzopyran-2-one, 6-ethylideneoctahydro- | Florex^{®} | 0.00011000 | 0.014665 |
| 68. | 84697-09-6 | Heptanal, 2-[(4-methylphenyl)methylene]- | Acalea | 0.00010100 | 0.013466 |
| 69. | 14595-54-1 | 4-Cyclopentadecen-1-one, (4*Z*)- | Exaltenone | 0.00009640 | 0.012852 |
| 70. | 32388-55-9 | Ethanone, 1-[(3*R*,3a*R*,7*R*, 8a*S*)-2,3,4,7,8,8a-hexahydro-3,6,8,8-tetramethyl-1*H*-3a,7-methanoazulen-5-yl]- | Vertofix^{®} | 0.00008490 | 0.011319 |
| 71. | 131812-67-4 | 1,3-Dioxolane, 2,4-dimethyl-2-(5,6,7,8-tetrahydro-5,5,8,8-tetramethyl-2-naphthalenyl)- | Okoumal^{®} | 0.00007600 | 0.010133 |
| 72. | 106-02-5 | Oxacyclohexadecan-2-one | Exaltolide^{®} | 0.00006430 | 0.0085726 |
| 73. | 141773-73-1 | 1-Propanol, 2-[1-(3,3-dimethylcyclohexyl) ethoxy]-2-methyl-, 1-propanoate | Helvetolide ^{®} | 0.00005790 | 0.0077194 |
| 74. | 63314-79-4 | 5-Cyclopentadecen-1-one, 3-methyl- | Delta Muscenone | 0.00005650 | 0.0075327 |
| 75. | 77-42-9 | 2-Penten-1-ol, 2-methyl-5-[(1*S*,2*R*,4*R*)-2-methyl-3-methylenebicyclo[2.2.1]hep t-2-yl]-, (2*Z*)- | cis-beta-Santalol | 0.00004810 | 0.0064128 |
| 76. | 362467-67-2 | 2*H*-1,5-Benzodioxepin-3(4*H*)-one, 7-(3-methylbutyl)- | Azurone | 0.00004770 | 0.0063595 |
| 77. | 28371-99-5 | Ethanone, 1-(2,6,10-trimethyl-2,5,9-cyclododecatrien-1-yl)- | Trimofix O | 0.00004580 | 0.0061062 |
| 78. | 16223-63-5 | 1*H*-3a,6-Methanoazulene-3-methanol, octahydro-7,7-dimethyl-8-methylene-, (3*S*,3a*R*,6*R*,8a*S*)- | Khusimol | 0.00004400 | 0.0058662 |
| 79. | 10461-98-0 | Benzeneacetonitrile, α-cyclohexylidene- | Peonile | 0.00004290 | 0.0057195 |
| 80. | 90-17-5 | Benzenemethanol, α-(trichloromethyl)-, 1-acetate | Rosacetol | 0.00004240 | 0.0056529 |
| 81. | 50607-64-2 | Benzoic acid, 2-[(2-methylpentylidene)amino]-, methyl ester | Mevantraal | 0.00004070 | 0.0054262 |
| 82. | 29895-73-6 | 5-Hydroxy-2-benzyl-1,3-dioxane | Acetal CD | 0.00004050 | 0.0053996 |
| 83. | 94-47-3 | Benzoic acid, 2-phenylethyl ester | Phenyl Ethyl Benzoate | 0.00003480 | 0.0046396 |
| 84. | 3100-36-5 | Cyclohexadec-8-en-1-one | Globanone ^{®} | 0.00003310 | 0.0044130 |
| 85. | 37609-25-9 | 5-Cyclohexadecen-1-One | Ambretone | 0.00003310 | 0.0044130 |
| 86. | 66072-32-0 | Cyclohexanol, 4-(1,7,7-trimethylbicyclo[2.2.1] hept-2-yl)- | Iso Bornyl Cyclohexa nol | 0.00003010 | 0.0040130 |
| 87. | 31906-04-4 | 3-Cyclohexene-1-carboxaldehyde, 4-(4-hydroxy-4-methylpentyl)- | Lyral^{®} | 0.00002940 | 0.0039197 |
| 88. | 21145-77-7 | Ethanone, 1-(5,6,7,8-tetrahydro-3,5,5,6,8,8-hexamethyl-2-naphthalenyl)- | Musk Plus | 0.00002860 | 0.0038130 |
| 89. | 21145-77-7 | Ethanone, 1-(5,6,7,8-tetrahydro-3,5,5,6,8,8-hexamethyl-2-naphthalenyl)- | Fixolide | 0.00002860 | 0.0038130 |
| 90. | 22442-01-9 | 2-Cyclopentadecen-1-one, 3-methyl- | Muscenone | 0.00002770 | 0.0036930 |
| 91. | 109-29-5 | Oxacycloheptadecan-2-one | Silvanone Ci | 0.00002600 | 0.0034664 |
| 92. | 101-94-0 | Benzeneacetic acid, 4-methylphenyl ester | Para Cresyl Phenyl Acetate | 0.00002330 | 0.0031064 |
| 93. | 102-20-5 | Benzeneacetic acid, 2-phenylethyl ester | Phenyl Ethyl Phenyl Acetate | 0.00002300 | 0.0030664 |
| 94. | 118562-73-5 | Cyclododecaneethanol, β-methyl- | Hydroxya mbran | 0.00001800 | 0.0023998 |
| 95. | 103-41-3 | 2-Propenoic acid, 3-phenyl-, phenylmethyl ester | Benzyl Cinnamate | 0.00001050 | 0.0013999 |
| 96. | 4707-47-5 | Benzoic acid, 2,4-dihydroxy-3,6-dimethyl-, methyl ester | Veramoss | 0.00001050 | 0.0013999 |
| 97. | 183551-83-9 | Naphtho[2,1-b]furan-6(7*H*) -one, 8,9-dihydro-1,5,8-trimethyl-, (8*R*)- | Myrrhone | 0.00000977 | 0.0013026 |
| 98. | 102-17-0 | Benzeneacetic acid, (4-methoxyphenyl)methyl ester | Para Anisyl Phenyl Acetate | 0.00000813 | 0.0010839 |
| 99. | 120-11-6 | Benzene, 2-methoxy-1-(phenylmethoxy)-4-(1-propen-1-yl)- | Benzyl Iso Eugenol | 0.00000676 | 0.00090126 |
| 100. | 102-22-7 | Benzeneacetic acid, (2*E*)-3, 7-dimethyl-2,6-octadien-1-yl ester | Geranyl Phenylacet ate | 0.00000645 | 0.00085993 |
| 101. | 111879-80-2 | Oxacyclohexadec-12-en-2-one, (12*E*)- | Habanolide 100% | 0.00000431 | 0.00057462 |
| 102. | 87-22-9 | Benzoic acid, 2-hydroxy-, 2-phenylethyl ester | Phenyl Ethyl Salicylate | 0.00000299 | 0.00039863 |
| 103. | 78-37-5 | 2-Propenoic acid, 3-phenyl-, 1-ethenyl-1,5-dimethyl-4-hexen-1-yl ester | Linalyl Cinnamate | 0.00000174 | 0.00023198 |
| 104. | 28645-51-4 | Oxacycloheptadec-10-en-2-one | Ambrettoli de | 0.00000139 | 0.00018532 |
| 105. | 123-69-3 | Oxacycloheptadec-8-en-2-one, (8*Z*)- | Ambrettoli de | 0.00000136 | 0.00018132 |
| 106. | 3391-83-1 | 1,7-Dioxacycloheptadecan-8-one | Musk RI | 0.00000057 | 0.000075994 |
| 107. | 68527-79-7 | 7-Octen-2-ol, 8-(1*H*-indol-1-yl)-2,6-dimethyl- | Indolene | 0.000000445 | 0.000059328 |
| 108. | 89-43-0 | Methyl 2-[(7-hydroxy-3,7-dimethyloctylidene)amino] benzoate | Aurantinol | 0.0000000100 | 1.3332E-06 |
| 109. | 54982-83-1 | 1,4-Dioxacyclohexadecane-5,16-dione | Zenolide | 0.00000000834 | 1.1119E-06 |
| 110. | 105-95-3 | 1,4-Dioxacycloheptadecan e-5,17-dione | Ethylene Brassylate | 0.00000000313 | 4.1730E-07 |
| 111. | 3681-73-0 | Hexadecanoic acid, (2*E*)-3, 7-dimethyl-2,6-octadien-1-yl ester | Hexarose | 0.00000000300 | 3.9997E-07 |
| 112. | 4159-29-9 | Phenol, 4-[3-(benzoyloxy)-1-propen-1-yl]-2-methoxy- | Coniferyl benzoate | 0.00000000170 | 2.2665E-07 |
| 113. | 144761-91-1 | Benzoic acid, 2-[(1-hydroxy-3 -phenylbutyl) amino]-, methyl ester | Trifone DIPG | 0.00000000093 | 1.2399E-07 |

| | | | | | |
|---|---|---|---|---|---|
| *¹* Non-limiting examples of alternative qualities from various suppliers can be purchased under the following tradenames: Kharismal^{®} Super (IFF), Kharismal^{®} (IFF), Hedione^{®} HC (Firmenich), Parisone (Firmenich), Cepionate (Zenon), Super cepionate (Zenon), Claigeon^{®} (Zenon). * Vapor Pressures are acquired as described in the Test Methods Section. ** Origin: The low volatile fragrance materials may be obtained from one or more of the following companies: Firmenich (Geneva, Switzerland), Symrise AG (Holzminden, Germany), Givaudan (Argenteuil, France), IFF (Hazlet, New Jersey), Bedoukian (Danbury, Connecticut), Sigma Aldrich (St. Louis, Missouri), Millennium Speciality Chemicals (Olympia Fields, Illinois), Polarone International (Jersey City, New Jersey), and Aroma & Flavor Specialities (Danbury, Connecticut). | | | | | |

Exemplary low volatile fragrance materials selected from the group of Table 1 Low Volatile Fragrance Materials are preferred. However, it is understood by one skilled in the art that other low volatile fragrance materials, not recited in Table 1, would also fall within the scope of the present invention, so long as they have a vapor pressure < 0.133 Pa (0.001 Torr) at 25 °C.

Preferably, the compositions of the present invention, wherein: (i)(a) the low volatile fragrance material is selected from the group of Table 1 Low Volatile Fragrance Materials 1, 4-6, 8, 12-16, 18, 22-25, 27-28, 31, 34-37, 41, 45, 47, 53-56, 58, 61, 62, 64, 66, 69, 70-74, 76, 79, 81, 84-85, 90, 95, 100, 103, 105, 107-109, and mixtures thereof; and (ii) the substantially non-odorous fragrance modulator is selected from the group of Table 4 Substantially Non-Odorous Fragrance Modulators 1-4, and mixtures thereof.

Preferably, the compositions of the present invention, wherein: (i)(a) the low volatile fragrance material is selected from the group consisting of Table 1 Low Volatile Fragrance Materials 1-6, 8-9, 12-14, 16, 18-19, 23, 25-28, 31, 34-35, 37, 41-42, 45, 47-49, 54-56, 58-61, 64, 66, 70, 72-74, 76, 79-80, 82, 85-86, 96, 101, 104, 106, 108, 110 and mixtures thereof; and (ii) the substantially non-odorous fragrance modulator is selected from the group of Table 4 Substantially Non-Odorous Fragrance Modulators 5-7, and mixtures thereof.

Preferably, the compositions of the present invention, the low volatile fragrance material is selected from the group (as described herein above), and wherein this group of low volatile fragrance material has at least about 20 wt%, at least about 30 wt%, at least about 40 wt%, at least about 50 wt%, at least about 60 wt%, or at least about 70 wt%, relative to the total weight of the low volatile fragrance material.

### (ii) Moderate Volatile Fragrance Materials

Preferable examples of moderate volatile fragrance materials having a vapor pressure in the range of 13.33 Pa (0.1 Torr) to 0.133 Pa (0.001 Torr) at 25 °C are provided in Table 2 Moderate Volatile Fragrance Materials. Preferably, the moderate volatile fragrance material is selected from at least 1 material, or at least 2 materials, or at least 3 materials, or at least 5 materials, or at least 7 moderate volatile fragrance materials as disclosed in Table 2.

**Table 2 - Moderate Volatile Fragrance Materials**

| **No.** | **CAS Number** | **IUPAC Name** | **Common Name**** | **Vapor Pressure (Torr at 25 °C)*** | **Vapor Pressure (Pa at 25 °C)** |
|---|---|---|---|---|---|
| 1. | 24168-70-5 | Pyrazine, 2-methoxy-3-(1-methylpropyl)- | Methoxyisobutyl pyrazine | 0.09950000 | 13.266 |
| 2. | 89-79-2 | Cyclohexanol, 5-methyl-2-(1-methylethenyl)-, (1*R*, 2*S,*5*R*)*-* | Iso-Pulegol | 0.09930000 | 13.239 |
| 3. | 112-12-9 | 2-Undecanone | Methyl Nonyl Ketone | 0.09780000 | 13.039 |
| 4. | 103-05-9 | Benzenepropanol, α, α-dimethyl- | Phenyl Ethyl Dimethyl Carbinol | 0.09770000 | 13.026 |
| 5. | 125-12-2 | Bicyclo[2.2.1]heptan-2-ol, 1,7,7-trimethyl-, 2-acetate, (1*R*,2*R*,4*R*) -rel- | Iso Bornyl Acetate | 0.09590000 | 12.786 |
| 6. | 78-70-6 | 1,6-Octadien-3-ol, 3, 7-dimethyl- | Linalool | 0.09050000 | 12.066 |
| 7. | 101-97-3 | Benzeneacetic acid, ethyl ester | Ethyl Phenyl Acetate | 0.08970000 | 11.959 |
| 8. | 100-86-7 | Benzeneethanol, α,α-dimethyl- | Dimethyl Benzyl Carbinol | 0.08880000 | 11.839 |
| 9. | 188570-78-7 | Cyclopropanecarboxy lic acid, (3*Z*)-3-hexen-1-yl ester | Montaverdi | 0.08640000 | 11.519 |
| 10. | 67634-25-7 | 3-Cyclohexene-1-methanol, 3,5-dimethyl-, 1-acetate | Floralate | 0.08500000 | 11.332 |
| 11. | 112-44-7 | Undecanal | Undecyl Aldehyde | 0.08320000 | 11.092 |
| 12. | 32669-00-4 | Ethanone, 1-(3-cycloocten-1-yl)- | Tanaisone^{®} | 0.08150000 | 10.866 |
| 13. | 98-53-3 | Cyclohexanone, 4-(1, 1-dimethylethyl)- | Patchi | 0.07780000 | 10.372 |
| 14. | 35854-86-5 | 6-Nonen-1-ol, (6*Z*)- | cis-6-None-1-ol | 0.07770000 | 10.359 |
| 15. | 5331-14-6 | Benzene, (2-butoxyethyl)- | Butyl phenethyl ether | 0.07760000 | 10.346 |
| 16. | 80-57-9 | Bicyclo[3.1.1]hept-3-en-2-one, 4,6,6-trimethyl- | Verbenone | 0.07730000 | 10.306 |
| 17. | 22471-55-2 | Cyclohexanecarboxyl ic acid, 2,2,6-trimethyl-, ethyl ester, (1*R*,6*S*)-rel- | Thesaron | 0.07670000 | 10.226 |
| 18. | 60-12-8 | Benzeneethanol | Phenethyl alcohol | 0.07410000 | 9.8792 |
| 19. | 106-26-3 | 2,6-Octadienal, 3,7-dimethyl-, (2*Z*)- | Neral | 0.07120000 | 9.4926 |
| 20. | 5392-40-5 | 2,6-Octadienal, 3,7-dimethyl- | Citral | 0.07120000 | 9.4926 |
| 21. | 89-48-5 | Cyclohexanol, 5-methyl-2-(1-methylethyl)-, 1-acetate, (1*R*,2*S*,5*R*)-rel- | Menthyl Acetate | 0.07070000 | 9.4259 |
| 22. | 119-36-8 | Benzoic acid, 2-hydroxy-, methyl ester | Methyl salicylate | 0.07000000 | 9.3326 |
| 23. | 4180-23-8 | Benzene, 1-methoxy-4-(1*E*)-1-propen-1-yl- | Anethol | 0.06870000 | 9.1592 |
| 24. | 7549-37-3 | 2,6-Octadiene, 1,1-dimethoxy-3,7-dimethyl- | Citral Dimethyl Acetal | 0.06780000 | 9.0393 |
| 25. | 25225-08-5 | Cyclohexanemethano 1, α,3,3-trimethyl-, 1-formate | Aphermate | 0.06780000 | 9.0393 |
| 26. | 3913-81-3 | 2-Decenal, (2*E*)- | 2-Decene-1-al | 0.06740000 | 8.9859 |
| 27. | 15373-31-6 | 3-Cyclopentene-1-acetonitrile, 2,2,3-trimethyl- | Cantryl^{®} | 0.06700000 | 8.9326 |
| 28. | 6485-40-1 | 2-Cyclohexen-1-one, 2-methyl-5-(1-methylethenyl)-, (5*R*) | Laevo carvone | 0.06560000 | 8.7459 |
| 29. | 16587-71-6 | Cyclohexanone, 4-(1, 1-dimethylpropyl)- | Orivone | 0.06490000 | 8.6526 |
| 30. | 62406-73-9 | 6,10-Dioxaspiro[4.5]decan e, 8,8-dimethyl-7-(1-methylethyl)- | Opalal CI | 0.06290000 | 8.3860 |
| 31. | 3720-16-9 | 2-Cyclohexen-1-one, 3-methyl-5-propyl- | Livescone | 0.06270000 | 8.3593 |
| 32. | 13816-33-6 | Benzonitrile, 4-(1-methylethyl)- | Cumin Nitrile | 0.06230000 | 8.3060 |
| 33. | 67019-89-0 | 2,6-Nonadienenitrile | Violet Nitrile | 0.06200000 | 8.2660 |
| 34. | 53398-85-9 | Butanoic acid, 2-methyl-, (3Z)-3-hexen-1-yl ester | cis-3-Hexenyl Alpha Methyl Butyrate | 0.06130000 | 8.1727 |
| 35. | 208041-98-9 | n/a | Jasmonitrile | 0.05920000 | 7.8927 |
| 36. | 16510-27-3 | Benzene, 1-(cyclopropylmethyl)-4-methoxy- | Toscanol | 0.05870000 | 7.8260 |
| 37. | 111-80-8 | 2-Nonynoic acid, methyl ester | Methyl Octine Carbonate | 0.05680000 | 7.5727 |
| 38. | 103-45-7 | Acetic acid, 2-phenylethyl ester | Phenyl Ethyl Acetate | 0.05640000 | 7.5194 |
| 39. | -- | -- | -- | -- | -- |
| 40. | 2550-26-7 | 2-Butanone, 4-phenyl- | Benzyl Acetone | 0.05570000 | 7.4261 |
| 41. | 13491-79-7 | Cyclohexanol, 2-(1,1-dimethylethyl)- | Verdol | 0.05430000 | 7.2394 |
| 42. | 7786-44-9 | 2,6-Nonadien-1-ol | 2,6-Nonadien-1-ol | 0.05370000 | 7.1594 |
| 43. | 103-28-6 | Propanoic acid, 2-methyl-, phenylmethyl ester | Benzyl Iso Butyrate | 0.05130000 | 6.8394 |
| 44. | 104-62-1 | Formic acid, 2-phenylethyl ester | Phenyl Ethyl Formate | 0.05050000 | 6.7328 |
| 45. | 28462-85-3 | Bicyclo[2.2.1]heptan-2-ol, 1,2,3,3-tetramethyl-, (1*R*,2*R*, 4*S*)-rel- | Humus Ether | 0.04870000 | 6.4928 |
| 46. | 122-03-2 | Benzaldehyde, 4-(1-methylethyl)- | Cuminic Aldehyde | 0.04820000 | 6.4261 |
| 47. | 358331-95-0 | 2,5-Octadien-4-one, 5,6,7-trimethyl-, (2*E*) | Pomarose | 0.04810000 | 6.4128 |
| 48. | 562-74-3 | 3-Cyclohexen-1-ol, 4-methyl-1-(1-methylethyl)- | Terpinenol-4 | 0.04780000 | 6.3728 |
| 49. | 68527-77-5 | 3-Cyclohexene-1-methanol, 2,4,6-trimethyl- | Isocyclogeraniol | 0.04640000 | 6.1862 |
| 50. | 35852-46-1 | Pentanoic acid, (3*Z*)-3-hexen-1-yl ester | Cis-3-Hexenyl Valerate | 0.04580000 | 6.1062 |
| 51. | 2756-56-1 | Bicyclo[2.2.1]heptan-2-ol, 1,7,7-trimethyl-, 2-propanoate, (1*R*,2*R*, 4*R*)-rel- | Iso Bornyl Propionate | 0.04540000 | 6.0528 |
| 52. | 14374-92-6 | Benzene, 1-methyl-4-(1-methylethyl)-2-(1-propen-1-yl)- | Verdoracine | 0.04460000 | 5.9462 |
| 53. | 6784-13-0 | 3-Cyclohexene-1-propanal, β,4-dimethyl- | Limonenal | 0.04380000 | 5.8395 |
| 54. | 8000-41-7 | 2-(4-methyl-1-cyclohex-3-enyl)propan-2-ol | Alpha Terpineol | 0.04320000 | 5.7595 |
| 55. | 41884-28-0 | 1-Hexanol, 5-methyl-2-(1-methylethyl)-, (2*R*)- | Tetrahydro Lavandulol | 0.04230000 | 5.6395 |
| 56. | 22457-23-4 | 3-Heptanone, 5-methyl-, oxime | Stemone^{®} | 0.04140000 | 5.5195 |
| 57. | 104-50-7 | 2(3*H*)-Furanone, 5-butyldihydro- | Gamma Octalactone | 0.04080000 | 5.4396 |
| 58. | 143-08-8 | 1-Nonanol | Nonyl Alcohol | 0.04070000 | 5.4262 |
| 59. | 3613-30-7 | Octanal, 7-methoxy-3,7-dimethyl- | Methoxycitronell al | 0.04020000 | 5.3596 |
| 60. | 67634-00-8 | Acetic acid, 2-(3-methylbutoxy)-, 2-propen-1-yl ester | Allyl Amyl Glycolate | 0.04000000 | 5.3329 |
| 61. | 464-45-9 | Bicyclo[2.2.1]heptan-2-ol, 1,7,7-trimethyl-, (1*S,*2*R,*4*S*)*-* | 1-Borneol | 0.03980000 | 5.3062 |
| 62. | 124-76-5 | Bicyclo[2.2.1]heptan-2-ol, 1,7,7-trimethyl-, (1*R,*2*R,*4*R*)-rel- | 1.7.7-Trimethyl-Bicyclo-1.2.2-Heptanol-2 | 0.03980000 | 5.3062 |
| 63. | 67874-72-0 | Cyclohexanol, 2-(1,1-dimethylpropyl)-, 1-acetate | Coniferan | 0.03980000 | 5.3062 |
| 64. | 80-26-2 | 3-Cyclohexene-1-methanol, α,α,4-trimethyl-, 1-acetate | Terpinyl Acetate | 0.03920000 | 5.2262 |
| 65. | 498-81-7 | Cyclohexanemethano 1, α,α,4-trimethyl- | Dihydro Terpineol | 0.03920000 | 5.2262 |
| 66. | 112-45-8 | 10-Undecenal | Undecylenic aldehyde | 0.03900000 | 5.1996 |
| 67. | 35044-57-6 | 2,4-Cyclohexadiene-1-carboxylic acid, 2,6,6-trimethyl-, ethyl ester | Ethyl Safranate | 0.03880000 | 5.1729 |
| 68. | 106-21-8 | 1-Octanol, 3,7-dimethyl- | Dimethyl Octanol | 0.03860000 | 5.1462 |
| 69. | 84560-00-9 | Cyclopentanol, 2-pentyl- | Cyclopentol | 0.03790000 | 5.0529 |
| 70. | 82461-14-1 | Furan, tetrahydro-2,4-dimethyl-4-phenyl- | Rhubafuran^{®} | 0.03780000 | 5.0396 |
| 71. | 56011-02-0 | Benzene, [2-(3-methylbutoxy)ethyl]- | Phenyl Ethyl Isoamyl Ether | 0.03690000 | 4.9196 |
| 72. | 103-37-7 | Butanoic acid, phenylmethyl ester | Benzyl Butyrate | 0.03660000 | 4.8796 |
| 73. | 6378-65-0 | Hexyl hexanoate | Hexyl hexanoate | 0.03490000 | 4.6530 |
| 74. | 118-61-6 | Benzoic acid, 2-hydroxy-, ethyl ester | Ethyl salicylate | 0.03480000 | 4.6396 |
| 75. | 98-52-2 | Cyclohexanol, 4-(1,1-dimethylethyl)- | Patchon | 0.03480000 | 4.6396 |
| 76. | 115-99-1 | 1,6-Octadien-3-ol, 3, 7-dimethyl-, 3-formate | Linalyl Formate | 0.03440000 | 4.5863 |
| 77. | 112-54-9 | Dodecanal | Lauric Aldehyde | 0.03440000 | 4.5863 |
| 78. | 53046-97-2 | 3,6-Nonadien-1-ol, (3*Z*,6*Z*)- | 3,6 Nonadien-1-ol | 0.03360000 | 4.4796 |
| 79. | 76649-25-7 | 3,6-Nonadien-1-ol | 3,6-Nonadien-1-ol | 0.03360000 | 4.4796 |
| 80. | 141-25-3 | 3,7-Dimethyloct-6-en-1-ol | Rhodinol | 0.03290000 | 4.3863 |
| 81. | 1975-78-6 | Decanenitrile | Decanonitrile | 0.03250000 | 4.3330 |
| 82. | 2216-51-5 | Cyclohexanol, 5-methyl-2-(1-methylethyl)-, (1*R*, 2*S,*5*R*)*-* | L-Menthol | 0.03230000 | 4.3063 |
| 83. | 3658-77-3 | 4-hydroxy-2,5-dimethylfuran-3-one | Pineapple Ketone | 0.03200000 | 4.2663 |
| 84. | 103-93-5 | Propanoic acid, 2-methyl-, 4-methylphenyl ester | Para Cresyl iso-Butyrate | 0.03120000 | 4.1597 |
| 85. | 24717-86-0 | Propanoic acid, 2-methyl-, (1*R*,2*S*,4*R*)-1,7,7-trimethylbicyclo[2.2 .1]hept-2-yl ester, rel- | Abierate | 0.03110000 | 4.1463 |
| 86. | 67845-46-9 | Acetaldehyde, 2-(4-methylphenoxy)- | Aldehyde XI | 0.03090000 | 4.1197 |
| 87. | 67883-79-8 | 2-Butenoic acid, 2-methyl-, (3*Z*)-3-hexen-1-yl ester, (2*E*) | Cis-3-Hexenyl Tiglate | 0.03060000 | 4.0797 |
| 88. | 33885-51-7 | Bicyclo [3.1.1]hept-2-ene-2-propanal, 6,6-dimethyl- | Pino Acetaldehyde | 0.03040000 | 4.0530 |
| 89. | 105-85-1 | 6-Octen-1-ol, 3,7-dimethyl-, 1-formate | Citronellyl Formate | 0.03000000 | 3.9997 |
| 90. | 70214-77-6 | 2-Nonanol, 6,8-dimethyl- | Nonadyl | 0.03010000 | 4.0130 |
| 91. | 215231-33-7 | Cyclohexanol, 1-methyl-3-(2-methylpropyl)- | Rossitol | 0.02990000 | 3.9863 |
| 92. | 120-72-9 | 1*H*-Indole | Indole | 0.02980000 | 3.9730 |
| 93. | 2463-77-6 | 2-Undecenal | 2-Undecene-1-al | 0.02970000 | 3.9597 |
| 94. | 675-09-2 | 2*H*-Pyran-2-one, 4,6-dimethyl- | Levistamel | 0.02940000 | 3.9197 |
| 95. | 98-55-5 | 3-Cyclohexene-1-methanol, α,α,4-trimethyl- | Alpha-Terpineol | 0.02830000 | 3.7730 |
| 96. | 81786-73-4 | 3-Hepten-2-one, 3,4, 5,6,6-pentamethyl-, (3*Z*)- | Koavone | 0.02750000 | 3.6664 |
| 97. | 122-97-4 | Benzenepropanol | Phenyl Propyl Alcohol | 0.02710000 | 3.6130 |
| 98. | 39212-23-2 | 2(3*H*)-Furanone, 5-butyldihydro-4-methyl- | Methyl Octalactone | 0.02700000 | 3.5997 |
| 99. | 53767-93-4 | 7-Octen-2-ol, 2,6-dimethyl-, 2-acetate | Dihydro Terpinyl Acetate | 0.02690000 | 3.5864 |
| 100. | 35044-59-8 | 1,3-Cyclohexadiene-1-carboxylic acid, 2, 6,6-trimethyl-, ethyl ester | Ethyl Safranate | 0.02660000 | 3.5464 |
| 101. | 104-55-2 | 2-Propenal, 3-phenyl- | Cinnamic Aldehyde | 0.02650000 | 3.5330 |
| 102. | 144-39-8 | 1,6-Octadien-3-ol, 3, 7-dimethyl-, 3-propanoate | Linalyl Propionate | 0.02630000 | 3.5064 |
| 103. | 61931-80-4 | 1,6-Nonadien-3-ol, 3, 7-dimethyl-, 3-acetate | 3,7-Dimethyl-1,6-nonadien-3-yl acetate | 0.02630000 | 3.5064 |
| 104. | 102-13-6 | Benzeneacetic acid, 2-methylpropyl ester | Iso Butyl Phenylacetate | 0.02630000 | 3.5064 |
| 105. | 65443-14-3 | Cyclopentanone, 2,2, 5-trimethyl-5-pentyl- | Veloutone | 0.02610000 | 3.4797 |
| 106. | 141-12-8 | 2,6-Octadien-1-ol, 3, 7-dimethyl-, 1-acetate, (2*Z*)- | Neryl Acetate | 0.02560000 | 3.4131 |
| 107. | 105-87-3 | 2,6-Octadien-1-ol, 3, 7-dimethyl-, 1-acetate, (2*E*)- | Geranyl acetate | 0.02560000 | 3.4131 |
| 108. | 68141-17-3 | Undecane, 1,1-dimethoxy-2-methyl- | Methyl Nonyl Acetaldehyde Dimethyl Acetal | 0.02550000 | 3.3997 |
| 109. | 2206-94-2 | Benzenemethanol, α-methylene-, 1-acetate | Indocolore | 0.02550000 | 3.3997 |
| 110. | 10528-67-3 | Cyclohexanepropanol , α-methyl- | Cyclohexylmagn ol | 0.02550000 | 3.3997 |
| 111. | 123-11-5 | Benzaldehyde, 4-methoxy- | Anisic Aldehyde | 0.02490000 | 3.3197 |
| 112. | 57576-09-7 | Cyclohexanol, 5-methyl-2-(1-methylethenyl)-, 1-acetate, (1*R*,2*S*,5*R*)- | Iso Pulegol Acetate | 0.02480000 | 3.3064 |
| 113. | 51566-62-2 | 6-Octenenitrile, 3,7-dimethyl- | Citronellyl Nitrile | 0.02470000 | 3.2931 |
| 114. | 60335-71-9 | 2*H*-Pyran, 3,6-dihydro-4-methyl-2-phenyl- | Rosyrane Super | 0.02470000 | 3.2931 |
| 115. | 30385-25-2 | 6-Octen-2-ol, 2,6-dimethyl- | Dihydromyrcenol | 0.02440000 | 3.2531 |
| 116. | 101-84-8 | Benzene, 1,1'-oxybis- | Diphenyl Oxide | 0.02230000 | 2.9731 |
| 117. | 136-60-7 | Benzoic acid, butyl ester | Butyl Benzoate | 0.02170000 | 2.8931 |
| 118. | 93939-86-7 | 5,8-Methano-2*H*-1-benzopyran, 6-ethylideneoctahydro- | Rhubo flor | 0.02120000 | 2.8264 |
| 119. | 83926-73-2 | Cyclohexanepropanol , α,α-dimethyl- | Coranol | 0.02100000 | 2.7998 |
| 120. | 125109-85-5 | Benzenepropanal, β-methyl-3-(1-methylethyl)- | Florhydral | 0.02070000 | 2.7598 |
| 121. | 104-21-2 | Benzenemethanol, 4-methoxy-, 1-acetate | Anisyl Acetate | 0.02050000 | 2.7331 |
| 122. | 1365-19-1 | 2-Furanmethanol, 5-ethenyltetrahydro-α,α,5-trimethyl- | Linalool Oxide | 0.02050000 | 2.7331 |
| 123. | 137-03-1 | Cyclopentanone, 2-heptyl- | Frutalone | 0.02040000 | 2.7198 |
| 124. | 2563-07-7 | Phenol, 2-ethoxy-4-methyl- | Ultravanil | 0.02030000 | 2.7064 |
| 125. | 1128-08-1 | 2-Cyclopenten-1-one, 3-methyl-2-pentyl- | Dihydrojasmone | 0.02020000 | 2.6931 |
| 126. | 7493-57-4 | Benzene, [2-(1-propoxyethoxy)ethyl]- | Acetaldehyde | 0.01990000 | 2.6531 |
| 127. | 141-25-3 | 7-Octen-1-ol, 3,7-dimethyl- | Rhodinol | 0.01970000 | 2.6265 |
| 128. | 216970-21-7 | Bicyclo[4.3.1]decane, 3-methoxy-7,7-dimethyl-10-methylene- | 3-Methoxy-7,7-dimethyl-10-methylenebicycl o[4.3.1]decane | 0.01960000 | 2.6131 |
| 129. | 319002-92-1 | Propanoic acid, 2-(1, 1-dimethylpropoxy)-, propyl ester, (2*S*)- | Sclareolate^{®} | 0.01960000 | 2.6131 |
| 130. | 85-91-6 | Benzoic acid, 2-(methylamino)-, methyl ester | Dimethyl anthranilate | 0.01930000 | 2.5731 |
| 131. | 13828-37-0 | Cyclohexanemethano l, 4-(1-methylethyl)-, cis- | Mayol | 0.01920000 | 2.5598 |
| 132. | 26330-65-4 | (*E*)-6-ethyl-3-methyloct-6-en-1-ol | Super Muguet | 0.01850000 | 2.4665 |
| 133. | 7540-51-4 | 6-Octen-1-ol, 3,7-dimethyl-, (3*S*)- | L-Citronellol | 0.01830000 | 2.4398 |
| 134. | 106-22-9 | 6-Octen-1-ol, 3,7-dimethyl- | Citronellol | 0.01830000 | 2.4398 |
| 135. | 543-39-5 | 7-Octen-2-ol, 2-methyl-6-methylene- | Myrcenol | 0.01820000 | 2.4265 |
| 136. | 7775-00-0 | Benzenepropanal, 4-(1-methylethyl)- | Cyclemax | 0.01820000 | 2.4265 |
| 137. | 18479-54-4 | 4,6-Octadien-3-ol, 3, 7-dimethyl- | Muguol | 0.01800000 | 2.3998 |
| 138. | 29214-60-6 | Octanoic acid, 2-acetyl-, ethyl ester | Gelsone | 0.01790000 | 2.3865 |
| 139. | 1209-61-6 | 5-Oxatricyclo[8.2.0.04, 6]dodecane, 4,9,12, 12-tetramethyl- | Tobacarol | 0.01730000 | 2.3065 |
| 140. | 57934-97-1 | 2-Cyclohexene-1-carboxylic acid, 2-ethyl-6,6-dimethyl-, ethyl ester | Givescone | 0.01710000 | 2.2798 |
| 141. | 14901-07-6 | 3-Buten-2-one, 4-(2, 6,6-trimethyl-1-cyclohexen-1-yl)-, (3*E*)*-* | Beta-Ionone | 0.01690000 | 2.2531 |
| 142. | 64001-15-6 | 4,7-Methano-1*H-*inden-5-ol, octahydro-, 5-acetate | Dihydro Cyclacet | 0.01630000 | 2.1732 |
| 143. | 95-41-0 | 2-Cyclopenten-1-one, 2-hexyl- | Iso Jasmone T | 0.01600000 | 2.1332 |
| 144. | 134-20-3 | Benzoic acid, 2-amino-, methyl ester | Methyl Anthranilate | 0.01580000 | 2.1065 |
| 145. | 100-06-1 | Ethanone, 1-(4-methoxyphenyl)- | Para Methoxy Acetophenone | 0.01550000 | 2.0665 |
| 146. | 105-86-2 | 2,6-Octadien-1-ol, 3, 7-dimethyl-, 1-formate, (2*E*)- | Geranyl Formate | 0.01540000 | 2.0532 |
| 147. | 154171-77-4 | Spiro[1,3-dioxolane-2,8'(5'*H*)-[2*H*-2,4a] methanonaphthalene], hexahydro-1',1',5',5'-tetramethyl-, (2'*S*, 4'aS,8'a*S*)- (9CI) | Ysamber K^{®} | 0.01470000 | 1.9598 |
| 148. | 154171-76-3 | Spiro[1,3-dioxolane-2,8'(5*'H*)-[2*H-*2,4a]methanonaphtha lene], hexahydro-1',1',5',5'-tetramethyl- | Ysamber | 0.01470000 | 1.9598 |
| 149. | 127-41-3 | 3-Buten-2-one, 4-(2, 6,6-trimethyl-2-cyclohexen-1-yl)-, (3*E*)*-* | Alpha-Ionone | 0.01440000 | 1.9198 |
| 150. | 151-05-3 | Benzeneethanol, α,α-dimethyl-, 1-acetate | Dimethyl Benzyl Carbinyl Acetate | 0.01390000 | 1.8532 |
| 151. | 2500-83-6 | 4,7-Methano-1*H-*inden-5-ol, 3a,4,5,6,7, 7a-hexahydro-, 5-acetate | Flor Acetate | 0.01370000 | 1.8265 |
| 152. | 150-84-5 | 6-Octen-1-ol, 3,7-dimethyl-, 1-acetate | Citronellyl acetate | 0.01370000 | 1.8265 |
| 153. | 30310-41-9 | 2*H*-Pyran, tetrahydro-2-methyl-4-methylene-6-phenyl- | Pelargene | 0.01350000 | 1.7999 |
| 154. | 68845-00-1 | Bicyclo[3.3.1] nonane, 2-ethoxy-2,6, 6-trimethyl-9-methylene- | Boisiris | 0.01350000 | 1.7999 |
| 155. | 106-24-1 | 2,6-Octadien-1-ol, 3, 7-dimethyl-, (2*E*)- | Geraniol | 0.01330000 | 1.7732 |
| 156. | 106-25-2 | 2,6-Octadien-1-ol, 3, 7-dimethyl-, (2*Z*)- | Nerol | 0.01330000 | 1.7732 |
| 157. | 75975-83-6 | Bicyclo[7.2.0]undec-4-ene, 4,11,11-trimethyl-8-methylene-, (1*R*,4*E*, 9*S*)- | Vetyvenal | 0.01280000 | 1.7065 |
| 158. | 19870-74-7 | 1*H*-3a,7-Methanoazulene, octahydro-6-methoxy-3,6,8,8-tetramethyl-, (3*R*,3a*S*, 6*S*,7*R*,8a*S*)- | Cedryl methyl ether | 0.01280000 | 1.7065 |
| 159. | 87-44-5 | Bicyclo[7.2.0]undec-4-ene, 4,11,11-trimethyl-8-methylene-, (1*R*,4*E*, 9*S*)- | Caryophyllene Extra | 0.01280000 | 1.7065 |
| 160. | 54440-17-4 | 1*H*-Inden-1-one, 2,3-dihydro-2,3,3-trimethyl- | Safraleine | 0.01260000 | 1.6799 |
| 161. | 110-98-5 | 2-Propanol, 1,1'-oxybis- | Dipropylene Glycol | 0.01250000 | 1.6665 |
| 162. | 41890-92-0 | 2-Octanol, 7-methoxy-3,7-dimethyl- | Osyrol^{®} | 0.01250000 | 1.6665 |
| 163. | 71077-31-1 | 4,9-Decadienal, 4,8-dimethyl- | Floral Super | 0.01230000 | 1.6399 |
| 164. | 65-85-0 | Benzoic Acid | Benzoic Acid | 0.01220000 | 1.6265 |
| 165. | 61444-38-0 | 3-Hexenoic acid, (3*Z*) -3-hexen-1-yl ester, (3*Z*)- | cis-3-hexenyl-cis-3-hexenoate | 0.01220000 | 1.6265 |
| 166. | 116044-44-1 | Bicyclo[2.2.1]hept-5-ene-2-carboxylic acid, 3-(1-methylethyl)-, ethyl ester, (1*R,*2*S,*3*S,*4*S*)-rel- | Herbanate | 0.01210000 | 1.6132 |
| 167. | 104-54-1 | 2-Propen-1-ol, 3-phenyl- | Cinnamic Alcohol | 0.01170000 | 1.5599 |
| 168. | 78-35-3 | Propanoic acid, 2-methyl-, 1-ethenyl-1, 5-dimethyl-4-hexen-1-yl ester | Linalyl Isobutyrate | 0.01170000 | 1.5599 |
| 169. | 23495-12-7 | Ethanol, 2-phenoxy-, 1-propanoate | Phenoxy Ethyl Propionate | 0.01130000 | 1.5065 |
| 170. | 103-26-4 | 2-Propenoic acid, 3-phenyl-, methyl ester | Methyl Cinnamate | 0.01120000 | 1.4932 |
| 171. | 67634-14-4 | Benzenepropanal, 2-ethyl-α,α-dimethyl- | Florazon (ortho-isomer) | 0.01110000 | 1.4799 |
| 172. | 5454-19-3 | Propanoic acid, decyl ester | N-Decyl Propionate | 0.01100000 | 1.4665 |
| 173. | 93-16-3 | Benzene, 1,2-dimethoxy-4-(1-propen-1-yl)- | Methyl Iso Eugenol | 0.01100000 | 1.4665 |
| 174. | 81782-77-6 | 3-Decen-5-ol, 4-methyl- | 4-Methyl-3-decen-5-ol | 0.01070000 | 1.4265 |
| 175. | 67845-30-1 | Bicyclo[2.2.2]oct-5-ene-2-carboxaldehyde, 6-methyl-8-(1-methylethyl)- | Maceal | 0.01060000 | 1.4132 |
| 176. | 97-53-0 | Phenol, 2-methoxy-4-(2-propen-1-yl)- | Eugenol | 0.01040000 | 1.3866 |
| 177. | 120-57-0 | 1,3-Benzodioxole-5-carboxaldehyde | Heliotropin | 0.01040000 | 1.3866 |
| 178. | 93-04-9 | Naphthalene, 2-methoxy- | Beta Naphthyl Methyl Ether Extra 99 | 0.01040000 | 1.3866 |
| 179. | 4826-62-4 | 2-Dodecenal | 2 Dodecene-1-al | 0.01020000 | 1.3599 |
| 180. | 20407-84-5 | 2-Dodecenal, (2*E*)- | Aldehyde Mandarin | 0.01020000 | 1.3599 |
| 181. | 5462-06-6 | Benzenepropanal, 4-methoxy-α-methyl- | Canthoxal | 0.01020000 | 1.3599 |
| 182. | 94-60-0 | 1,4-Cyclohexanedicarbox ylic acid, 1,4-dimethyl ester | Dimethyl 1,4-cyclohexanedicar boxylate | 0.01020000 | 1.3599 |
| 183. | 57378-68-4 | 2-Buten-1-one, 1-(2, 6,6-trimethyl-3-cyclohexen-1-yl)- | delta-Damascone | 0.01020000 | 1.3599 |
| 184. | 17283-81-7 | 2-Butanone, 4-(2,6,6-trimethyl-1-cyclohexen-1-yl)- | Dihydro Beta Ionone | 0.01020000 | 1.3599 |
| 185. | 1885-38-7 | 2-Propenenitrile, 3-phenyl-, (2*E*)- | Cinnamalva | 0.01010000 | 1.3466 |
| 186. | 103-48-0 | Propanoic acid, 2-methyl-, 2-phenylethyl ester | Phenyl Ethyl Iso Butyrate | 0.00994000 | 1.3252 |
| 187. | 488-10-8 | 2-Cyclopenten-1-one, 3-methyl-2-(2*Z*)-2-penten-1-yl- | Cis Jasmone | 0.00982000 | 1.3092 |
| 188. | 7492-67-3 | Acetaldehyde, 2-[(3, 7-dimethyl-6-octen-1-yl)oxy]- | Citronellyloxyac etaldehyde | 0.00967000 | 1.2892 |
| 189. | 68683-20-5 | 1-Cyclohexene-1-ethanol, 4-(1-methylethyl)-, 1-formate | Iso Bergamate | 0.00965000 | 1.2866 |
| 190. | 3025-30-7 | 2,4-Decadienoic acid, ethyl ester, (2*E*,4*Z*)- | Ethyl 2,4-Decadienoate | 0.00954000 | 1.2719 |
| 191. | 103-54-8 | 2-Propen-1-ol, 3-phenyl-, 1-acetate | Cinnamyl Acetate | 0.00940000 | 1.2532 |
| 192. | 18127-01-0 | Benzenepropanal, 4-(1,1-dimethylethyl)- | Bourgeonal | 0.00934000 | 1.2452 |
| 193. | 3738-00-9 | Naphtho[2,1-b]furan, dodecahydro-3a,6,6,9a-tetramethyl- | Ambrox^{®} or Cetalox^{®} or Synambran | 0.00934000 | 1.2452 |
| 194. | 51519-65-4 | 1,4-Methanonaphthalen-5(1*H*)-one, 4,4a,6,7,8, 8a-hexahydro- | Tamisone | 0.00932000 | 1.2426 |
| 195. | 148-05-1 | Dodecanoic acid, 12-hydroxy-, λ-lactone (6CI,7CI); 1,12- | Dodecalactone | 0.00931000 | 1.2412 |
| 196. | 6790-58-5 | (3a*R*,5a*S*,9a*S*,9b*R*)-3a,6,6,9a-tetramethyl-2,4,5,5a,7,8,9,9b-octahydro-1*H-*benzo[e][1]benzofura n | Ambronat^{®} or Ambroxan^{®} | 0.00930000 | 1.2399 |
| 197. | 86-26-0 | 1,1'-Biphenyl, 2-methoxy- | Methyl Diphenyl Ether | 0.00928000 | 1.2372 |
| 198. | 68738-94-3 | 2-Naphthalenecarboxal dehyde, octahydro-8,8-dimethyl | Cyclomyral^{®} | 0.00920000 | 1.2266 |
| 199. | 2705-87-5 | Cyclohexanepropanoi c acid, 2-propen-1-yl ester | Allyl Cyclohexane Propionate | 0.00925000 | 1.2332 |
| 200. | 7011-83-8 | 2(3*H*)-Furanone, 5-hexyldihydro-5-methyl- | Lactojasmone^{®} | 0.00885000 | 1.1799 |
| 201. | 61792-11-8 | 2,6-Nonadienenitrile, 3,7-dimethyl- | Lemonile^{®} | 0.00884000 | 1.1786 |
| 202. | 692-86-4 | 10-Undecenoic acid, ethyl ester | Ethyl Undecylenate | 0.00882000 | 1.1759 |
| 203. | 103-95-7 | Benzenepropanal, α-methyl-4-(1-methylethyl)- | Cymal | 0.00881000 | 1.1746 |
| 204. | 13019-22-2 | 9-Decen-1-ol | Rosalva | 0.00879000 | 1.1719 |
| 205. | 94201-19-1 | 1-Oxaspiro[4.5] decan-2-one, 8-methyl- | Methyl Laitone 10% TEC | 0.00872000 | 1.1626 |
| 206. | 104-61-0 | 2(3*H*)-Furanone, dihydro-5-pentyl- | γ-Nonalactone | 0.00858000 | 1.1439 |
| 207. | 706-14-9 | 2(3*H*)-Furanone, 5-hexyldihydro- | y -Decalactone | 0.00852000 | 1.1359 |
| 208. | 24720-09-0 | 2-Buten-1-one, 1-(2, 6,6-trimethyl-2-cyclohexen-1-yl)-, (2*E*)- | α-Damascone | 0.00830000 | 1.1066 |
| 209. | 39872-57-6 | 2-Buten-1-one, 1-(2, 4,4-trimethyl-2-cyclohexen-1-yl)-, (2*E*)- | Isodamascone | 0.00830000 | 1.1066 |
| 210. | 705-86-2 | 2*H*-Pyran-2-one, tetrahydro-6-pentyl- | Decalactone | 0.00825000 | 1.0999 |
| 211. | 67634-15-5 | Benzenepropanal, 4-ethyl-α,α-dimethyl- | Floralozone | 0.00808000 | 1.0772 |
| 212. | 40527-42-2 | 1,3-Benzodioxole, 5-(diethoxymethyl)- | Heliotropin Diethyl Acetal | 0.00796000 | 1.0612 |
| 213. | 56973-85-4 | 4-Penten-1-one, 1-(5, 5-dimethyl-1-cyclohexen-1-yl)- | Neobutenone α | 0.00763000 | 1.0172 |
| 214. | 128-51-8 | Bicyclo[3.1.1]hept-2-ene-2-ethanol, 6,6-dimethyl-, 2-acetate | Nopyl Acetate | 0.00751000 | 1.0013 |
| 215. | 103-36-6 | 2-Propenoic acid, 3-phenyl-, ethyl ester | Ethyl Cinnamate | 0.00729000 | 0.97192 |
| 216. | 5182-36-5 | 1,3-Dioxane, 2,4,6-trimethyl-4-phenyl- | Floropal^{®} | 0.00709000 | 0.94526 |
| 217. | 42604-12-6 | Cyclododecane, (methoxymethoxy)- | Boisambrene | 0.00686000 | 0.91459 |
| 218. | 33885-52-8 | Bicyclo[3.1.1]hept-2-ene-2-propanal, α,α,6,6-tetramethyl- | Pinyl Iso Butyrate Alpha | 0.00685000 | 0.91326 |
| 219. | 92015-65-1 | 2(3*H*)-Benzofuranone, hexahydro-3,6-dimethyl- | Natactone | 0.00680000 | 0.90659 |
| 220. | 63767-86-2 | Cyclohexanemethano l, α-methyl-4-(1-methylethyl)- | Mugetanol | 0.00678000 | 0.90393 |
| 221. | 3288-99-1 | Benzeneacetonitrile, 4-(1,1-dimethylethyl)- | Marenil CI | 0.00665000 | 0.88659 |
| 222. | 35044-68-9 | 2-Buten-1-one, 1-(2, 6,6-trimethyl-1-cyclohexen-1-yl)- | beta-Damascone | 0.00655000 | 0.87326 |
| 223. | 41724-19-0 | 1,4-Methanonaphthalen-6(2*H*)-one, octahydro-7-methyl- | Plicatone | 0.00652000 | 0.86926 |
| 224. | 75147-23-8 | Bicyclo[3.2.1]octan-8-one, 1,5-dimethyl-, oxime | Buccoxime^{®} | 0.00647000 | 0.86260 |
| 225. | 25634-93-9 | 2-Methyl-5-phenylpentan-1-ol | Rosaphen^{®} 600064 | 0.00637000 | 0.84926 |
| 226. | 55066-48-3 | 3-Methyl-5-phenylpentanol | Phenyl Hexanol | 0.00637000 | 0.84926 |
| 227. | 495-62-5 | Cyclohexene, 4-(1,5-dimethyl-4-hexen-1-ylidene)-1-methyl- | Bisabolene | 0.00630000 | 0.83993 |
| 228. | 2785-87-7 | Phenol, 2-methoxy-4-propyl- | Dihydro Eugenol | 0.00624000 | 0.83193 |
| 229. | 87-19-4 | Benzoic acid, 2-hydroxy-, 2-methylpropyl ester | Iso Butyl Salicylate | 0.00613000 | 0.81727 |
| 230. | 4430-31-3 | 2*H*-1-Benzopyran-2-one, octahydro- | Octahydro Coumarin | 0.00586000 | 0.78127 |
| 231. | 38462-22-5 | Cyclohexanone, 2-(1-mercapto-1-methylethyl)-5-methyl- | Ringonol 50 TEC | 0.00585000 | 0.77994 |
| 232. | 77-83-8 | 2-Oxiranecarboxylic acid, 3-methyl-3-phenyl-, ethyl ester | Ethyl Methyl Phenyl Glycidate | 0.00571000 | 0.76127 |
| 233. | 37677-14-8 | 3-Cyclohexene-1-carboxaldehyde, 4-(4-methyl-3-penten-1-yl)- | Iso Hexenyl Cyclohexenyl Carboxaldehyde | 0.00565000 | 0.75327 |
| 234. | 103-60-6 | Propanoic acid, 2-methyl-, 2-phenoxyethyl ester | Phenoxy Ethyl iso-Butyrate | 0.00562000 | 0.74927 |
| 235. | 18096-62-3 | Indeno[1,2-d]-1,3-dioxin, 4,4a,5,9b-tetrahydro- | Indoflor^{®} | 0.00557000 | 0.74261 |
| 236. | 63500-71-0 | 2*H*-Pyran-4-ol, tetrahydro-4-methyl-2-(2-methylpropyl)- | Florosa Q | 0.00557000 | 0.74261 |
| 237. | 65405-84-7 | Cyclohexanebutanal, α,2,6,6-tetramethyl- | Cetonal^{®} | 0.00533000 | 0.71061 |
| 238. | 171102-41-3 | 4,7-Methano-1*H-*inden-6-ol, 3a,4,5,6,7, 7a-hexahydro-8,8-dimethyl-, 6-acetate | Flor Acetate | 0.00530000 | 0.70661 |
| 239. | 10339-55-6 | 1,6-Nonadien-3-ol, 3, 7-dimethyl- | Ethyl linalool | 0.00520000 | 0.69328 |
| 240. | 23267-57-4 | 3-Buten-2-one, 4-(2, 2,6-trimethyl-7-oxabicyclo[4.1.0] hept-1-yl)- | Ionone Epoxide Beta | 0.00520000 | 0.69328 |
| 241. | 97-54-1 | Phenol, 2-methoxy-4-(1-propen-1-yl)- | Isoeugenol | 0.00519000 | 0.69194 |
| 242. | 67663-01-8 | 2(3*H*)-Furanone, 5-hexyldihydro-4-methyl- | Peacholide | 0.00512000 | 0.68261 |
| 243. | 33885-52-8 | Bicyclo[3.1.1]hept-2-ene-2-propanal, α,α,6, 6-tetramethyl- | Pinyl Iso Butyrate Alpha | 0.00512000 | 0.68261 |
| 244. | 23696-85-7 | 2-Buten-1-one, 1-(2, 6,6-trimethyl-1,3-cyclohexadien-1-yl)- | Damascenone | 0.00503000 | 0.67061 |
| 245. | 80-71-7 | 2-Cyclopenten-1-one, 2-hydroxy-3-methyl- | Maple Lactone | 0.00484000 | 0.64528 |
| 246. | 67662-96-8 | Propanoic acid, 2,2-dimethyl-, 2-phenylethyl ester | Pivarose Q | 0.00484000 | 0.64528 |
| 247. | 2437-25-4 | Dodecanenitrile | Clonal | 0.00480000 | 0.63995 |
| 248. | 141-14-0 | 6-Octen-1-ol, 3,7-dimethyl-, 1-propanoate | Citronellyl Propionate | 0.00469000 | 0.62528 |
| 249. | 54992-90-4 | 3-Buten-2-one, 4-(2,2,3,6-tetramethylcyclohexy 1)- | Myrrhone | 0.00460000 | 0.61328 |
| 250. | 55066-49-4 | Benzenepentanal, β-methyl- | Mefranal | 0.00455000 | 0.60662 |
| 251. | 7493-74-5 | Acetic acid, 2-phenoxy-, 2-propen-1-yl ester | Allyl Phenoxy Acetate | 0.00454000 | 0.60528 |
| 252. | 80-54-6 | Benzenepropanal, 4-(1,1-dimethylethyl)-α-methyl- | Lilial^{®} | 0.00444000 | 0.59195 |
| 253. | 86803-90-9 | 4,7-Methano-1*H-*indene-2-carboxaldehyde, octahydro-5-methoxy- | Scentenal^{®} | 0.00439000 | 0.58529 |
| 254. | 68991-97-9 | 2-Naphthalenecarboxal dehyde, 1,2,3,4,5,6,7, 8-octahydro-8,8-dimethyl- | Melafleur | 0.00436000 | 0.58129 |
| 255. | 18871-14-2 | Pentitol, 1,5-anhydro-2,4-dideoxy-2-pentyl-, 3-acetate | Jasmal | 0.00434000 | 0.57862 |
| 256. | 58567-11-6 | Cyclododecane, (ethoxymethoxy)- | Boisambren Forte | 0.00433000 | 0.57729 |
| 257. | 94400-98-3 | Naphth[2,3-b]oxirene, 1a,2,3,4,5,6,7,7a-octahydro-1a,3,3,4,6,6-hexamethyl-, (1a*R*,4*S*,7a*S*)-rel- | Molaxone | 0.00425000 | 0.56662 |
| 258. | 79-69-6 | 3-Buten-2-one, 4-(2, 5,6,6-tetramethyl-2-cyclohexen-1-yl)- | alpha-Irone | 0.00419000 | 0.55862 |
| 259. | 65442-31-1 | Quinoline, 6-(1-methylpropyl)- | Iso Butyl Quinoline | 0.00408000 | 0.54396 |
| 260. | 87731-18-8 | Carbonic acid, 4-cycloocten-1-yl methyl ester | Violiff | 0.00401000 | 0.53462 |
| 261. | 173445-65-3 | 1*H*-Indene-5-propanal, 2,3-dihydro-3,3-dimethyl- | Hivernal (A-isomer) | 0.00392000 | 0.52262 |
| 262. | 23911-56-0 | Ethanone, 1-(3-methyl-2-benzofuranyl)- | Nerolione | 0.00383000 | 0.51062 |
| 263. | 52474-60-9 | 3-Cyclohexene-1-carboxaldehyde, 1-methyl-3-(4-methyl-3-penten-1-yl)- | Precyclemone B | 0.00381000 | 0.50796 |
| 264. | 139539-66-5 | 6-Oxabicyclo[3.2.1] octane, 5-methyl-1-(2,2,3-trimethyl-3-cyclopenten-1-yl)- | Cassifix | 0.00381000 | 0.50796 |
| 265. | 80858-47-5 | Benzene, [2-(cyclohexyloxy) ethyl]- | Phenafleur | 0.00380000 | 0.50663 |
| 266. | 32764-98-0 | 2*H*-Pyran-2-one, tetrahydro-6-(3-penten-1-yl)- | Jasmolactone | 0.00355000 | 0.47329 |
| 267. | 78417-28-4 | 2,4,7-Decatrienoic acid, ethyl ester | Ethyl 2,4,7-decatrienoate | 0.00353000 | 0.47063 |
| 268. | 140-26-1 | Butanoic acid, 3-methyl-, 2-phenylethyl ester | Beta Phenyl Ethyl Isovalerate | 0.00347000 | 0.46263 |
| 269. | 105-90-8 | 2,6-Octadien-1-ol, 3, 7-dimethyl-, 1-propanoate, (2*E*)- | Geranyl Propionate | 0.003360000 | 0.44796 |
| 270. | 41816-03-9 | Spiro[1,4-methanonaphthalene-2(1*H*),2'-oxirane], 3, 4,4a,5,8,8a-hexahydro-3',7-dimethyl- | Rhubofix^{®} | 0.00332000 | 0.44263 |
| 271. | 7070-15-7 | Ethanol, 2-[[(1*R*,2*R*, 4*R*)-1,7,7-trimethylbicyclo[2.2 .1]hept-2-yl]oxy]-, rel- | Arbanol | 0.00326000 | 0.43463 |
| 272. | 93-29-8 | Phenol, 2-methoxy-4-(1-propen-1-yl)-, 1-acetate | Iso Eugenol Acetate | 0.00324000 | 0.43196 |
| 273. | 476332-65-7 | 2*H*-Indeno[4,5-b] furan, decahydro-2,2, 6,6,7,8,8-heptamethyl- | Amber Xtreme Compound 1 | 0.00323000 | 0.43063 |
| 274. | 68901-15-5 | Acetic acid, 2-(cyclohexyloxy)-, 2-propen-1-yl ester | Cyclogalbanate | 0.00323000 | 0.43063 |
| 275. | 107-75-5 | Octanal, 7-hydroxy-3, 7-dimethyl- | Hydroxycitronell al | 0.00318000 | 0.42397 |
| 276. | 68611-23-4 | Naphtho[2,1-b]furan, 9b-ethyldodecahydro-3a,7,7-trimethyl- | Grisalva | 0.00305000 | 0.40663 |
| 277. | 313973-37-4 | 1,6-Heptadien-3-one, 2-cyclohexyl- | Pharaone | 0.00298000 | 0.39730 |
| 278. | 137-00-8 | 5-Thiazoleethanol, 4-methyl- | Sulfurol | 0.00297000 | 0.39597 |
| 279. | 7779-30-8 | 1-Penten-3-one, 1-(2,6,6-trimethyl-2-cyclohexen-1-yl)- | Methyl Ionone | 0.00286000 | 0.38130 |
| 280. | 127-51-5 | 3-Buten-2-one, 3-methyl-4-(2,6,6-trimethyl-2-cyclohexen-1-yl)- | Isoraldeine Pure | 0.00282000 | 0.37597 |
| 281. | 72903-27-6 | 1,4-Cyclohexanedicarbox ylic acid, 1,4-diethyl ester | Fructalate^{™} | 0.00274000 | 0.36530 |
| 282. | 7388-22-9 | 3-Buten-2-one, 4-(2, 2-dimethyl-6-methylenecyclohexyl )-3-methyl- | Ionone Gamma Methyl | 0.00272000 | 0.36264 |
| 283. | 104-67-6 | 2(3*H*)-Furanone, 5-heptyldihydro- | gamma-Undecalactone (racemic) | 0.00271000 | 0.36130 |
| 284. | 1205-17-0 | 1,3-Benzodioxole-5-propanal, α-methyl- | Helional | 0.00270000 | 0.35997 |
| 285. | 33704-61-9 | 4*H*-Inden-4-one, 1,2, 3,5,6,7-hexahydro-1, 1,2,3,3-pentamethyl- | Cashmeran | 0.00269000 | 0.35864 |
| 286. | 36306-87-3 | Cyclohexanone, 4-(1-ethoxyethenyl)-3,3,5, 5-tetramethyl- | Kephalis | 0.00269000 | 0.35864 |
| 287. | 97384-48-0 | Benzenepropanenitril e, α-ethenyl-α-methyl- | Citrowanil^{®} B | 0.00265000 | 0.35330 |
| 288. | 141-13-9 | 9-Undecenal, 2,6,10-trimethyl- | Adoxal | 0.00257000 | 0.34264 |
| 289. | 2110-18-1 | Pyridine, 2-(3-phenylpropyl)- | Corps Racine VS | 0.00257000 | 0.34264 |
| 290. | 27606-09-3 | Indeno[1,2-d]-1,3-dioxin, 4,4a,5,9b-tetrahydro-2,4-dimethyl- | Magnolan | 0.00251000 | 0.33464 |
| 291. | 67634-20-2 | Propanoic acid, 2-methyl-, 3a,4,5,6,7, 7a-hexahydro-4,7-methano-1*H*-inden-5-yl ester | Cyclabute | 0.00244000 | 0.32531 |
| 292. | 65405-72-3 | 1-Naphthalenol, 1,2, 3,4,4a,7,8,8a-octahydro-2,4a,5,8a-tetramethyl-, 1-formate | Oxyoctaline Formate | 0.00236000 | 0.31464 |
| 293. | 122-40-7 | Heptanal, 2-(phenylmethylene)- | Amyl Cinnamic Aldehyde | 0.00233000 | 0.31064 |
| 294. | 103694-68-4 | Benzenepropanol, β, β,3-trimethyl- | Majantol^{®} | 0.00224000 | 0.29864 |
| 295. | 13215-88-8 | 2-Cyclohexen-1-one, 4-(2-buten-1-ylidene) -3,5,5-trimethyl- | Tabanone Coeur | 0.00223000 | 0.29731 |
| 296. | 25152-85-6 | 3-Hexen-1-ol, 1-benzoate, (3Z)- | Cis-3-Hexenyl Benzoate | 0.00203000 | 0.27064 |
| 297. | 406488-30-0 | 2-Ethyl-*N*-methyl-*N-*(m-tolyl)butanamide | Paradisamide | 0.00200000 | 0.26664 |
| 298. | 121-33-5 | Benzaldehyde, 4-hydroxy-3-methoxy- | Vanillin | 0.00194000 | 0.25865 |
| 299. | 77-54-3 | 1*H*-3a,7-Methanoazulen-6-ol, octahydro-3,6,8,8-tetramethyl-, 6-acetate, (3*R*,3a*S*,6*R*, 7*R*,8a*S*)- | Cedac | 0.00192000 | 0.25598 |
| 300. | 76842-49-4 | 4,7-Methano-1*H-*inden-6-ol, 3a,4,5,6,7, 7a-hexahydro-8,8-dimethyl-, 6-propanoate | Frutene | 0.00184000 | 0.24531 |
| 301. | 121-39-1 | 2-Oxiranecarboxylic acid, 3-phenyl-, ethyl ester | Ethyl Phenyl Glycidate | 0.00184000 | 0.24531 |
| 302. | 211299-54-6 | 4*H*-4a,9-Methanoazuleno[5,6-d]-1,3-dioxole, octahydro-2,2,5,8,8, 9a-hexamethyl-, (4a*R*,5*R*,7a*S*,9*R*)- | Ambrocenide^{®} | 0.00182000 | 0.24265 |
| 303. | 285977-85-7 | (2,5-Dimethyl-1,3-dihydroinden-2-yl)methanol | Lilyflore | 0.00180000 | 0.23998 |
| 304. | 10094-34-5 | Butanoic acid, 1,1-dimethyl-2-phenylethyl ester | Dimethyl Benzyl Carbinyl Butyrate | 0.00168000 | 0.22398 |
| 305. | 40785-62-4 | Cyclododeca[c]furan, 1,3,3a,4,5,6,7,8,9,10, 11,13a-dodecahydro- | Muscogene | 0.00163000 | 0.21732 |
| 306. | 75490-39-0 | Benzenebutanenitrile, α,α,γ-trimethyl- | Khusinil | 0.00162000 | 0.21598 |
| 307. | 55418-52-5 | 2-Butanone, 4-(1,3-benzodioxol-5-yl)- | Dulcinyl | 0.00161000 | 0.21465 |
| 308. | 3943-74-6 | Benzoic acid, 4-hydroxy-3-methoxy-, methyl ester | Carnaline | 0.00157000 | 0.20932 |
| 309. | 72089-08-8 | 3-Cyclopentene-1-butanol, β,2,2,3-tetramethyl-2-Methyl-4-(2,2,3-trimethyl-3-cyclopenten-1-yl) butanol | Brahmanol^{®} | 0.00154000 | 0.20532 |
| 310. | 3155-71-3 | 2-Butenal, 2-methyl-4-(2,6,6-trimethyl-1-cyclohexen-1-yl)- | Boronal | 0.00147000 | 0.19598 |
| 311. | 2050-08-0 | Benzoic acid, 2-hydroxy-, pentyl ester | Amyl Salicylate | 0.00144000 | 0.19198 |
| 312. | 41199-20-6 | 2-Naphthalenol, decahydro-2,5,5-trimethyl- | Ambrinol | 0.00140000 | 0.18665 |
| 313. | 12262-03-2 | ndecanoic acid, 3-methylbutyl ester | Iso Amyl Undecylenate | 0.00140000 | 0.18665 |
| 314. | 107-74-4 | 1,7-Octanediol, 3,7-dimethyl- | Hydroxyol | 0.00139000 | 0.18532 |
| 315. | 91-64-5 | 2*H*-1-Benzopyran-2-one | Coumarin | 0.00130000 | 0.17332 |
| 316. | 68901-32-6 | 1,3-Dioxolane, 2-[6-methyl-8-(1-methylethyl) bicyclo[2.2.2]oct-5-en-2-yl]- | Glycolierral | 0.00121000 | 0.16132 |
| 317. | 68039-44-1 | Propanoic acid, 2,2-dimethyl-, 3a,4,5,6,7, 7a-hexahydro-4,7-methano-1*H*-inden-6-yl ester | Pivacyclene | 0.00119000 | 0.15865 |
| 318. | 106-29-6 | Butanoic acid, (2E)-3,7-dimethyl-2,6-octadien-1-yl ester | Geranyl Butyrate | 0.00116000 | 0.15465 |
| 319. | 5471-51-2 | 2-Butanone, 4-(4-hydroxyphenyl)- | Raspberry ketone | 0.00106000 | 0.14132 |
| 320. | 109-42-2 | 10-Undecenoic acid, butyl ester | Butyl Undecylenate | 0.00104000 | 0.13866 |

| | | | | | |
|---|---|---|---|---|---|
| * Vapor Pressures are acquired as described in the Test Methods Section. ** Origin: Same as for Table 1 hereinabove. | | | | | |

Exemplary moderate volatile fragrance materials selected from the group of Table 2 Moderate Volatile Fragrance Materials are preferred. However, it is understood by one skilled in the art that other moderate volatile fragrance materials, not recited in Table 2, would also fall within the scope of the present invention, so long as they have a vapor pressure of 13.33 to 0.133 Pa (0.1 to 0.001 Torr) at 25 °C.

Preferably, the moderate volatile fragrance material is selected from the group of Table 2 Moderate Volatile Fragrance Materials 1-9, 11-12, 14-15, 17-18, 20-25, 27-35, 37-38, 40-44, 46-47, 49-54, 56-62, 64, 66, 68-72, 74-78, 80, 82-85, 87-92, 94-123, 125-126, 131-132, 134-136, 138, 140-146, 148-150, 152, 154-156, 158, 162-163, 165-170, 172-192, 194, 196-199, 201-204, 206-216, 219-220,
222, 224-242, 244, 246-251, 253-256, 258-263, 265-266, 268-269, 272, 274-277, 280-301, 303-305, 307, 309-311, 313-320, and mixtures thereof, and (ii) the substantially non-odorous fragrance modulator is selected from the group of Table 4 Substantially Non-Odorous Fragrance Modulators 1-4, and mixtures thereof.

Preferably, the moderate volatile fragrance material is selected from the group consisting of Table 2 Moderate Volatile Fragrance Materials 1, 3, 4, 6, 7, 9, 11-12, 14, 15, 17-18, 20-25, 30-31, 34-35, 37-38, 42-43, 46-47, 50, 52-54, 56, 58-60, 66-71, 74, 76-78, 80-81, 83, 87-90, 92-95, 99, 102-108, 112-114, 116-123, 125-126, 131-134, 136, 138, 140-144, 146, 148-150, 152, 154-156, 158-160, 162-163, 165-169, 172-181, 183-184, 186-192, 194, 196-199, 201-204, 206-214, 219-220, 222-223, 225-230, 232-242, 244, 246-251, 253, 254, 255-256, 258-263, 265-266, 268-270, 272, 274-277, 280-301, 303-305, 307, 309-311, 313, 315-320, and mixtures thereof; and (ii) the substantially non-odorous fragrance modulator is selected from the group of Table 4 Substantially Non-Odorous Fragrance Modulators 5-7, and mixtures thereof.

Preferably, the low volatile fragrance material is selected from the group (as described herein above), and wherein this group of low volatile fragrance material has at least about 20 wt%, at least about 30 wt%, at least about 40 wt%, at least about 50 wt%, at least about 60 wt%, or at least about 70 wt%, relative to the total weight of the low volatile fragrance material.

### (iii)High Volatile Fragrance Materials

Preferable examples of high volatile fragrance materials having a vapor pressure > 13.33 Pa (0.1 Torr) at 25 °C are provided in Table 3 High Volatile Fragrance Materials. Preferably, the high volatile fragrance material is selected from at least 1 material, or at least 2 materials, or at least 3 materials, or at least 5 materials, or at least 7 high volatile fragrance materials as disclosed in Table 3.

**Table 3-High Volatile Fragrance Materials**

| **No.** | **CAS Number** | **IUPAC Name** | **Common Name**** | **Vapor Pressure (Torr at 25 °C)*** | **Vapor Pressure (Pa at 25 °C)** |
|---|---|---|---|---|---|
| 1. | 107-31-3 | Formic acid, methyl ester | Methyl Formate | 732.00000000 | 97592 |
| 2. | 75-18-3 | Methane, 1,1'-thiobis- | Dimethyl Sulfide 1.0% In DEP | 647.00000000 | 86260 |
| 3. | 141-78-6 | Acetic acid ethyl ester | Ethyl Acetate | 112.00000000 | 14932 |
| 4. | 105-37-3 | Propanoic acid, ethyl ester | Ethyl Propionate | 44.50000000 | 5932.8 |
| 5. | 110-19-0 | Acetic acid, 2-methylpropyl ester | Isobutyl Acetate | 18.00000000 | 2399.8 |
| 6. | 105-54-4 | Butanoic acid, ethyl ester | Ethyl Butyrate | 13.90000000 | 1853.2 |
| 7. | 14765-30-1 | 1-Butanol | Butyl Alcohol | 8.52000000 | 1135.9 |
| 8. | 7452-79-1 | Butanoic acid, 2-methyl-, ethyl ester | Ethyl-2-Methyl Butyrate | 7.85000000 | 1046.6 |
| 9. | 123-92-2 | 1-Butanol, 3-methyl-, 1-acetate | Iso Amyl Acetate | 5.68000000 | 757.27 |
| 10. | 66576-71-4 | Butanoic acid, 2-methyl-, 1-methylethyl ester | Iso Propyl 2-Methylbutyrate | 5.10000000 | 679.94 |
| 11. | 110-43-0 | 2-Heptanone | Methyl Amyl Ketone | 4.73000000 | 630.61 |
| 12. | 6728-26-3 | 2-Hexenal, (2*E*)- | Trans-2 Hexenal | 4.62000000 | 615.95 |
| 13. | 123-51-3 | 1-Butanol, 3-methyl- | Isoamyl Alcohol | 4.16000000 | 554.62 |
| 14. | 1191-16-8 | 2-Buten-1-ol, 3-methyl-, 1-acetate | Prenyl acetate | 3.99000000 | 531.96 |
| 15. | 57366-77-5 | 1,3-Dioxolane-2-methanamine, N-methyl- | Methyl Dioxolan | 3.88000000 | 517.29 |
| 16. | 7785-70-8 | Bicyclo[3.1.1]hept-2-ene, 2,6,6-trimethyl-, (1*R*,5*R*)- | Alpha Pinene | 3.49000000 | 465.30 |
| 17. | 79-92-5 | Bicyclo[2.2.1] heptane, 2,2-dimethyl-3-methylene- | Camphene | 3.38000000 | 450.63 |
| 18. | 94087-83-9 | 2-Butanethiol, 4-methoxy-2-methyl- | 4-Methoxy-2-Methyl-2-Butanenthiol | 3.31000000 | 441.30 |
| 19. | 39255-32-8 | Pentanoic acid, 2-methyl-, ethyl ester | Manzanate | 2.91000000 | 387.97 |
| 20. | 3387-41-5 | Bicyclo[3.1.0]hexane, 4-methylene-1-(1-methylethyl)- | Sabinene | 2.63000000 | 350.64 |
| 21. | 127-91-3 | Bicyclo[3.1.1] heptane, 6,6-dimethyl-2-methylene- | Beta Pinene | 2.40000000 | 319.97 |
| 22. | 105-68-0 | 1-Butanol, 3-methyl-, 1-propanoate | Amyl Propionate | 2.36000000 | 314.64 |
| 23. | 123-35-3 | 1,6-Octadiene, 7-methyl-3-methylene- | Myrcene | 2.29000000 | 305.31 |
| 24. | 124-13-0 | Octanal | Octyl Aldehyde | 2.07000000 | 275.98 |
| 25. | 7392-19-0 | 2*H*-Pyran, 2-ethenyltetrahydro-2, 6,6-trimethyl- | Limetol | 1.90000000 | 253.31 |
| 26. | 111-13-7 | 2-Octanone | Methyl Hexyl Ketone | 1.72000000 | 229.31 |
| 27. | 123-66-0 | Hexanoic acid, ethyl ester | Ethyl Caproate | 1.66000000 | 221.32 |
| 28. | 470-82-6 | 2-Oxabicyclo[2.2.2] octane, 1,3,3-trimethyl- | Eucalyptol | 1.65000000 | 219.98 |
| 29. | 99-87-6 | Benzene, 1-methyl-4-(1-methylethyl)- | Para Cymene | 1.65000000 | 219.98 |
| 30. | 104-93-8 | Benzene, 1-methoxy-4-methyl- | Para Cresyl Methyl Ether | 1.65000000 | 219.98 |
| 31. | 13877-91-3 | 1,3,6-Octatriene, 3,7-dimethyl- | Ocimene | 1.56000000 | 207.98 |
| 32. | 138-86-3 | Cyclohexene, 1-methyl-4-(1-methylethenyl)- | dl-Limonene | 1.54000000 | 205.32 |
| 33. | 5989-27-5 | Cyclohexene, 1-methyl-4-(1-methylethenyl)-, (4*R*) | d-limonene | 1.54000000 | 205.32 |
| 34. | 106-68-3 | 3-Octanone | Ethyl Amyl Ketone | 1.50000000 | 199.98 |
| 35. | 110-41-8 | Undecanal, 2-methyl- | Methyl Nonyl Acetaldehyde | 1.43000000 | 190.65 |
| 36. | 142-92-7 | Acetic acid, hexyl ester | Hexyl acetate | 1.39000000 | 185.32 |
| 37. | 110-93-0 | 5-Hepten-2-one, 6-methyl- | Methyl Heptenone | 1.28000000 | 170.65 |
| 38. | 81925-81-7 | 2-Hepten-4-one, 5-methyl- | Filbertone 1% in TEC | 1.25000000 | 166.65 |
| 39. | 3681-71-8 | 3-Hexen-1-ol, 1-acetate, (3Z)- | cis-3-Hexenyl acetate | 1.22000000 | 162.65 |
| 40. | 97-64-3 | Propanoic acid, 2-hydroxy-, ethyl ester | Ethyl Lactate | 1.16000000 | 154.65 |
| 41. | 586-62-9 | Cyclohexene, 1-methyl-4-(1-methylethylidene)- | Terpineolene | 1.13000000 | 150.65 |
| 42. | 51115-64-1 | Butanoic acid, 2-methylbutyl ester | Amyl butyrate | 1.09000000 | 145.32 |
| 43. | 106-27-4 | Butanoic acid, 3-methylbutyl ester | Amyl Butyrate | 1.09000000 | 145.32 |
| 44. | 99-85-4 | 1,4-Cyclohexadiene, 1-methyl-4-(1-methylethyl)- | Gamma Terpinene | 1.08000000 | 143.99 |
| 45. | 18640-74-9 | Thiazole, 2-(2-methylpropyl)- | 2-Isobutylthiazole | 1.07000000 | 142.65 |
| 46. | 928-96-1 | 3-Hexen-1-ol, (3Z)- | cis-3-Hexenol | 1.04000000 | 138.66 |
| 47. | 100-52-7 | Benzaldehyde | Benzaldehyde | 0.97400000 | 129.86 |
| 48. | 141-97-9 | Butanoic acid, 3-oxo-, ethyl ester | Ethyl Acetoacetate | 0.89000000 | 118.66 |
| 49. | 928-95-0 | 2-Hexen-1-ol, (2*E*)- | Trans-2-Hexenol | 0.87300000 | 116.39 |
| 50. | 928-94-9 | 2-Hexen-1-ol, (2Z)- | Beta Gamma Hexenol | 0.87300000 | 116.39 |
| 51. | 24691-15-4 | Cyclohexane, 3-ethoxy-1,1,5-trimethyl-, cis- (9CI) | Herbavert | 0.85200000 | 113.59 |
| 52. | 19872-52-7 | 2-Pentanone, 4-mercapto-4-methyl- | 4-Methyl-4-Mercaptopentan -2-one 1ppm TEC | 0.84300000 | 112.39 |
| 53. | 3016-19-1 | 2,4,6-Octatriene, 2,6-dimethyl-, (4*E,*6*E*)- | Allo-Ocimene | 0.81600000 | 108.79 |
| 54. | 69103-20-4 | Oxirane, 2,2-dimethyl-3-(3-methyl-2,4-pentadien-1-yl)- | Myroxide | 0.80600000 | 107.46 |
| 55. | 189440-77-5 | 4,7-Octadienoic acid, methyl ester, (4*E*)- | Anapear | 0.77700000 | 103.59 |
| 56. | 67633-96-9 | Carbonic acid, (3Z)-3-hexen-1-yl methyl ester | Liffarome^{™} | 0.72100000 | 96.125 |
| 57. | 123-68-2 | Hexanoic acid, 2-propen-1-yl ester | Allyl Caproate | 0.67800000 | 90.393 |
| 58. | 106-72-9 | 5-Heptenal, 2,6-dimethyl- | Melonal | 0.62200000 | 82.927 |
| 59. | 106-30-9 | Heptanoic acid, ethyl ester | Ethyl Oenanthate | 0.60200000 | 80.260 |
| 60. | 68039-49-6 | 3-Cyclohexene-1-carboxaldehyde, 2,4-dimethyl- | Ligustral or Triplal | 0.57800000 | 77.060 |
| 61. | 101-48-4 | Benzene, (2,2-dimethoxyethyl)- | Phenyl Acetaldehyde Dimethyl Acetal | 0.55600000 | 74.127 |
| 62. | 16409-43-1 | 2*H*-Pyran, tetrahydro-4-methyl-2-(2-methyl-1-propen-1-yl)- | Rose Oxide | 0.55100000 | 73.461 |
| 63. | 925-78-0 | 3-Nonanone | Ethyl Hexyl Ketone | 0.55100000 | 73.461 |
| 64. | 100-47-0 | Benzonitrile | Benzyl Nitrile | 0.52400000 | 69.861 |
| 65. | 589-98-0 | 3-Octanol | Octanol-3 | 0.51200000 | 68.261 |
| 66. | 58430-94-7 | 1-Hexanol, 3,5,5-trimethyl-, 1-acetate | Iso Nonyl Acetate | 0.47000000 | 62.662 |
| 67. | 10250-45-0 | 4-Heptanol, 2,6-dimethyl-, 4-acetate | Alicate | 0.45400000 | 60.528 |
| 68. | 105-79-3 | Hexanoic acid, 2-methylpropyl ester | Iso Butyl Caproate | 0.41300000 | 55.062 |
| 69. | 2349-07-7 | Propanoic acid, 2-methyl-, hexyl ester | Hexyl isobutyrate | 0.41300000 | 55.062 |
| 70. | 23250-42-2 | Cyclohexanecarboxyl ic acid, 1,4-dimethyl-, methyl ester, trans- | Cyprissate | 0.40500000 | 53.996 |
| 71. | 122-78-1 | Benzeneacetaldehyde | Phenyl acetaldehyde | 0.36800000 | 49.063 |
| 72. | 5405-41-4 | Butanoic acid, 3-hydroxy-, ethyl ester | Ethyl-3-Hydroxy Butyrate | 0.36200000 | 48.263 |
| 73. | 105-53-3 | Propanedioic acid, 1, 3-diethyl ester | Diethyl Malonate | 0.34400000 | 45.863 |
| 74. | 93-58-3 | Benzoic acid, methyl ester | Methyl Benzoate | 0.34000000 | 45.330 |
| 75. | 16356-11-9 | 1,3,5-Undecatriene | Undecatriene | 0.33600000 | 44.796 |
| 76. | 65405-70-1 | 4-Decenal, (4*E*)- | Decenal (Trans-4) | 0.33100000 | 44.130 |
| 77. | 54546-26-8 | 1,3-Dioxane, 2-butyl-4,4,6-trimethyl- | Herboxane | 0.33000000 | 43.996 |
| 78. | 13254-34-7 | 2-Heptanol, 2,6-dimethyl- | Dimethyl-2 6-Heptan-2-ol | 0.33000000 | 43.996 |
| 79. | 98-86-2 | Ethanone, 1-phenyl- | Acetophenone | 0.29900000 | 39.863 |
| 80. | 93-53-8 | Benzeneacetaldehyde , α-methyl- | Hydratropic aldehyde | 0.29400000 | 39.197 |
| 81. | 80118-06-5 | Propanoic acid, 2-methyl-, 1,3-dimethyl-3-buten-1-yl ester | Iso Pentyrate | 0.28500000 | 37.997 |
| 82. | 557-48-2 | 2,6-Nonadienal, (2*E*, 6*Z*)- | E Z-2,6-Nonadien-1-al | 0.28000000 | 37.330 |
| 83. | 24683-00-9 | Pyrazine, 2-methoxy-3-(2-methylpropyl)- | 2-Methoxy-3-Isobutyl Pyrazine | 0.27300000 | 36.397 |
| 84. | 104-57-4 | Formic acid, phenylmethyl ester | Benzyl Formate | 0.27300000 | 36.397 |
| 85. | 104-45-0 | Benzene, 1-methoxy-4-propyl- | Dihydroanethole | 0.26600000 | 35.464 |
| 86. | 491-07-6 | Cyclohexanone, 5-methyl-2-(1-methylethyl)-, (2*R*, 5*R*)-rel- | Iso Menthone | 0.25600000 | 34.131 |
| 87. | 89-80-5 | Cyclohexanone, 5-methyl-2-(1-methylethyl)-, (2*R*, 5*S*)-rel- | Menthone Racemic | 0.25600000 | 34.131 |
| 88. | 2463-53-8 | 2-Nonenal | 2 Nonen-1-al | 0.25600000 | 34.131 |
| 89. | 55739-89-4 | Cyclohexanone, 2-ethyl-4,4-dimethyl- | Thuyacetone | 0.25000000 | 33.331 |
| 90. | 150-78-7 | Benzene, 1,4-dimethoxy- | Hydroquinone Dimethyl Ether | 0.25000000 | 33.331 |
| 91. | 64988-06-3 | Benzene, 1-(ethoxymethyl)-2-methoxy- | Rosacene | 0.24600000 | 32.797 |
| 92. | 76-22-2 | Bicyclo[2.2.1]heptan-2-one, 1,7,7-trimethyl- | Camphor gum | 0.22500000 | 29.998 |
| 93. | 67674-46-8 | 2-Hexene, 6,6-dimethoxy-2,5,5-trimethyl- | Methyl Pamplemousse | 0.21400000 | 28.531 |
| 94. | 112-31-2 | Decanal | Decyl Aldehyde | 0.20700000 | 27.598 |
| 95. | 16251-77-7 | Benzenepropanal, β-methyl- | Trifernal | 0.20600000 | 27.464 |
| 96. | 93-92-5 | Benzenemethanol, α-methyl-, 1-acetate | Methylphenylca rbinol Acetate | 0.20300000 | 27.064 |
| 97. | 143-13-5 | Acetic acid, nonyl ester | Nonyl Acetate | 0.19700000 | 26.265 |
| 98. | 122-00-9 | Ethanone, 1-(4-methylphenyl)- | Para Methyl Acetophenone | 0.18700000 | 24.931 |
| 99. | 24237-00-1 | 2*H*-Pyran, 6-butyl-3, 6-dihydro-2,4-dimethyl- | Gyrane | 0.18600000 | 24.798 |
| 100. | 41519-23-7 | Propanoic acid, 2-methyl-, (3*Z*)-3-hexen-1-yl ester | Hexenyl Isobutyrate | 0.18200000 | 24.265 |
| 101. | 93-89-0 | Benzoic acid, ethyl ester | Ethyl Benzoate | 0.18000000 | 23.998 |
| 102. | 20780-48-7 | 3-Octanol, 3,7-dimethyl-, 3-acetate | Tetrahydro Linalyl Acetate | 0.18000000 | 23.998 |
| 103. | 101-41-7 | Methyl 2-phenylacetate | Methylphenyl acetate | 0.17600000 | 23.465 |
| 104. | 40853-55-2 | 1-Hexanol, 5-methyl-2-(1-methylethyl)-, 1-acetate | Tetrahydro Lavandulyl Acetate | 0.17300000 | 23.065 |
| 105. | 933-48-2 | Cyclohexanol, 3,3,5-trimethyl-, (1*R*,5*R*)-rel- | Trimethylcyclo hexanol | 0.17300000 | 23.065 |
| 106. | 35158-25-9 | 2-Hexenal, 5-methyl-2-(1-methylethyl)- | Lactone of Cis Jasmone | 0.17200000 | 22.931 |
| 107. | 18479-58-8 | 7-Octen-2-ol, 2,6-dimethyl- | Dihydromyrcen ol | 0.16600000 | 22.132 |
| 108. | 140-11-4 | Acetic acid, phenylmethyl ester | Benzyl acetate | 0.16400000 | 21.865 |
| 109. | 14765-30-1 | Cyclohexanone, 2-(1-methylpropyl)- | 2-sec-Butyl Cyclo Hexanone | 0.16300000 | 21.732 |
| 110. | 20125-84-2 | 3-Octen-1-ol, (3Z)- | Octenol | 0.16000000 | 21.332 |
| 111. | 142-19-8 | Heptanoic acid, 2-propen-1-yl ester | Allyl Heptoate | 0.16000000 | 21.332 |
| 112. | 100-51-6 | Benzenemethanol | Benzyl Alcohol | 0.15800000 | 21.065 |
| 113. | 10032-15-2 | Butanoic acid, 2-methyl-, hexyl ester | Hexyl-2-Methyl Butyrate | 0.15800000 | 21.065 |
| 114. | 695-06-7 | 2(3*H*)-Furanone, 5-ethyldihydro- | Gamma Hexalactone | 0.15200000 | 20.265 |
| 115. | 21722-83-8 | Cyclohexaneethanol, 1-acetate | Cyclohexyl Ethyl Acetate | 0.15200000 | 20.265 |
| 116. | 111-79-5 | 2-Nonenoic acid, methyl ester | Methyl-2-Nonenoate | 0.14600000 | 19.465 |
| 117. | 16491-36-4 | Butanoic acid, (3*Z*)-3-hexen-1-yl ester | Cis 3 Hexenyl Butyrate | 0.13500000 | 17.999 |
| 118. | 111-12-6 | 2-Octynoic acid, methyl ester | Methyl Heptine Carbonate | 0.12500000 | 16.665 |
| 119. | 59323-76-1 | 1,3-Oxathiane, 2-methyl-4-propyl-, (2*R*,4*S*)-rel- | Oxane | 0.12300000 | 16.399 |
| 120. | 62439-41-2 | Heptanal, 6-methoxy-2,6-dimethyl- | Methoxy Melonal | 0.11900000 | 15.865 |
| 121. | 13851-11-1 | Bicyclo[2.2.1]heptan-2-ol, 1,3,3-trimethyl-, 2-acetate | Fenchyl Acetate | 0.11700000 | 15.599 |
| 122. | 115-95-7 | 1,6-Octadien-3-ol, 3, 7-dimethyl-, 3-acetate | Linalyl acetate | 0.11600000 | 15.465 |
| 123. | 18479-57-7 | 2-Octanol, 2,6-dimethyl- | Tetra-Hydro Myrcenol | 0.11500000 | 15.332 |
| 124. | 78-69-3 | 3,7-dimethyloctan-3-ol | Tetra-Hydro Linalool | 0.11500000 | 15.332 |
| 125. | 111-87-5 | 1-Octanol | Octyl Alcohol | 0.11400000 | 15.199 |
| 126. | 71159-90-5 | 3-Cyclohexene-1-methanethiol, α,α,4-trimethyl- | Grapefruit mercaptan | 0.10500000 | 13.999 |
| 127. | 80-25-1 | Cyclohexanemethano 1, α,α,4-trimethyl-, 1-acetate | Menthanyl Acetate | 0.10300000 | 13.732 |
| 128. | 88-41-5 | Cyclohexanol, 2-(1,1-dimethylethyl)-, 1-acetate | Verdox^{™} | 0.10300000 | 13.732 |
| 129. | 32210-23-4 | Cyclohexanol, 4-(1,1-dimethylethyl)-, 1-acetate | Vertenex | 0.10300000 | 13.732 |
| 130. | 112-44-7 | Undecanal | n-Undecanal | 0.10200000 | 13.599 |
| 131. | 124-19-6 | Nonanal | Nonanal Aldehyde C-9 | 0.53200000 | 70.928 |
| 132. | 929253-05-4 | 6-methoxy-2,6-dimethyloctanal | 6-methoxy-2,6-dimethyl octanal | 0.04020000 | 5.3596 |
| 133. | 68039-47-4 | 2-propan-2-yloxyethylbenzene | Phenethyl Isopropyl Ether | 0.24900000 | 33.197 |
| 134. | 6413-10-1 | ethyl 2-(2-methyl-1,3-dioxolan-2-yl)acetate | Apple Ketal | 0.21900000 | 29.198 |
| 135. | 106-23-0 | 3,7-dimethyloct-6-enal | citronellal | 0.21500000 | 28.664 |

| | | | | | |
|---|---|---|---|---|---|
| * Vapor Pressures are acquired as described in the Test Methods Section. ** Origin: Same as for Table 1 hereinabove. | | | | | |

Exemplary high volatile fragrance materials selected from the group of Table 3 High Volatile Fragrance Materials are preferred. However, it is understood by one skilled in the art that other high volatile fragrance materials, not recited in Table 3, would also fall within the scope of the present invention, so long as they have a vapor pressure of > 13.33 Pa (0.1 Torr) at 25 °C.

Preferably, the high volatile fragrance material is selected from the group of Table 3 High Volatile Fragrance Materials 1, 2, 6, 8, 9, 12, 14, 19, 36, 39, 46, 47, 56, 57, 58, 60, 62, 74, 78, 93, 94, 96, 100, 106, 111, 117, 119, 120, 128, 129, 131-135 and mixtures thereof; and (ii) the substantially non-odorous fragrance modulator is selected from the group of Table 4 Substantially Non-Odorous Fragrance Modulators 1-4, and mixtures thereof.

Preferably, the high volatile fragrance material is selected from the group consisting of Table 3 high Volatile Fragrance Materials 1, 2, 6, 8, 9, 12, 14, 19, 36, 39, 46, 47, 56, 57, 58, 60, 62, 74, 78, 93, 94, 96, 100, 106, 111, 117, 119, 120, 128, 129, 131-135, and mixtures thereof; and (ii) the substantially non-odorous fragrance modulator is selected from the group of Table 4 Substantially Non-Odorous Fragrance Modulators 5-7, and mixtures thereof.

Preferably, the high volatile fragrance material is selected from the group (as described herein above), and wherein this group of high volatile fragrance material has at least about 20 wt%, at least about 30 wt%, at least about 40 wt%, at least about 50 wt%, at least about 60 wt%, or at least about 70 wt%, relative to the total weight of the high volatile fragrance material.

### Fragrance Modulators

The compositions used in the present invention comprise at least one substantially non-odorous modulator selected from the group consisting of:
(a) Methyl Glucoside Polyol; Ethyl Glucoside Polyol; Propyl Glucoside Polyol; and their mixtures;
(b) Isocetyl Alcohol;
(c) PPG-3 Myristyl Ether; Neopentyl Glycol Diethylhexanoate; and their mixtures;
(d) Sucrose Laurate, Sucrose Myristate, Sucrose Palmitate, Sucrose Stearate and mixtures thereof;
(e) Cycloalkyl Acetal derivatives having the formula (I): 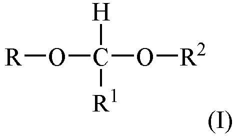 wherein:
   R is C₁-C₈ cycloalkyl;
   R¹ is hydrogen or methyl; and
   R² is methyl, ethyl, propyl, isopropyl, allyl or propargyl;
(f) Trimethylcyclohexane derivatives having the formula (II): 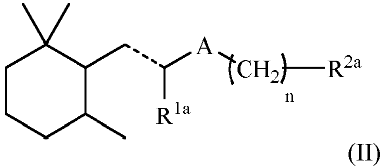 wherein:
   n is 0, 1 or 2;
   A is C=O or CH-OH;
   R^{1a} is hydrogen or methyl;
   R^{2a} is a C₂-C₁₀ hydrocarbon group; and
   ------ is a saturated or unsaturated carbon-carbon bond;
(g) 2-(1-methoxy)ethane-1-ol; 1-(1-menthoxy)propane-2-ol; 3-(1-menthoxy)propane-1-ol; 3-(1-menthoxy)propane-1,2-diol; 2-methyl-3-(1-menthoxy)propane-1,2-diol; 4-(1-menthoxy) butane-1-ol; and their mixtures;
(h) L-menthoxy ether derivatives having the formula (III): 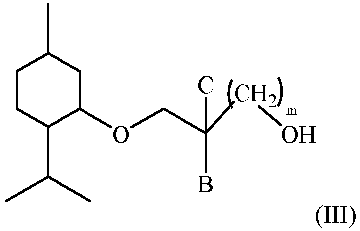 wherein:
   m is 0, 1 or 2;
   B is hydrogen or OH; and
   C is hydrogen or methyl;
(i) Tetra-hydronaphthalene derivatives having the formula (IV): 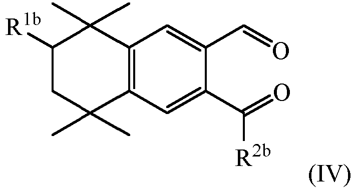 wherein:
   R^{1b} is hydrogen or methyl; and
   R^{2b} is alkyl;
(j) Hyaluronic acid disaccharide sodium salt, sodium hyaluronate and their mixtures;
(k) neopentyl glycol diisononanoate, cetearyl ethylhexanoate and their mixtures;
(l) N-hexadecyl n-nonanoate, N-octadecyl n-nonanoate and their mixtures;
(m)Ether derivatives having the formula (V) or formula (VI):

   C₃H*ₗ*Oₘ-(OR^{1c})ₙ (V)

   wherein:
   C₅H*ₗ*Oₘ is a pentose residue, wherein *l* is an integer from 6 to 9, and m is an integer from 1 to 4;
   n is an integer from 1 to 4; and
   R^{1c} is C₄-C₂₀ hydrocarbon group; and

      C₆HₓO_{y}-(OR^{1d})_{z} (VI)

      wherein:
      C₆HₓO_{y} is a hexose residue, wherein x is an integer from 7 to 11, and y is an integer from 1 to 5;
      z is an integer from 1 to 5; and
      R^{1d} is C₄-C₂₀ hydrocarbon group; and
(n) Diethylene Glycol Ether derivatives having the formula (VII) or formula (VIII):

   C₅H_{c}O_{d}-(OCH₂CH₂-O-CH₂CH₂-O-R^{1e})ₑ (VII)

   wherein:
   C₅H_{c}O_{d} is a pentose residue, wherein c is an integer from 6 to 8, and d is an integer from 1 to 3;
   e is an integer from 2 to 4; and
   R^{1e} is C₁-C₆ alkyl group; and

      C₆H_{f}O_{g}-(OCH₂CH₂-O-CH₂CH₂-O-R^{1f})ₕ (VIII)

      wherein:
      C₆H_{f}O_{g} is a hexose residue, wherein f is an integer from 7 to 10, and g is an integer from 1 to 4;
      h is an integer from 2 to 5; and
      R^{1f} is C₁-C₆ alkyl group;
(o) Hydroquinone Glycoside derivatives having the formula (IX): 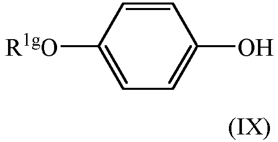 wherein:
   R^{1g} is selected from the group consisting of: (i) pentose residue, hexose residue, aminosaccharide residue, uronic acid residue and their mixtures; (ii) methylated versions of group (i); and (iii) mixtures of groups (i) and (ii); and
(p) Propylene Glycol Propyl Ether; Dicetyl Ether; Polyglycerin-4 Ethers; Isoceteth-5; Isoceteth-7, Isoceteth-10; Isocetteth-12; Isoceteth-15; Isoceteth-20; Isoceteth-25; Isoceteth-30; Disodium Lauroamphodipropionate; Hexaethylene glycol monododecyl ether; and their mixtures;
(q) Neopentyl Glycol Diisononanoate; Cetearyl Ethylhexanoate; and their mixtures;
(r) Glyceryl Ether derivatives having the formula (X): 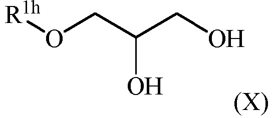 wherein:
   R^{1h} is C₄-C₁₂ aliphatic hydrocarbon group;
(s) Panthenol Ethyl Ether, DL-Panthenol and their mixtures;
(t) Aliphatic Dibasic Acid Diester derivatives having the formula (XI):

   R¹ⁱOCO^{R21}COOR³ⁱ (XI)

   wherein:
   R¹ⁱ is C₄-C₅ alkyl;
   R²ⁱ is C₄ alkylene; and
   R³ⁱ is C₄-C₅ alkyl; and
(u) Tricyclodecane Amide derivatives selected from the group consisting of:
   (i) the compounds of formula (XII): 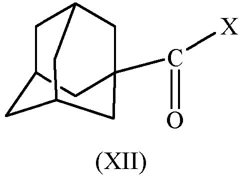 wherein:
      X is selected from: 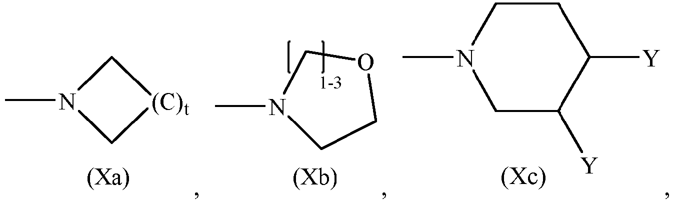 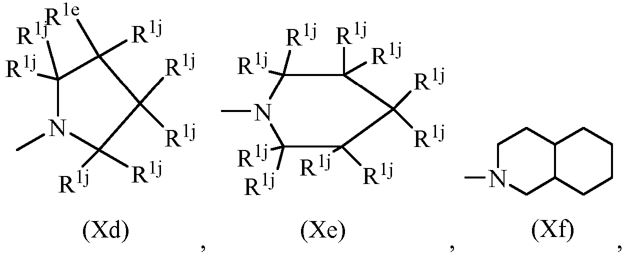 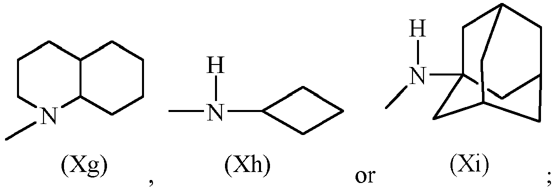
      t is 1 to 8;
      Y is hydrogen, 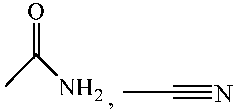 or a halogen; and
      each R^{1j} is independently selected from a hydrogen, or C₁-C₄ alkyl;
   (ii) the compounds of formula (XIII): 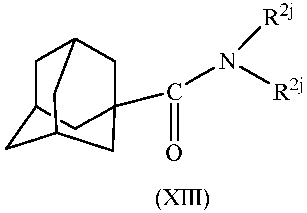 wherein:
      each R^{2j} is independently selected from a hydrogen, methyl, ethyl or
      C₃-C₁₈ alkyl, cycloalkyl or cycloheteroalkyl, with the proviso that both R^{2e} groups are not hydrogen; and
   (iii) mixtures of the compounds of formulae (XII) and (XIII);
(v) Aliphatic ether derivatives having the formula (XIV):

   R^{1k}-O-(CH(CH₃)-CH₂O)ₚ-(CH₂-CH₂O)_{q}-H (XIV)

   wherein:
   p and q are integers such that the sum of p and q is from 1 to 4; and
   R^{1k} is an aliphatic chain comprising from 8 to 18 carbons; and
(w)propyl {4-[2-(diethylamino)-2-oxoethoxy]-3-methoxyphenyl}acetate;
(x) Glycerol exthoxylate derivatives selected from the group consisting of:
   (i) the compounds of formula (XV): 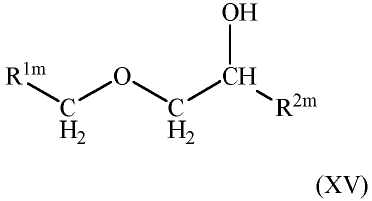 wherein:
      R^{1m} is C₆-C₁₂ alkyl; and
      R^{2m} is amino or hydroxyl group; and
   (ii) the compounds of formula (XVI): 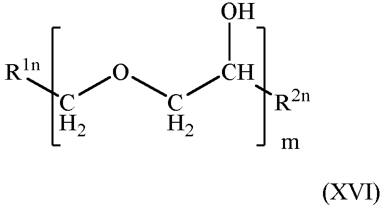 wherein:
      m is 1 or 5;
      R¹ⁿ is C₆-C₁₂ alkyl;
      R²ⁿ is C₁-C₆alkylene-R³ⁿ; and
      R³ⁿ is hydroxyl or amino group;
(y) Bis-methoxy PEG-13 PEG-438/PPG-110 SMDI Copolymer;
(z) mixtures thereof.

Preferably, the substantially non-odorous fragrance modulator is selected from the group of materials disclosed in Table 4.

**Table 4: Substantially Non-Odorous Fragrance Modulators**

| **No.** | **Group** | **Chemical Name** | **CAS Number** | **Supplier** |
|---|---|---|---|---|
| 1. | (a) | PPG-10 Methyl Glucose Ether | 61849-72-7 | Lubrizol |
| 2. | | PPG-20 Methyl Glucose Ether *¹* | 61849-72-7 | |
| 3. | | Ethoxylated Methyl Glucose Ether *²* | 68239-42-9 | |
| 4. | | Caprylyl/Capryl Glucoside *³* | 68515-73-1 | BASF |
| 5. | | Undecyl Glucoside *^{3a}* | -- | SEPPIC (France) |
| 6. | (b) | Isocetyl Alcohol *⁴* | 36653-82-4 | Ashland Speciality Ingredients |
| 7. | (c) | PPG-3 Myristyl Ether *⁵* | -- | Evonik |
| 8. | | Neopentyl Glycol Diethylhexanoate *⁶* | 28510-23-8 | Lubrizol |
| 9. | (d) | Sucrose Laurate | 25339-99-5 | Alfa Chemicals Ltd. (UK) |
| 10. | | Sucrose Myristate | 27216-47-3 | Mitsubishi Chemicals |
| 11. | | Sucrose Palmitate | 26446-38-8 | Alfa Chemicals |
| 12. | | Sucrose Stearate | 25168-73-4 | Ltd. (UK) |
| 13. | (e) | 1-ethoxyethoxy-cyclododecane *⁷* | 389083-83-4 | Firmenich (Switzerland) |
| 14. | (f) | (*E*)-1-(2,2,6-trimethylcyclohexyl)oct-1-en-3-one*⁸* | -- | Takasago (Japan) |
| 15. | (g) | 2-(1-methoxy)ethane-1-ol *⁹* | -- | Takasago (Japan) |
| 16. | | 1-(1-menthoxy)propane-2-ol *⁹* | -- | |
| 17. | | 3-(1-menthoxy)propane-1-ol *⁹* | -- | |
| 18. | | 3-(1-menthoxy)propane-1,2-diol*⁹* | -- | |
| 19. | | 2-methyl-3-(1-menthoxy)propane-1,2-diol⁹ | -- | |
| 20. | | 4-(1-menthoxy) butane-1-ol*⁹* | -- | |
| 21. | (h) | 1,1,4,4-tetramethyl-6-acetyl-7-formyl-1,2,3,4-tetrahydronaphthalene *¹⁰* | -- | Givaudan (Switzerland) |
| 22. | | 1,1,2,4,4-pentamethyl-6-acetyl-7-formyl-1,2,3,4-tetrahydronaphthalene *¹⁰* | -- | |
| 23. | (j) | Hyaluronic acid disaccharide sodium salt *¹¹* | 9004-61-9 | Sigma Aldrich (UK) |
| 24. | | Sodium Hyaluronate *¹¹* | 9067-32-7 | |
| 25. | (m) | Mono-o-(linalyl)-glucopyranose *¹²* | -- | Kanebo (Japan) |
| 26. | | Di-o-(linalyl)-glucopyranose *¹²* | -- | |
| 27. | | Tri-o-(linalyl)-glucopyranose *¹²* | -- | |
| 28. | | Tetra-o-(linalyl)-glucopyranose *¹²* | -- | |
| 29. | | Penta-o-(linalyl)-glucopyranose *¹²* | -- | |
| 30. | | Mono-o-(cis-3-hexenyl)-glactopyranose *¹²* | -- | |
| 31. | | Di-o-(cis-3-hexenyl)-glactopyranose *¹²* | -- | |
| 32. | | Tri-o-(cis-3-hexenyl)-glactopyranose *¹²* | -- | |
| 33. | | Tetra-o-(cis-3-hexenyl)-glactopyranose *¹²* | -- | |
| 34. | | Penta-o-(cis-3-hexenyl)-glactopyranose *¹²* | -- | |
| 35. | (n) | Bis-O-(3,6-dioxadecanyl)-glucopyranose *¹³* | -- | |
| 36. | | Tris-O-(3,6-dioxadecanyl)-glucopyranose *¹³* | -- | |
| 37. | | Tetrakis-O-(3,6-dioxadecanyl)-glucopyranose *¹³* | -- | |
| 38. | | Pentakis-O-(3,6-dioxadecanyl)-glucopyranose*¹³* | -- | |
| 39. | | Bis-O-(3,6-dioxaoctanyl)-galactopyranose *¹³* | -- | |
| 40. | | Tris-O-(3,6-dioxaoctanyl)-galactopyranose *¹³* | -- | |
| 41. | | Tetrakis-O-(3,6-dioxaoctanyl)-galactopyranose*¹³* | -- | |
| 42. | | Pentakis-O-(3,6-dioxaoctanyl)-galactopyranose *¹³* | -- | |
| 43. | | Bis-O-(3,6-dioxaheptanyl)-xylopyranose *¹³* | -- | |
| 44. | | Tris-O-(3,6-dioxaheptanyl)-xylopyranose *¹³* | -- | |
| 45. | | Tetrakis-O-(3,6-dioxaheptanyl)-xylopyranose *¹³* | -- | |
| 46. | | Bis-O-(3,6-dioxadodecanyl)-glucopyranose *¹³* | -- | |
| 47. | | Tris-O-(3,6-dioxadodecanyl)-glucopyranose *¹³* | -- | |
| 48. | | Tetrakis-O-(3,6-dioxadodecanyl)-glucopyranose *¹³* | -- | |
| 49. | | Pentakis-O-(3,6-dioxadodecanyl)-glucopyranose *¹³* | -- | |
| 50. | (o) | Hydroquinone beta-D-glycoside *¹⁴* | 497-76-7 | Shiseido |
| 51. | (p) | Propylene Glycol Propyl Ether | 1569-01-3 | Sigma Aldrich (UK) |
| 52. | | Dicetyl Ether | 4113-12-6 | |
| 53. | | Polyglycerin-4 Ethers | 25618-55-7 | Solvay Chemicals |
| 54. | | Isoceteth-5 | 69364-63-2 | Nihon Emulsion Company Ltd. |
| 55. | | Isoceteth-7 | -- | |
| 56. | | Isoceteth-10 | -- | |
| 57. | | Isoceteth-15 | -- | |
| 58. | | Isoceteth-20 | -- | |
| 59. | | Isoceteth-25 | -- | |
| 60. | | Isoceteth-30 | -- | |
| 61. | | Disodium Lauroamphodipropionate | 68929-04-4 | Rhodia |
| 62. | | Hexaethylene glycol monododecyl ether | 3055-96-7 | Sigma Aldrich (US) |
| 63. | (q) | Neopentyl Glycol Diisononanoate *¹⁵* | 27841-07-2 | Symrise |
| 64. | | Cetearyl Ethylhexnoate *¹⁶* | 90411-68-0 | |
| 65. | (r) | 2-ethylhexyloxypropanediol *¹⁷* | 70455-33-9 | Takasago (JP) |
| 66. | (s) | Panthenol Ethyl Ether *¹⁸* | 667-83-4 | Givaudan |
| 67. | | DL-Panthenol | 16485-10-2 | DSM Nutritional Products, Inc. (USA) |
| 68. | (t) | Diisobutyl Adipate *¹⁹* | 141-04-8 | Shiseido |
| 69. | | Diisoamyl Adipate *¹⁹* | 6624-70-0 | |
| 70. | (u) | methanone, (morphonyl)tricyclo[3.3.1.1^{3,7}]dec-1-yl- *²⁰* | -- | Unilever (UK) |
| 71. | | methanone, (piperidinyl)tricyclo[3.3.1.1^{3,7}]dec-1-yl- *²⁰* | -- | |
| 72. | | methanone, (pyrrolidinyl)tricyclo[3.3.1.1^{3,7}]dec-1-yl *²⁰* | -- | |
| 73. | | methanone, (azetidinyl)tricyclo[3.3.1.1^{3,7} ]dec-1-yl- *²⁰* | -- | |
| 74. | | methanone, (hexahydroazepinyl)tricyclo[3.3.1.1^{3,7}]dec-1-yl*-²⁰* | -- | |
| 75. | | methanone, (4-cyano-piperidinyl)tricyclo[3.3.1.1^{3,7}]dec-1-yl- *²⁰* | -- | |
| 76. | | methanone, (4-amido-piperidinyl)tricyclo[3.3.1.1^{3,7}]dec-1-yl- *²⁰* | -- | |
| 77. | | methanone, (Tricyclo[3.3.1.1^{3,7}]decanyl)-*N* tricyclo[3.3.1.1^{3,7}]dec-1-yl- *²⁰* | -- | |
| 78. | | methanone, (decahydroisoquinolinyl)tricyclo[3.3.1.1^{3,7}]dec-1-yl- *²⁰* | -- | |
| 79. | | methanone, (decahydroisoquinolinyl)tricyclo[3.3.1.1^{3,7}]dec-1-yl- *²⁰* | -- | |
| 80. | | methanone, (decahydroquinolinyl)tricyclo[3.3.1.1^{3,7}]dec-1-yl*-²⁰* | -- | |
| 81. | | methanone, (3,3-dimethyl-1-piperidinyl)tricyclo[3.3.1.1^{3,7}]dec-1-yl- *²⁰* | -- | |
| 82. | | methanone, (2-methyl-1-piperidinyl)tricyclo[3.3.1.1^{3,7}]dec-1-yl- *²⁰* | -- | |
| 83. | | methanone, (4-methyl-1-piperidinyl)tricyclo[3.3.1.1^{3,7}]dec-1-yl- *²⁰* | -- | |
| 84. | | methanone, (3-methyl-1-piperidinyl)tricyclo[3.3.1.1^{3,7}]dec-1-yl- *²⁰* | -- | |
| 85. | | methanone, (3,5-dimethyl-1-piperidinyl)tricyclo[3.3.1.1^{3,7}]dec-1-yl- *²⁰* | -- | |
| 86. | | methanone, (4-methyl-4-ethy-piperidinyl)tricyclo[3.3.1.1^{3,7}]dec-1-yl- *²⁰* | -- | |
| 87. | | methanone, (3,3-diethyl-1-pyrrolidinyl)tricyclo[3.3.1.1^{3,7}]dec-1-yl- *²⁰* | -- | |
| 88. | | methanone, (*N*,*N*-diisopropyl) tricyclo[3.3.1.1^{3,7}]dec-1-yl- *²⁰* | -- | |
| 89. | | methanone, (3,3-dimethylbutylaminyl) tricyclo[3.3.1.1^{3,7}]dec-1-yl- *²⁰* | -- | |
| 90. | | methanone, (2,2-dimethylpropylaminyl) tricyclo[3.3.1.1^{3,7}]dec-1-yl- *²⁰* | -- | |
| 91. | | methanone, (1,1-dimethyl-3,3-dimethylbutylaminyl) tricyclo[3.3.1.1^{3,7}]dec-1-yl- *²⁰* | -- | |
| 92. | | methanone, (1,3-dimethyl-butylaminyl) tricycle[3.3.1.1^{3,7}]dec-1-yl- *²⁰* | -- | |
| 93. | w | propyl {4-[2-(diethylamino)-2-oxoethoxy]-3-methoxyphenyl} acetate *²¹* | 61791-12-6 | Sigma Aldrich (US) |
| 94. | x | 3-((2-ethylhexyl)oxy)propane-1,2-diol | 70445-33-9 | -- |
| 95. | | 3-((2-propylheptyl)oxy)propane-1,2-diol | -- | |
| 96. | | 1-amino-3-((2-ethylhexyl)oxy)propan-2-ol | 99509-00-9 | |
| 97. | y | Bis-methoxy PEG-13 PEG-438/PPG-110 SMDI Copolymer *²²* | 936645-35-1 | PolymerExpert S.A. (Pessac, France) |

| | | | | |
|---|---|---|---|---|
| *¹* available as GLUCAM^{™} P-20. *²* available as Glucam^{™} E-20. *³* available as Plantacare^{®} 810 UP. *^{3a}* available as Simulsol^{®} SL 11W. *⁴* available as CERAPHYL^{®} ICA. *⁵* available as Tegosoft^{®} APM. *⁶* available as Schercemol^{™} NGDO. *⁷* disclosed in U.S. Patent No. 6,737,396B2 (Firmenich), column 1, lines 43-47. *⁸* diclosed as compound 1'i in U.S. Patent No. 6,440,400B1 (Takasago Int. Corp.), col. 5. *⁹* disclosed in U.S. Patent No. 7,538,081B2 (Takasago Int. Corp.), column 7, lines 50-53. *¹⁰* disclosed in U.S. Patent No. 6,147,049 (Givaudan Roure), col. 5, line 24, to col. 6, line 17. *¹¹* disclosed in PCT Publication No. WO85/04803 (Diagnostic), pg. 2, line 1 to pg. 4, line 2. *¹²* disclosed in JP Patent No. 61-083114 (Kanebo). *¹³* disclosed in JP Patent No. 61-063612 (Kanebo). *¹⁴* disclosed in JP Patent No. 62-084010 (Shiseido). *¹⁵* disclosed in U.S. Patent Publication No. 2011/0104089A1 (Symrise), para. [0001]. *¹⁶* available as PCL-Liquid^{®} 100. *¹⁷* disclosed in U.S. Patent No. 7,196,052 (Takasago Int. Corp.), col. 4, lines 34-35. *¹⁸* disclosed in EP Patent Publication No. 616800A2 (Givaudan), pg. 2, lines 12-25. *¹⁹* disclosed in U.S. Patent No. 4,110,626 (Shiseido), column 3, lines 54-56. *²⁰* disclosed as compounds C1-C22 in WO2014/139952 (Unilever). *²¹* available as Kolliphor^{®} EL. ²² available as Expert Gel^{®} EG56. | | | | |

The compounds, as described above in Table 4, act as a substantially non-odorous fragrance modulator of the perceived fidelity and/or longevity of the fragrance profile of the composition of the present invention. For example, the substantially non-odorous fragrance modulators, with a fragrance component having a diamond construction, act to prolong the duration during which the fragrance profile, preferably the aromas attributable from the moderate and high volatile fragrance materials, can be perceived as compared to a control composition in the absence of the modulators or having the classical fragrance pyramid three-tiered structure. As another example, the substantially non-odorous fragrance modulators, with a fragrance component having a diamond construction, can improve the fidelity of the fragrance profile, preferably the aromas attributable from the moderate and high volatile fragrance materials, such that it remains significantly the same from initial impression to the end as compared to a control composition in the absence of the modulators or having the classical fragrance pyramid three-tiered structure. While not wishing to be bound by theory, it is believed that the substantially non-odorous fragrance modulators associate to the fragrance materials and retard evaporation.

### TEST METHODS

### Test Method 1: Determining Vapor Pressure

In order to determine the vapor pressure for the fragrance materials, go to the website *https:*//*scifinder.cas.org*/*scifinder*/*view*/*scifinder*/*scifinderExplore.jsf* and follow these steps to acquire the vapor pressure.
1. Input the CAS registry number for the particular fragrance material.
2. Select the vapor pressure from the search results.
3. Record the vapor pressure (given in Torr at 25 °C).

SciFinder uses Advanced Chemistry Development (ACD/Labs) Software Version 11.02. (^{©} 1994-2013). If the CAS number for the particular fragrance material is unknown or does not exist, you can utilize the ACD/Labs reference program to directly determine the vapor pressure.

### Test Method 2: Olfactory Tests

In order to show the effect of the substantially non-odorous fragrance modulators and fragrance component having a diamond construction on the perception of fragrance profile in a composition of the present invention, test compositions are made, as described in the Example section, and given to panelists to evaluate.

At the testing facility, 50 µL samples of the compositions or the controls are applied to glass slides and placed on a hot plate at 32 °C to represent skin temperature for varying durations. The panelists are asked to evaluate the perceived fragrance profile (intensity and/or character) from each pair of samples, *i.e.,* that of the test composition of the present invention vs. the corresponding control, at time 0 and later time points (1, 2, 3 4 and 6 hours post application) as the fragrance profile evolves. Their assessments are recorded. Panelists are selected from individuals who are either trained to evaluate fragrances according to the scales below or who have experience of fragrance evaluation in the industry.

### (a) Fragrance Intensity:

The panelists are asked to give a score on a scale of 0 to 5 for perceived fragrance intensity according to the odour intensity scale set out in Table 4 herein below.

**Table 4 - Odour Intensity Scale**

| **Score** | **Fragrance Intensity** |
|---|---|
| 0 | None |
| 1 | Very Weak |
| 2 | Weak |
| 3 | Moderate |
| 4 | Strong |
| 5 | Very Strong |

### (b) Fragrance Character:

The panelists are asked to assess the fragrance character in one of 2 ways:
i) a score on a scale of 0 to 3 for the dominance of particular characters that are relevant to that particular fragrance, e.g.: floral, rose, muguet, fruity, apple, berry, citrus, woody, musk just to name a few, according to the odour grading scale set out in Table 5(i) herein below;
ii) a score on a scale of 1 to 5 for changes in the perceived fragrance profile change for the test compositions versus the controls according to the odour grading scale set out in Table 5(ii) herein below.

**Table 5(i) - Character Dominance Odour Grading Scale**

| **Score** | **Fragrance Character Dominance** |
|---|---|
| 0 | Not noticeable |
| 1 | Slight presence of the character |
| 2 | Moderate presence of the character |
| 3 | Dominance of the character |

**Table 5(ii) - Character Difference Odour Grading Scale**

| **Score** | **Fragrance Profile Change** |
|---|---|
| 1 | Frargrance profile is unchanged, *i.e.,* no difference between the sample vs. the control. |
| 2 | Slight fragrance profile change when compared directly with the control. |
| 3 | Moderate fragrance profile but similar character to the control, |
| 4 | Large difference in fragrance profile from the control. |
| 5 | Total difference in the fragrance profile from the control. |

The results of the panelists are averaged and then analysed using Analysis of Variance methods. The model treats the subject as a random effect and looks at the impact of product, time and the interaction between product and time. From the analysis the least square means for the product and time interaction are obtained. These means (as well as their confidence intervals) are then plotted to enable comparisons between products at each time point. It should be noted that the confidence levels plotted are intended as a guide, and not as a statistical comparison, as they do not take into account that multiple testing has been performed. As well as a graphical assessment, statistical comparisons between the two products at each of the time points are performed with a Sidak correction for multiple comparisons. The p-values for the product differences are obtained, with p-values < 0.05 indicating a statistical difference between the two products at 5% significance (or 95% confidence).

### Test Method 3: Analytical Evaporation Tests

The following test is carried out to demonstrate the improved or enhanced longevity of a fragrance profile of a composition of the present invention vs. a control. In particular, the test measures the effect of a substantially non-odorous fragrance modulator on the evaporation rate of one or more fragrance materials formulated in a composition. The evaporation response of the fragrance materials to the modulator, as a function of time, is measured through the use of gas chromatography ("GC").
1. A test composition comprising the substantially non-odorous fragrance modulator with either: (i) a fragrance material, or (ii) a blend of fragrance materials is made. The inventive compositions also contain high purity ethanol, such as Hayman 100%EP/BP grade, and deionised water. Samples tests compositions are provided in Tables 18 and 19. All of the ingredients are admixed until evenly distributed in the test compositions.
2. A control composition, without the substantially non-odorous fragrance modulator or with normal or high levels of the low volatile fragrance materials, is made in a similar manner to Step 1. Sample control compositions are provided in Tables 18 and 19.
3. An internal standard is needed to correct for variations of the amount of composition dispensed in the evaporation test as well as loss during the GC analysis. The internal standard has a vapor pressure of less than 0.133 Pa (0.001 Torr) at 25 °C and is soluble in the composition and fragrance material. Suitable non-limiting examples of internal standard include: isopropyl myristate or triethyl citrate. The internal standard and fragrance material are admixed until evenly distributed at a level of 90 to 95 parts by weight of fragrance material and the required amount of internal standard to reach 100 parts. This mixture is then use to prepare the sample compositions in Step 1 and 2. Alternatively, the internal standard and test or control composition are admixed until evenly distributed at a level of 99 to 99.75 parts by weight of composition and the required amount of internal standard to reach 100 parts. This resultant solution is used in subsequent steps.
4. A hotplate is set to a temperature of 32 °C. An aluminum container, such as TA Instruments T-Zero^{™} pan or lids, or a Cole Parmer Micro aluminum weighing dish, with dimensions 12 mm (L) x 4 mm (W) x 2.7 mm (H), is placed on the hotplate. 20 µL of the test or control composition is introduced in the aluminum container using a micropipette. Alternatively, the aluminum container may be filled with the test or control composition to its full capacity. The time at which this takes place is determined to be time zero (*i.e.,* T=0). Multiple aluminum containers are prepared and left at the set temperature for pre-determined periods of time, such as for example 20 mins, 1 hr, 2 hrs, 3 hrs, 4 hrs, 5 hrs, 6 hrs, 8 hrs and up to 12 hrs.
5. The aluminum container is removed from the hotplate at the end of the pre-determined time period and transferred by being inserted into a 4 mL glass vial already containing at least 2 mL of highly volatile solvent, such as high purity ethanol or hexane.
6. The glass vial is mixed using a Heidolph multi REAX shaker for 5 to 10 mins to extract the fragrance materials into the solvent phase. 1.5mL of the resultant solution is transferred to a 2 mL GC vial.
7. The GC vial is analysed on an Agilent GC system 6890B equipped with an autosampler, or equivalent. A GC column such as a DB-5 or Rxi-5 SilMS model, with a length of 30 m, an inner diameter of 0.25 mm and a film thickness of 1 µm is used. The GC parameters are set to the values indicated as follows:

**Table 5(iii) - GC Parameters**

| | |
|---|---|
| Injector temperature: | 270 °C |
| Initial gas velocity: | 25 to 40 cm/sec (for Helium as the carrier gas) |
| Initial oven temperature: | 50 °C |
| Temperature ramp: | 8 °C/min |
| Final oven temperature: | 310 °C |

Gas chromatography with flame ionization detection ("FID") or with mass spectrometry ("MS") can be used for the identification and quantification of fragrance material in the compositions. Either detection system can be used in conjunction with GC. The column dimensions as well as GC settings described in this method, such as injector temperature, carrier gas velocity, temperature ramp and final oven temperature can be adjusted to optimize the response of the fragrance material and internal standard being monitored. The detection system settings, such as FID gas flows and temperature or MS parameters, should be optimized by a trained analyst to enable the precise detection and quantification of the analytes of interest.
8. The peak area of the fragrance material and internal standard are recorded. The peak area ratio of the fragrance material and the internal standard is calculated at each time point for each sample composition and the % loss from T=0 calculated. Significance is determined by comparison with the % loss for the same fragrance material or fragrance mixture in the control composition.

### EXAMPLES

Absorbent articles according to the present invention can be prepared using the inventive fragrance composition described below and adding an amount of the composition e.g. in liquid form on the surface of the articles, for example on the topsheet of diapers or sanitary napkins. The liquid fragrance composition can be added in droplets at a central position of the absorbent article for example in an amount of about 200 milligrams per absorbent article.

Fragrance examples 1, 2, 3, 4 and 5 are provided below in Tables 6, 7, 8, 9 and 10, respectively, as non-limiting examples of formulations of fragrance materials intended to form the fragrance component of the compositions of the present invention. The exemplary formulations of the fragrance materials span the range from "simple accords" (<10 fragrance materials) to "complex fragrances" (> 30 fragrance materials). Typically, full bodied fragrance compositions do not comprise less than about 30 fragrance materials.

Fragrance example 6 provided in Table 11 below as example of a formulation of volatile fragrance materials in equimolar proportions, particularly suited to analytical evaporation tests and not intended for olfactive assessments.

Fragrance examples 7 and 8 are provided in Tables 12 and 13 below as examples of a formulation of fragrance materials intended to form the fragrance component that fall outside the scope of the present invention.

Fragrance examples 9 to 16 are provided in Tables 14 and 15 below as examples of formulations of fragrance materials containing higher than 30 wt% of the low volatile fragrance materials.

Fragrance examples 17 and 18 are provided in Tables 16 and 17 below as comparative samples of formulations of fragrance materials intended to form the fragrance component.

The following fragrance formulations are made by mixing the listed ingredients in the listed proportions (wt%) at room temperature, wherein the wt% is relative to the total weight of the fragrance component.

**Table 6 - Fragrance Example 1 (Fresh Floral Accord - 10 wt% of Low Volatile Fragrance Materials**

| **Ingredients** | **CAS Number** | **Vapor Pressure (Torr at 25°C)** | **Vapor Pressure (Pa at 25°C)** | **Parts (wt%)** |
|---|---|---|---|---|
| Benzyl acetate | 140-11-4 | 0.1640 | 21.865 | 10.8 |
| Linalool | 78-70-6 | 0.0905 | 12.066 | 9.8 |
| Phenethyl alcohol | 60-12-8 | 0.0741 | 9.8792 | 15.7 |
| Indole | 120-72-9 | 0.0298 | 3.9730 | 1.0 |
| α-Terpineol | 98-55-5 | 0.0283 | 3.7730 | 2.9 |
| Geranyl acetate | 105-87-3 | 0.0256 | 3.4131 | 4.9 |
| Cymal | 103-95-7 | 0.00881 | 1.1746 | 5.9 |
| Hydroxycitronellal | 107-75-5 | 0.00318 | 0.42397 | 22.4 |
| Majantol | 103694-68-4 | 0.00224 | 0.29864 | 16.6 |
| Hexyl cinnamic aldehyde | 101-86-0 | 0.000697 | 0.092926 | 10.0 |
| Total | | | | 100.00 |

**Table 7 - Fragrance Example 2 (Fresh Male Accord - 13.51 wt% of Low Volatile Fragrance Materials**

| **Ingredients** | **CAS Number** | **Vapor Pressure (Torr at 25°C)** | **Vapor Pressure (Pa at 25°C)** | **Parts (wt%)** |
|---|---|---|---|---|
| d-Limonene | 5989-27-5 | 1.540000 | 205.32 | 10.0 |
| Dihydromyrcenol | 18479-58-8 | 0.166000 | 22.132 | 10.0 |
| Boisiris | 68845-00-1 | 0.013500 | 1.7999 | 6.5 |
| Canthoxal | 5462-06-6 | 0.010200 | 1.3599 | 8.0 |
| Helional | 1205-17-0 | 0.002700 | 0.35997 | 10.0 |
| Kephalis | 36306-87-3 | 0.002690 | 0.35864 | 20.0 |
| Majantol | 103694-68-4 | 0.002240 | 0.29864 | 15.5 |
| Javanol^{®} | 198404-98-7 | 0.000902 | 0.12026 | 5.0 |
| Galaxolide* | 1222-05-5 | 0.000414 | 0.055195 | 7.5 |
| Isopropyl Myristate | 110-27-0 | -- | -- | 7.5 |
| Total | | | | 100.00 |

| | | | | |
|---|---|---|---|---|
| * Supplied at 50% in Isopropyl myristate. | | | | |

**Table 8 - Fragrance Example 3 (Sweet Dream 18 Fragrance - 11.15 wt% of Low Volatile Fragrance Materials)**

| **Ingredients** | **CAS Number** | **Vapor Pressure (Torr at 25°C)** | **Vapor Pressure (Pa at 25°C)** | **Parts (wt%)** |
|---|---|---|---|---|
| Prenyl acetate | 1191-16-8 | 3.99000000 | 531.96 | 0.100 |
| Manzanate | 39255-32-8 | 2.91000000 | 387.97 | 0.200 |
| Hexyl acetate | 142-92-7 | 1.39000000 | 185.32 | 0.700 |
| cis-3-Hexenyl acetate | 3681-71-8 | 1.22000000 | 162.65 | 0.200 |
| Benzaldehyde | 100-52-7 | 0.97400000 | 129.86 | 0.200 |
| Liffarome | 67633-96-9 | 0.72100000 | 96.125 | 0.150 |
| Hexyl isobutyrate | 2349-07-7 | 0.41300000 | 55.062 | 0.055 |
| Dihydromyrcenol | 18479-58-8 | 0.16600000 | 22.132 | 2.500 |
| Benzyl acetate | 140-11-4 | 0.16400000 | 21.865 | 0.700 |
| Linalyl acetate | 115-95-7 | 0.11600000 | 15.465 | 2.500 |
| Verdox | 88-41-5 | 0.10300000 | 13.732 | 4.000 |
| Phenethyl alcohol | 60-12-8 | 0.07410000 | 9.8792 | 8.000 |
| Rossitol | 215231-33-7 | 0.02990000 | 3.9863 | 1.500 |
| alpha-Terpineol | 98-55-5 | 0.02830000 | 3.7730 | 1.500 |
| Geranyl acetate | 105-87-3 | 0.02560000 | 3.4131 | 1.500 |
| Rhodinol | 141-25-3 | 0.01970000 | 2.6265 | 0.700 |
| Givescone | 57934-97-1 | 0.01710000 | 2.2798 | 0.700 |
| Methyl anthranilate | 134-20-3 | 0.01580000 | 2.1065 | 0.050 |
| Ysamber K | 154171-77-4 | 0.01470000 | 1.9598 | 1.000 |
| alpha-Ionone | 127-41-3 | 0.01440000 | 1.9198 | 3.000 |
| Citronellyl acetate | 150-84-5 | 0.01370000 | 1.8265 | 0.500 |
| cis-3-hexenyl-cis-3-hexenoate | 61444-38-0 | 0.01220000 | 1.6265 | 0.200 |
| Cinnamic alcohol | 104-54-1 | 0.01170000 | 1.5599 | 0.100 |
| delta-damascone | 57378-68-4 | 0.01020000 | 1.3599 | 0.200 |
| Citronellyloxyacetald ehyde | 7492-67-3 | 0.00967000 | 1.2892 | 0.100 |
| Cymal | 103-95-7 | 0.00881000 | 1.1746 | 0.500 |
| Floralozone | 67634-15-5 | 0.00808000 | 1.0772 | 0.100 |
| Ethylmethylphenylgl ycidate | 77-83-8 | 0.00571000 | 0.76127 | 0.200 |
| Florosa Q | 63500-71-0 | 0.00557000 | 0.74261 | 3.000 |
| Ethyl linalool | 10339-55-6 | 0.00520000 | 0.69328 | 6.400 |
| Pivarose | 67662-96-8 | 0.00484000 | 0.64528 | 2.500 |
| Hydroxycitronellal | 107-75-5 | 0.00318000 | 0.42397 | 7.500 |
| Methyl Ionone | 7779-30-8 | 0.00286000 | 0.38130 | 4.000 |
| gamma-Undecalactone | 104-67-6 | 0.00271000 | 0.36130 | 0.500 |
| Kephalis | 36306-87-3 | 0.00269000 | 0.35864 | 5.000 |
| Cashmeran | 33704-61-9 | 0.00269000 | 0.35864 | 1.000 |
| Magnolan | 27606-09-3 | 0.00251000 | 0.33464 | 3.000 |
| Majantol | 103694-68-4 | 0.00224000 | 0.29864 | 6.900 |
| Brahmanol | 72089-08-8 | 0.00154000 | 0.20532 | 3.000 |
| Coumarin | 91-64-5 | 0.00130000 | 0.17332 | 0.500 |
| Glycolierral | 68901-32-6 | 0.00121000 | 0.16132 | 0.100 |
| Raspberry ketone | 5471-51-2 | 0.00106000 | 0.14132 | 0.100 |
| Top Mango base*³* | -- | -- | -- | 0.500 |
| Cherry base *³* | -- | -- | -- | 0.200 |
| Cassis base *³* | -- | -- | -- | 0.300 |
| Bergamot Oil *⁴* | -- | -- | -- | 6.000 |
| Prunella base *³* | -- | -- | -- | 0.500 |
| Hexyl cinnamic aldehyde | 101-86-0 | 0.00069700 | 0.092926 | 1.500 |
| Sandalore | 65113-99-7 | 0.00062500 | 0.083326 | 3.000 |
| Dupical | 30168-23-1 | 0.00044100 | 0.058795 | 0.005 |
| Galaxolide *¹* | 1222-05-5 | 0.00041400 | 0.055195 | 1.500 |
| Ebanol | 67801-20-1 | 0.00028100 | 0.037464 | 2.000 |
| Helvetolide | 141773-73-1 | 0.00005790 | 0.0077194 | 2.000 |
| Warm Milk base *⁵* | -- | -- | -- | 0.200 |
| Vanilla Absolute ^{*2*, *6*} | -- | -- | -- | 0.100 |
| Isopropyl Myristate | -- | -- | -- | 1.500 |
| Dipropylene Glycol | -- | -- | -- | 6.040 |
| | | | Total | 100.00 |

| | | | | |
|---|---|---|---|---|
| *¹* Supplied at 50% in IPM. *²* Supplied at 50% in DiPG. *³* Proprietary bases that contain a mixture of perfume raw materials, judged to be of high volatility for the purposes of calculating % of low volatility PRMs. *⁴* Natural oils or extracts that contain a mixture of perfume raw materials, judged to be of high volatility for the purposes of calculating % of low volatility PRMs. ⁵ Proprietary bases that contain a mixture of perfume raw materials, judged to be of low volatility for the purposes of calculating % of low volatility PRMs. *⁶* Natural oils or extracts that contain a mixture of perfume raw materials, judged to be of low volatility for the purposes of calculating % of low volatility PRMs. | | | | |

**Table 9 - Fragrance Example 4 (Floral Magnifica Mod1 - 24.7 wt% of Low Volatile Fragrance Materials**

| **Ingredients** | **CAS Number** | **Vapor Pressure (Torr at 25°C)** | **Vapor Pressure (Pa at 25°C)** | **Parts (wt%)** |
|---|---|---|---|---|
| Beta Gamma Hexenol | 928-96-1 | 2.126000 | 283.44 | 0.20 |
| Cis 3 Hexenyl Acetate | 3681-71-8 | 1.219000 | 162.52 | 0.30 |
| Benzyl Acetate | 140-11-4 | 0.304000 | 40.530 | 2.00 |
| Liffarome | 67633-96-9 | 0.721000 | 96.125 | 0.20 |
| Ligustral Or Triplal | 68039-49-6 | 0.578000 | 77.060 | 0.10 |
| Methyl Pamplemousse | 67674-46-8 | 0.214000 | 28.531 | 0.40 |
| Phenyl Acetaldehyde *¹* | | 0.368000 | 49.063 | 0.0002 |
| Precyclemone B | 52475-86-2 | 0.003810 | 0.50796 | 0.20 |
| Ethyl 2 4-Decadienoate | 3025-30-7 | 0.009540 | 1.2719 | 0.20 |
| Ambronat | 6790-58-5 | 0.009340 | 1.2452 | 2.00 |
| Alpha Damascone | 24720-09-0 | 0.008300 | 1.1066 | 0.06 |
| Butylated Hydroxy Toluene | 128-37-0 | 0.006240 | 0.83193 | 0.35 |
| Citronellol | 106-22-9 | 0.032900 | 4.3863 | 1.00 |
| Cyclemax | 7775-00-0 | 0.018200 | 2.4265 | 0.40 |
| Cyclo Galbanate | 68901-15-5 | 0.003230 | 0.43063 | 0.10 |
| Cymal | 103-95-7 | 0.008810 | 1.1746 | 1.50 |
| Dimethyl Benzyl Carbinyl Butyrate | 10094-34-5 | 0.001680 | 0.22398 | 0.50 |
| Ethyl Linalool | 10339-55-6 | 0.005200 | 0.69328 | 12.00 |
| Florol | 63500-71-0 | 0.005570 | 0.74261 | 9.67 |
| Gamma Decalactone | 706-14-9 | 0.008520 | 1.1359 | 0.20 |
| Geraniol | 106-24-1 | 0.013300 | 1.7732 | 5.00 |
| Geranyl Acetate | 105-87-3 | 0.009760 | 1.3012 | 2.00 |
| Helional | 1205-17-0 | 0.002700 | 0.35997 | 3.00 |
| Heliotropin | 120-57-0 | 0.010400 | 1.3866 | 0.20 |
| Hydroxycitronellal | 107-75-5 | 0.003180 | 0.42397 | 4.00 |
| Ionone Beta | 14901-07-6 | 0.003080 | 0.41063 | 0.40 |
| Ionone Gamma Methyl | 127-51-5 | 0.002820 | 0.37597 | 3.00 |
| Jasmal | 18871-14-2 | 0.004340 | 0.57862 | 5.00 |
| Jasmolactone | 32764-98-0 | 0.003550 | 0.47329 | 0.20 |
| Linalyl Propionate | 144-39-8 | 0.026300 | 3.5064 | 1.20 |
| Magnolan 690304 | 27606-09-3 | 0.002510 | 0.33464 | 3.00 |
| Majantol | 103694-68-4 | 0.002240 | 0.29864 | 3.00 |
| Phenyl Ethyl Alcohol | 60-12-8 | 0.074100 | 9.8792 | 5.00 |
| Phenyl Hexanol | 55066-48-3 | 0.006370 | 0.84926 | 3.00 |
| Undecavertol | 81782-77-6 | 0.010700 | 1.4265 | 2.00 |
| Vanillin | 121-33-5 | 0.001940 | 0.25865 | 0.10 |
| cis-3-Hexenyl cis-3-Hexenoate | 61444-38-0 | 0.012200 | 1.6265 | 0.10 |
| Phenoxy Ethyl Iso Butyrate | 103-60-6 | 0.005620 | 0.74927 | 0.50 |
| 5-Cyclohexadecen-1-One | 37609-25-9 | 0.000033 | 0.0043996 | 4.00 |
| Ambrettolide | 28645-51-4 | 0.000001 | 0.00013332 | 5.00 |
| Cis-3-Hexenyl Salicylate | 65405-77-8 | 0.000246 | 0.032797 | 1.00 |
| Delta Muscenone 962191 | 63314-79-4 | 0.000165 | 0.021998 | 1.00 |
| Hedione Hc | 24851-98-7 | 0.000710 | 0.094659 | 8.00 |
| Iso E Super Or Wood | 54464-57-2 | 0.000538 | 0.071727 | 5.00 |
| Para Hydroxy Phenyl Butanone | 5471-51-2 | 0.001060 | 0.14132 | 0.20 |
| Polysantol | 107898-54-4 | 0.000117 | 0.015599 | 0.50 |
| Orange Terpenes *²* | 8028-48-6 | n/a | n/a | 3.00 |
| Neo Hivernal | 300371-33-9 | n/a | n/a | 0.20 |
| Total | | | | 100.00 |

| | | | | |
|---|---|---|---|---|
| *¹* 1% in DPG. *²* Natural and assumed to be a volatile fragrance material for calculations. | | | | |

**Table 10 - Fragrance Example 5 (Muguesia Magnifica Mod1 - 26.2 wt% of Low Volatile Fragrance Materials)**

| **Ingredients** | **CAS Number** | **Vapor Pressure (Torr at 25°C)** | **Vapor Pressure (Pa at 25°C)** | **Parts (wt%)** |
|---|---|---|---|---|
| Benzyl Alcohol | 100-51-6 | 0.158000 | 21.065 | 0.10 |
| Methyl Phenyl Carbinyl Acetate | 93-92-5 | 0.203000 | 27.064 | 0.40 |
| Benzyl Acetate | 140-11-4 | 0.304000 | 40.530 | 7.30 |
| Beta Gamma Hexenol | 928-96-1 | 2.126000 | 283.44 | 0.40 |
| Cis 3 Hexenyl Acetate | 3681-71-8 | 1.219000 | 162.52 | 0.20 |
| Linalyl Acetate | 115-95-7 | 0.077400 | 10.319 | 1.00 |
| Jasmal | 18871-14-2 | 0.004340 | 0.57862 | 4.00 |
| Indol | 120-72-9 | 0.029800 | 3.9730 | 0.10 |
| Hydroxycitronellal | 107-75-5 | 0.003180 | 0.42397 | 4.00 |
| Helional | 1205-17-0 | 0.002700 | 0.35997 | 2.00 |
| Geranyl Acetate | 105-87-3 | 0.009760 | 1.3012 | 4.00 |
| Geraniol | 106-24-1 | 0.013300 | 1.7732 | 4.00 |
| Florol | 63500-71-0 | 0.005570 | 0.74261 | 5.00 |
| Butylated Hydroxy Toluene | 128-37-0 | 0.006240 | 0.83193 | 0.34 |
| Cinnamic Alcohol | 104-54-1 | 0.005720 | 0.76260 | 0.20 |
| Cis Jasmone | 488-10-8 | 0.020100 | 2.6798 | 0.50 |
| Citronellol | 106-22-9 | 0.032900 | 4.3863 | 3.00 |
| Citronellyl Acetate | 150-84-5 | 0.013700 | 1.8265 | 4.00 |
| Citronellyl Oxyacetaldehyde | 7492-67-3 | 0.009670 | 1.2892 | 0.10 |
| Cyclemax | 7775-00-0 | 0.018200 | 2.4265 | 0.40 |
| Cyclo Galbanate | 68901-15-5 | 0.003230 | 0.43063 | 0.20 |
| Cymal | 103-95-7 | 0.008810 | 1.1746 | 2.00 |
| Ethyl Linalool | 10339-55-6 | 0.005200 | 0.69328 | 10.00 |
| Florhydral | 125109-85-5 | 0.020700 | 2.7598 | 0.20 |
| Majantol | 103694-68-4 | 0.002240 | 0.29864 | 4.00 |
| Phenyl Ethyl Acetate | 103-45-7 | 0.056400 | 7.5194 | 0.40 |
| Phenyl Ethyl Alcohol | 60-12-8 | 0.074100 | 9.8792 | 15.00 |
| Ambrettolide | 28645-51-4 | 0.000001 | 0.00013332 | 5.00 |
| Hexyl Cinnamic Aldehyde | 101-86-0 | 0.000123 | 0.016399 | 0.06 |
| Cis-3-Hexenyl Salicylate | 65405-77-8 | 0.000246 | 0.032797 | 3.00 |
| Hedione Hc | 24851-98-7 | 0.000710 | 0.094659 | 8.00 |
| Iso E Super Or Wood | 54464-57-2 | 0.000538 | 0.071727 | 10.00 |
| Phenyl Acetaldehyde Dimethyl Acetal | 101-48-4 | 0.000538 | 0.071727 | 0.10 |
| Orange Terpenes ¹ | 8028-48-6 | n/a | n/a | 1.00 |

| | | | | |
|---|---|---|---|---|
| *¹* Natural and assumed to be a volatile fragrance material for calculations. | | | | |

**Table 11 - Fragrance Example 6 (Volatile Accord - 0 wt% of Low Volatile Fragrance Materials**

| **Ingredients** | **CAS Number** | **Vapor Pressure (Torr at 25°C)** | **Vapor Pressure (Pa at 25°C)** | **Parts (wt%)** |
|---|---|---|---|---|
| Lauric aldehyde | 112-54-9 | 0.0344 | 4.5863 | 6.229044 |
| Terpinyl acetate | 80-26-2 | 0.0392 | 5.2262 | 6.633490 |
| Dimethyl Benzyl Carbinyl Acetate | 151-05-3 | 0.0139 | 1.8532 | 6.497235 |
| Rosaphen 600064 | 25634-93-9 | 0.00637 | 0.84926 | 6.024662 |
| Dimethyl Benzyl Carbinol | 100-86-7 | 0.0888 | 11.839 | 5.076607 |
| Ethyl Safranate | 35044-59-8 | 0.0266 | 3.5464 | 6.565362 |
| Indocolore | 2206-94-2 | 0.0255 | 3.3997 | 5.481052 |
| Diphenyl Oxide | 101-84-8 | 0.0223 | 2.9731 | 5.752156 |
| Phenyl Hexanol | 55066-48-3 | 0.00637 | 0.84926 | 6.024662 |
| Phenyl ethyl alcohol | 60-12-8 | 0.074100 | 9.8792 | 4.128603 |
| Fructalate | 72903-27-6 | 0.00274000 | 0.3653 | 7.714998 |
| Flor Acetate | 5413-60-5 | 0.0137 | 1.8265 | 6.497235 |
| Heliotropin | 120-57-0 | 0.0104 | 1.3866 | 5.073694 |
| Laevo Carvone | 6485-40-1 | 0.0656 | 8.7459 | 5.076607 |
| Citrowanil B | 97384-48-0 | 0.00265 | 0.3533 | 5.787002 |
| Safraleine | 54440-17-4 | 0.0126 | 1.6799 | 5.888411 |
| Eugenol | 97-53-0 | 0.0104 | 1.3866 | 5.549180 |
| Total | | | | 100.00 |

**Table 12 - Fragrance Example 7 (Fresh Floral GF 6-7 Accord - 40.14 wt% of Low Volatile Fragrance Materials)**

| **Ingredients** | **CAS Number** | **Vapor Pressure (Torr at 25°C)** | **Vapor Pressure (Pa at 25°C)** | **Parts (wt%)** |
|---|---|---|---|---|
| Ligustral or Triplal | 68039-49-6 | 0.578000 | 77.060 | 0.15 |
| Benzyl acetate | 140-11-4 | 0.164000 | 21.865 | 0.31 |
| Verdox | 88-41-5 | 0.103000 | 13.732 | 5.38 |
| Phenethyl alcohol | 60-12-8 | 0.074100 | 9.8792 | 1.54 |
| Indole | 120-72-9 | 0.029800 | 3.9730 | 0.02 |
| Heliotropin | 120-57-0 | 0.010400 | 1.3866 | 1.23 |
| gamma-Decalactone | 706-14-9 | 0.008520 | 1.1359 | 0.38 |
| Florol | 63500-71-0 | 0.005570 | 0.74261 | 15.38 |
| Ethyl linalool | 10339-55-6 | 0.005200 | 0.69328 | 26.15 |
| Isoeugenol | 97-54-1 | 0.005190 | 0.69194 | 0.08 |
| alpha-Irone | 79-69-6 | 0.004190 | 0.55862 | 1.54 |
| Vanillin | 121-33-5 | 0.001940 | 0.25865 | 6.15 |
| Dimethyl benzyl carbinyl butyrate | 10094-34-5 | 0.001680 | 0.22398 | 1.54 |
| Methyl betanaphthyl ketone | 93-08-3 | 0.000957 | 0.12759 | 0.77 |
| Methyl dihydrojasmonate | 24851-98-7 | 0.000710 | 0.094659 | 30.60 |
| Benzyl salicylate | 118-58-1 | 0.000175 | 0.023331 | 7.69 |
| Polysantol | 107898-54-4 | 0.000117 | 0.015599 | 0.77 |
| Lrg 201 | 4707-47-5 | 0.000029 | 0.0038663 | 0.31 |
| | | | Total | 100.00 |

**Table 13 - Fragrance Example 8 (Traditional Floral Accord - 54.00 wt% of Low Volatile Fragrance Materials)**

| **Ingredients** | **CAS Number** | **Vapor Pressure (Torr at 25°C)** | **Vapor Pressure (Pa at 25°C)** | **Parts (wt%)** |
|---|---|---|---|---|
| Benzyl acetate | 140-11-4 | 0.1640 | 21.865 | 5.5 |
| Linalool | 78-70-6 | 0.0905 | 12.066 | 5.0 |
| Phenethyl alcohol | 60-12-8 | 0.0741 | 9.8792 | 8.0 |
| Indole | 120-72-9 | 0.0298 | 3.9730 | 0.5 |
| α-Terpineol | 98-55-5 | 0.0283 | 3.7730 | 1.5 |
| Geranyl acetate | 105-87-3 | 0.0256 | 3.4131 | 2.5 |
| Cymal | 103-95-7 | 0.00881 | 1.1746 | 3.0 |
| Hydroxycitronellal | 107-75-5 | 0.00318 | 0.42397 | 11.5 |
| Majantol | 103694-68-4 | 0.00224 | 0.29864 | 8.5 |
| Hexyl cinnamic aldehyde | 101-86-0 | 0.000697 | 0.092926 | 4.0 |
| iso gamma super | 68155-66-8 | 0.000565 | 0.075327 | 12.50 |
| Sandalore | 65113-99-7 | 0.000625 | 0.083326 | 18.75 |
| Habanolide | 111879-80-2 | 0.00000431 | 0.00057462 | 18.75 |
| Total | | | | 100.00 |

**Table 14 - Fragrance Examples 9, 10, 11 and 12 (Traditional Flora Magnifica - > 30 wt% of Low Volatile Fragrance Materials)**

| **Ingredients** | **Fragrance Example 9** | **Fragrance Example 10** | **Fragrance Example 11** | **Fragrance Example 12** |
|---|---|---|---|---|
| | **Weight %** | **Weight %** | **Weight %** | **Weight %** |
| Flora Magnifica MOD1 *¹* | 86.96 | 83.33 | 74.07 | 68.97 |
| Ethylene Brassylate | 4.35 | 4.167 | 3.704 | 6.90 |
| Methyl Dihydro Jasmonate | 4.35 | 8.33 | 14.82 | 13.79 |
| Iso E super | 4.35 | 4.167 | 7.407 | 10.35 |
| Total | 100.00 | 100 | 100 | 100.00 |
| Wt% Low Volatile Fragrance Materials | 34.53 | 37.25 | 44.23 | 48.07 |

| | | | | |
|---|---|---|---|---|
| *¹* Fragrance Example 4. | | | | |

**Table 15 - Fragrance Examples 13, 14, 15 and 16 (Traditional Muguesia Magnifica - > 30 wt% of Low Volatile Fragrance Materials)**

| **Ingredients** | **Fragrance Example 13** | **Fragrance Example 14** | **Fragrance Example 15** | **Fragrance Example 16** |
|---|---|---|---|---|
| | **Weight %** | **Weight %** | **Weight %** | **Weight %** |
| Muguesia Magnifica MOD1 *¹* | 86.96 | 83.33 | 74.07 | 68.97 |
| Ethylene Brassylate | 4.35 | 4.17 | 3.70 | 6.90 |
| Methyl Dihydro Jasmonate | 4.35 | 8.33 | 14.82 | 13.79 |
| Iso E super | 4.35 | 4.17 | 7.41 | 10.35 |
| Total | 100.00 | 100.00 | 100.00 | 100.00 |
| Wt% Low Volatile Fragrance Materials | 35.79 | 38.47 | 45.304 | 49.08 |

| | | | | |
|---|---|---|---|---|
| *¹* Fragrance Example 5. | | | | |

Fragrance example 17 (as disclosed in Table 16) is composed of 30.30 wt% of high volatile fragrance materials, 38.22 wt% of moderate volatile fragrance materials and 31.49 wt% of low volatile fragrance materials, wherein the wt% is relative to the total weight of the fragrance component.

**Table 16 - Fragrance Example 17 (Comparative Fragrance 1 - 31.49 wt% of Low Volatile Fragance Materials**

| **Ingredients** | **CAS Number** | **Vapor Pressure (Torr at 25 °C)** | **Vapor Pressure (Pa at 25 °C)** | **Amount** | |
|---|---|---|---|---|---|
| | | | | **Parts by Weight** | **Parts (wt%)** |
| Limonene | 5989-27-5 | 1.541 | 205.45 | 2576 | 30.04 |
| Cis-3-Hexenol | 928-96-1 | 1.039 | 138.52 | 21 | 0.24 |
| Zestover *⁶* | 78-70-6 | 0.578 | 77.060 | 1 | 0.01 |
| Linalol | 78-70-6 | 0.0905 | 12.066 | 553 | 6.45 |
| Aphermate *⁴* (10% DIPG) *⁷* | 25225-08-5 | 0.0678 | 9.0393 | 7 | 0.08 |
| Cyclosal | 535-86-4 | 0.0311 | 4.1463 | 35 | 0.41 |
| Coranol | 83926-73-2 | 0.0210 | 2.7998 | 371 | 4.33 |
| Sclareolate^{®}* *¹* | 319002-92-1 | 0.0196 | 2.6131 | 630 | 7.35 |
| 3-Methoxy -7,7 - dimethyl-10-methylene-bicyclo[ 4.3.1]decane | 216970-21-7 | 0.0196 | 2.6131 | 371 | 4.33 |
| Cedramber *²* | 19870-74-8 | 0.0128 | 1.7065 | 1050 | 12.24 |
| Ambrox^{®}* | 3738-00-9 | 0.00934 | 1.2452 | 1 | 0.01 |
| Decal | 706-14-9 | 0.00852 | 1.1359 | 21 | 0.24 |
| Damascone Alpha* (10% DIPG) *⁷* | 24720-09-0 | 0.00830 | 1.1066 | 9.1 | 0.11 |
| (Methoxymethoxy)Cy clododecane | 42604-12-6 | 0.00686 | 0.91459 | 182 | 2.12 |
| Lilial^{®} | 80-54-6 | 0.00444 | 0.59195 | 26 | 0.30 |
| γ-Undecalactone* | 104-67-6 | 0.00271 | 0.36130 | 21 | 0.24 |
| Calone^{®}* *³* | 28940-11-6 | 0.000831 | 0.11079 | 50 | 0.58 |
| Paradisone ^{*5*®}* | 24851-98-7 | 0.000710 | 0.094659 | 1000 | 11.66 |
| Galaxolide^{®} (70% MIP Extra) *⁷* | 1222-05-5 | 0.000414 | 0.055195 | 700 | 8.1.6 |
| Exaltenone | 14595-54-1 | 0.0000964 | 0.012852 | 950 | 11.08 |
| Total | | | | 8575.10 | 100 wt% |

| | | | | | |
|---|---|---|---|---|---|
| * origin: Firmenich SA (Geneva, Switzerland). *¹* Propyl (*S*)-2-(1,1-dimethylpropxy)propanoate. *²* 8-Methoxy-2,6,6,8-tetramethyl-tricyclo[5.3.1.0(1,5)]undecane. *³* 7-Methyl-2*H*,4*H*-1,5-benzodioxepin-3-one. *⁴* 1-(3,3-dimethyl-1-cyclohexyl)ethyl formate; origin: International Flavors & Fragrances. *⁵* Methyl dihydrojasmonate. *⁶* Linalool. *⁷* Fragrance materials added as dilutions in a non-volatile solvent. For the purposes of calculating the fragrance oil composition actual fragrance materials levels added are used. | | | | | |

Fragrance example 18 (as disclosed in Table 17) is composed of 18 wt% of high volatile fragrance materials, 69 wt% of moderate volatile fragrance materials and 9 wt% of low volatile fragrance materials, wherein the wt% is relative to the total weight of the fragrance component.

**Table 17 - Fragrance Example 18 (Comparative Fragrance 2 - 9 wt% of Low Volatile Fragance Materials**

| **Ingredients** | **CAS Number** | **Vapor Pressure (Torr at 25 °C)** | **Vapor Pressure (Pa at 25 °C)** | **Amount** | |
|---|---|---|---|---|---|
| | | | | **Parts by Weight** | **Parts (wt%)** |
| D-Limonene | 5989-27-5 | 1.540 | 205.32 | 50.00 | 5.21 |
| cis-3-Hexenol (10% in DPG) *⁴* | 928-96-1 | 1.040 | 138.66 | 0.5 | 0.05 |
| Acetophenone (10% in DPG) *⁴* | 98-86-2 | 0.299 | 39.863 | 1.00 | 0.10 |
| Methylphenyl Acetate | 101-41-7 | 0.176 | 23.465 | 10.00 | 1.04 |
| Dihydromyrcenol | 18479-58-8 | 0.166 | 22.132 | 50.00 | 5.21 |
| Benzyl acetate | 140-11-4 | 0.164 | 21.865 | 60.00 | 6.25 |
| Tetra-Hydro Linalool | n/a | 0.115 | 15.332 | 50.00 | 5.21 |
| n-Undecanal | n/a | 0.102 | 13.599 | 5.00 | 0.52 |
| Linalool | 78-70-6 | 0.0905 | 12.066 | 40.00 | 4.17 |
| Phenylethyl Alcohol | 60-12-8 | 0.0741 | 9.8792 | 245.00 | 25.53 |
| Allyl amyl glycolate (10% in DPG) *⁴* | 67634-00-8 | 0.04000 | 5.3329 | 2.00 | 0.21 |
| Indole (10% in DPG) *⁴* | 120-72-9 | 0.02980 | 3.9730 | 1.00 | 0.10 |
| Alpha-Terpineol | 98-55-5 | 0.02830 | 3.7730 | 30.00 | 3.13 |
| Diphenyl Oxide | 101-84-8 | 0.02230 | 2.9731 | 5.00 | 0.52 |
| L-Citronellol | 7540-51-4 | 0.01830 | 2.4398 | 80.00 | 8.34 |
| Beta-Ionone | 14901-07-6 | 0.01690 | 2.2531 | 5.00 | 0.52 |
| Alpha-Ionone | 127-41-3 | 0.01440 | 1.9198 | 15.00 | 1.56 |
| Dimethyl benzyl carbinyl acetate | 151-05-3 | 0.01390 | 1.8532 | 30.00 | 3.13 |
| Geraniol | 106-24-1 | 0.01330 | 1.7732 | 40.00 | 4.17 |
| Nerol | n/a | 0.01330 | 1.7732 | 20.00 | 2.08 |
| Lilial^{® *1*} | 80-54-6 | 0.00444 | 0.59195 | 60.00 | 6.25 |
| Gamma-Undecalactone | 104-67-6 | 0.00271 | 0.36130 | 15.00 | 1.56 |
| Amyl salicylate | 2050-08-0 | 0.00144 | 0.19198 | 25.00 | 2.61 |
| Galaxolide^{®} | 1222-05-5 | 0.000414 | 0.055195 | 20.00 | 2.08 |
| cis-3-Hexenyl salicylate | 65405-77-8 | 0.000246 | 0.032797 | 20.00 | 2.08 |
| Ethylene Brassylate | 105-95-3 | 0.00000000313 | 4.1730E-07 | 30.00 | 3.13 |
| Styrolyl Acetate⁵ | n/a | n/a | n/a | 20.00 | 2.08 |
| Decenol trans-9 *³* | n/a | n/a | n/a | 15.00 | 1.56 |
| Geranium oil *²* | n/a | n/a | n/a | 15.00 | 1.56 |
| Total | | | | 959.5 | 100 wt% |

| | | | | | |
|---|---|---|---|---|---|
| *¹* Benzenepropanal, 4-(1,1-dimethylethyl)-α-methyl-. *²* Natural oil that is judged to be volatile for the purposes of calculating levels of the volatile fragrance materials. *³* Proprietary oil that is judged to be volatile for the purposes of calculating levels of the volatile fragrance materials. *⁴* Fragrance materials added as dilutions in a non-volatilee solvent. For the purposes of calculating the fragrance oil composition actual fragrance materials levels added are used. *⁵* Unknown oil that is judged to be of low volatility for the purposes of calculating levels of the volatile fragrance materials. | | | | | |

### Example 2 - Compositions Comprising Substantially Non-Odorous Fragrance Modulators

Compositions A, D, G, J and M are examples of fragrance compositions according to the present invention, made with any one of fragrance examples 1 to 5 or fragrance compositions comprising 17 fragrance materials of fragrance example 6, respectively. Compositions B, E, H, K and N are compositions containing fragrance examples (namely fragrance examples 7-17) outside the scope of the present invention. All of the compositions are prepared by admixture of the components described in Table 18, in the proportions indicated. In parallel, control compositions C, F, I, L and O are prepared by replacing the different substantially non-odorous fragrance modulators by the same amount of deionized water or ethanol.

**Table 18 - Fragrance Compositions**

| **Ingredients** | **Fragrance Composition (wt%) *¹*** | | | | | | | | | | | | | | |
|---|---|---|---|---|---|---|---|---|---|---|---|---|---|---|---|
| | **A** | **B** | **C** | **D** | **E** | **F** | **G** | **H** | **I** | **J** | **K** | **L** | **M** | **N** | **O** |
| Fragrance A1 *²* | 5-10 | - | - | 0.01 -2 | - | - | 3-10 | - | - | 5-10 | - | - | 0.1-5 | - | - |
| Fragrance B *³* | - | 5-10 | - | - | 0.01 -2 | - | - | 3-10 | - | - | 5-10 | - | - | 0.1-5 | - |
| Fragrance A1 or B | - | - | 5-10 | - | - | 0.01 -2 | - | - | 3-10 | - | - | 5-10 | - | - | 0.1-5 |
| Ethanol | 60-99.99 | | | | | | | | | | | | | | |
| Modulator A *⁴* | 5-20 | 5-20 | - | - | - | - | - | - | - | - | - | - | - | - | - |
| Modulator B*⁵* | - | - | - | 0.1 | 0.1 | - | - | - | - | - | - | - | - | - | - |
| Modulator C *⁶* | - | - | - | - | - | - | 0.1-5 | 0.1-5 | - | - | - | - | - | - | - |
| Modulator D*⁷* | - | - | - | - | - | - | - | - | - | 2-10 | 2-10 | - | - | - | - |
| Modulator E *⁸* | - | - | - | - | - | - | - | - | - | - | - | - | 0.1-3 | 0.1-3 | - |
| Deionized water | to 100.00 | | | | | | | | | | | | | | |

| | | | | | | | | | | | | | | | |
|---|---|---|---|---|---|---|---|---|---|---|---|---|---|---|---|
| *¹* Wt% is relative to the total weight of the composition. *²* Can be any one of fragrance examples 1-6. *³* Can be any one of fragrance examples 7-17. *⁴* Can be any one of the substantially non-odorous fragrance modulators examples: ryoto sugar ester; Sucrose Laurate; sucrose sterate; 1-ethoxyethoxy-cyclododecane; 1,1,4,4-Tetramethyl-6-acetyl-7-formyl-1,2,3,4-tetrahydronaphthalene; or 1,1,2,4,4-pentamethyl-6-acetyl-7-formyl-1,2,3,4-tetrahydronaphthalene. *⁵* Substantially non-odorous fragrance modulator is (E)-1-(2,2,6-trimethylcyclohexyl)oct-1-en-3-one. *⁶* Can be any one of the substantially non-odorous fragrance modulators examples: 2-(*1-*methoxy) ethane-1-ol; 1-(*1*-menthoxy) propane-2-ol; 3-(*1*-menthoxy) propane-1-ol; 3-(*1-*menthoxy) propane-1,2-diol; 2-methyl-3-(*1*-menthoxy)propane-1,2-diol; or 4-(*1*-menthoxy) butane-1-ol. *⁷* Substantially non-odorous fragrance modulator is Hydroquinone beta-D-glycoside. *⁸* Substantially non-odorous fragrance modulator is Hyaluronic acid or Sodium Hyaluronate | | | | | | | | | | | | | | | |

Compositions AA, DD, GG, JJ and MM are examples of fine fragrance compositions according to the present invention, made with fragrance examples 1 to 5 or fragrance compositions comprising 17 fragrance materials of fragrance example 6, respectively. Compositions BB, EE, HH, KK and NN are compositions containing fragrance examples (namely fragrance examples 7-17) outside the scope of the present invention. All of the compositions are prepared by admixture of the components described in Table 19a, in the proportions indicated. In parallel, control compositions CC, FF, II, LL and OO are prepared by replacing the different substantially non-odorous fragrance modulators by the same amount of deionized water.

**Table 19a - Fragrance Compositions**

| **Ingredient s** | **Fragrance Composition (wt%) *¹*** | | | | | | | | | | | | | | |
|---|---|---|---|---|---|---|---|---|---|---|---|---|---|---|---|
| | **A A** | **B B** | **C C** | **DD** | **EE** | **F F** | **GG** | **HH** | **II** | **JJ** | **KK** | **L L** | **M M** | **NN** | **OO** |
| Fragrance A1*²* | 5-10 | - | - | 5-15 | - | - | 2.5 -10 | - | - | 5-20 | - | - | 0.1-20 | - | - |
| Fragrance B *³* | - | 5-10 | - | - | 5-15 | - | - | 2.5 -10 | - | - | 5-20 | - | - | 0.1 -20 | - |
| Fragrance A1 or B | - | - | 5-10 | - | - | 5-15 | - | - | 2.5 -10 | - | - | 5-20 | - | - | 0.1 -20 |
| Ethanol | 60-99.9 | | | | | | | | | | | | | | |
| Modulator A *⁴* | 5-20 | 5-20 | 0 | - | - | - | - | - | - | - | - | - | - | - | - |
| Modulator B*⁵* | - | - | - | 0.5 -5 | 0.5 -5 | 0 | - | - | - | - | - | - | - | - | - |
| Modulator C*⁶* | - | - | - | - | - | - | 0.1 3.0 | 0.1 3.0 | 0 | - | - | - | - | - | - |
| Modulator D*⁷* | - | - | - | - | - | - | - | - | - | 2.5 -15 | 2.5 -15 | 0 | - | - | - |
| Modulator E *8* | - | - | - | - | - | - | - | - | - | - | - | - | 0.1-20 | 0.1 -20 | 0 |
| Deionized water | to 100.00 | | | | | | | | | | | | | | |

| | | | | | | | | | | | | | | | |
|---|---|---|---|---|---|---|---|---|---|---|---|---|---|---|---|
| *¹* Wt% is relative to the total weight of the composition. *²* Can be any one of fragrance examples 1-6. *³* Can be any one of fragrance examples 7-17. *⁴* Can be any one of the substantially non-odorous fragrance modulators examples: Propylene Glycol Propyl Ether, Hexaethylene glycol monododecyl ether, Panthenol Ethyl Ether, DL-Panthenol , Diisobutyl Adipate, Diisoamyl Adipate *⁵* Neopentyl Glycol Diisononanoate *⁶* 2-ethylhexyloxypropanediol *⁷* PPG-11 Stearyl EtherNeopentyl *⁸* Can be any one of the substantially non-odorous fragrance modulators examples: Dicetyl Ether; Polyglycerin-4 Ethers; Isoceteth-5; Isoceteth-7, Isoceteth-10; Isocetteth-12; Isoceteth-15; Isoceteth-20; Isoceteth-25; Isoceteth-30; Disodium Lauroamphodipropionate; or Cetearyl Ethylhexnoate. | | | | | | | | | | | | | | | |

Compositions X1, X4 and X7 are examples of fine fragrance compositions according to the present invention, made with fragrance examples 1 to 5 or fragrance compositions comprising 17 fragrance materials of fragrance example 6, respectively. Compositions X2, X5 and X8 are compositions containing fragrance examples (namely fragrance examples 7-17) outside the scope of the present invention. All of the compositions are prepared by admixture of the components described in Table 19b, in the proportions indicated. In parallel, control compositions X3, X6 and X9 are prepared by replacing the different substantially non-odorous fragrance modulators by the same amount of deionized water.

**Table 19b - Fragrance Compositions**

| **Ingredients** | **Fragraance Composition (wt%) *¹*** | | | | | | | | |
|---|---|---|---|---|---|---|---|---|---|
| | **X1** | **X2** | **X3** | **X4** | **X5** | **X6** | **X7** | **X8** | **X9** |
| Fragrance A1 *²* | 3-10 | | - | 3-10 | - | - | 1-10 | - | - |
| Fragrance B *³* | - | 3-10 | - | - | 3-10 | - | - | 1-10 | - |
| Fragrance A1 or B | - | - | 3-10 | - | - | 3-10 | - | - | 1-10 |
| Ethanol | 60 - 99.9 | | | | | | | | |
| Expert Gel^{®} EG56 | 1-5 | 1-5 | 0 | - | - | - | - | - | - |
| Kolliphor^{®} EL | - | - | | 5-20 | 5-20 | 0 | - | - | - |
| Glycerol Alkxoylates ⁴ | - | - | - | - | - | - | 0.1-20 | 0.1-20 | 0.0 |
| Deionised water | to 100 | | | | | | | | |

| | | | | | | | | | |
|---|---|---|---|---|---|---|---|---|---|
| *¹* Wt% is relative to the total weight of the composition. *²* Can be any one of fragrance examples 1-6. *³* Can be any one of fragrance examples 7-17. *⁴* Can be any one of the substantially non-odorous modulators examples: 3-((2-ethylhexyl)oxy)propane-1,2-diol; 3-((2-propylheptyl)oxy)propane-1,2-diol; or 1-amino-3-((2-ethylhexyl)oxy)propan-2-ol. | | | | | | | | | |

### Example 3 - Single Fragrance Material Compositions containing Substantially Non-Odorous Fragrance Modulators

Compositions P, R, T, V, and W to Z are examples of compositions according to the present invention, made with single fragrance materials and the substantially non-odorous fragrance modulators, respectively. They are prepared by admixture of the components in Table 20, in the proportions indicated. In parallel, control Compositions Q, S, and U are prepared without a substantially non-odorous fragrance modulator as a control.

**Table 20 - Single Fragrance Material Compositions**

| **Ingredients** | **Single PRM Composition (wt%) *¹*** | | | | | | | | | | |
|---|---|---|---|---|---|---|---|---|---|---|---|
| | **P** | **Q** | **R** | **S** | **T** | **U** | **V** | **W** | **X** | **Y** | **Z** |
| Dimethyl Benzyl Carbinol | 1 | 1 | - | - | - | - | - | - | - | - | - |
| Ethyl Safranate | - | | 1 | 1 | - | - | - | - | - | - | - |
| Phenylethyl alcohol | - | - | - | - | 1 | 1 | - | - | - | - | - |
| Fragrance C *⁶* | - | - | - | - | - | - | 0.5-1 | 0.5-1 | 0.5-1 | 0.5-1 | 0.5-1 |
| Ryoto sugar ester | 3.8 | 0 | 3.0 | 0 | 4.6 | 0 | - | - | - | - | - |
| Modulator A2 *²* | - | - | - | - | - | - | 1-5 | - | - | - | - |
| Modulator B *³* | - | - | - | - | - | - | - | 0.1 | - | - | - |
| Modulator C *⁴* | - | - | - | - | - | - | - | - | 0.1-5 | - | - |
| Modulator D *⁵* | - | - | - | - | - | - | - | - | - | 1-4 | - |
| Modulator E *⁷* | | | | | | | | | | | 0.1-3 |
| Ethanol | to 100 | | | | | | | | | | |

| | | | | | | | | | | | |
|---|---|---|---|---|---|---|---|---|---|---|---|
| *¹*Wt% is relative to the total weight of the composition. *²* Can be any one of the substantially non-odorous modulators examples: sucrose laurate, sucrose sterate, 1-ethoxyethoxy-cyclododecane; 1,1,4,4-Tetramethyl-6-acetyl-7-formyl-1,2,3,4-tetrahydronaphthalene; or 1,1,2,4,4-pentamethyl-6-acetyl-7-formyl-1,2,3,4-tetrahydronaphthalene. *³* Can be any one of the substantially non-odorous modulators examples: (*E*)-1-(2,2,6-trimethylcyclohexyl)oct-1-en-3-one. *⁴* Can be any one of the substantially non-odorous modulators examples: 2-(*1*-methoxy) ethane-1-ol; 1-(*1*-menthoxy) propane-2-ol; 3-(*1*-menthoxy) propane-1-ol; 3-(*1*-menthoxy) propane-1,2-diol; 2-methyl-3-(*1*-menthoxy)propane-1,2-diol; or 4-(*1*-menthoxy) butane-1-ol. *⁵* Substantially non-odorous fragrance modulator is Hydroquinone beta-D-glycoside. *⁴* Can be any one of the substantially non-odorous modulators examples: Dimethyl Benzyl Carbinol; Ethyl Safranate or Phenyl ethyl alcohol. *⁷* Substantially non-odorous fragrance modulator is Hyaluronic acid disaccharide sodium salt or Sodium Hyaluronate. | | | | | | | | | | | |

Compositions PP, RR and TT-XX are examples of compositions according to the present invention, made with single fragrance materials and the substantially non-odorous fragrance modulators, respectively. They are prepared by admixture of the components in Table 21, in the proportions indicated. In parallel, control Compositions QQ, SS and YY are prepared without a substantially non-odorous fragrance modulator as a control.

**Table 21 - Single Fragrance Material Compositions**

| **Ingredients** | **Single Fragrance Material Composition (wt%) *¹*** | | | | | | | | | |
|---|---|---|---|---|---|---|---|---|---|---|
| | **PP** | **QQ** | **RR** | **SS** | **TT** | **UU** | **VV** | **WW** | **XX** | **YY** |
| Dimethyl Benzyl Carbinyl Acetate | - | - | 1.0 | 1.0 | - | - | - | - | - | - |
| Eugenol | 1.0 | 1.0 | - | - | - | - | - | - | - | - |
| Fragrance C *⁶* | - | - | - | - | 0.1-1 | 0.1-1 | 0.1-1 | 0.1-1 | 0.1-1 | 0.1-1 |
| Propylene Glycol Propyl Ether | 0.8 | 0.0 | - | - | - | - | - | - | - | - |
| Diisobutyl adipate | - | - | 1.4 | 0.0 | - | - | - | - | - | - |
| Modulator A2 *²* | - | - | - | - | 0.1-5 | - | - | - | - | 0 |
| Modulator B *³* | - | - | - | - | - | 0.1-5 | - | - | - | 0 |
| Modulator C *⁴* | - | - | - | - | - | - | 0.1-5 | - | - | 0 |
| Modulator D *⁵* | - | - | - | - | - | - | - | 0.1-5 | - | 0 |
| Modulator E *⁷* | - | - | - | - | - | - | - | - | 0.1-5 | 0 |
| Ethanol | to 100 | | | | | | | | | |

| | | | | | | | | | | |
|---|---|---|---|---|---|---|---|---|---|---|
| *¹* Wt% is relative to the total weight of the composition. *²* Can be any one of the substantially non-odorous modulators examples: Hexaethylene glycol monododecyl ether, Panthenol Ethyl Ether, DL-Panthenol, or Diisoamyl Adipate. *³* Neopentyl Glycol Diisononanoate. *⁴* 2-ethylhexyloxypropanediol. *⁵* PPG-11 Stearyl EtherNeopentyl. *⁶* Can be any one of the single fragrance materials examples: Dimethyl Benzyl Carbonyl Acetate or Eugenol. *⁷* Can be any one of the substantially non-odorous modulators examples: Dicetyl Ether; Polyglycerin-4 Ethers; Isoceteth-5; Isoceteth-7, Isoceteth-10; Isocetteth-12; Isoceteth-15; Isoceteth-20; Isoceteth-25; Isoceteth-30; Disodium Lauroamphodipropionate; or Cetearyl Ethylhexnoate. | | | | | | | | | | |

Compositions AAA, CCC, EEE, GGG and III are examples of compositions according to the present invention, made with single fragrance materials and the substantially non-odorous fragrance modulators, respectively. They are prepared by admixture of the components in Table 22, in the proportions indicated. In parallel, control Compositions BBB, DDD, FFF, HHH and JJJ are prepared without a substantially non-odorous fragrance modulator as a control.

**Table 22 - Single Fragrance Material Compositions**

| **Ingredients** | **Single Fragrance Material Composition (wt%) *¹*** | | | | | | | | | |
|---|---|---|---|---|---|---|---|---|---|---|
| | **AAA** | **BBB** | **CCC** | **DDD** | **EEE** | **FF** | **GGG** | **HHH** | **III** | **JJJ** |
| | | | | | | **F** | | | | |
| Indocolore | 1.0 | 1.0 | - | - | - | - | - | - | - | - |
| Dimethyl Benzyl Carbinol | - | - | 1.0 | 1.0 | - | - | - | - | - | - |
| Eugenol | - | - | - | - | 1.0 | 1.0 | - | - | - | - |
| Phenylethyl alcohol | - | - | - | - | - | - | 1.0 | 1.0 | - | - |
| Fragrance C *²* | - | - | - | - | - | - | - | - | 1.0 | 1.0 |
| Expert Gel^{®} EG56 | 5.0 | 0.0 | - | - | - | - | - | - | - | - |
| Kolliphor^{®} EL | - | - | 16.6 | 0.0 | 15.2 | 0.0 | 13.0 | 0.0 | - | - |
| Glycerol Alkxoylates *³* | - | - | - | - | - | - | - | - | 0.1-20 | 0.0 |
| Ethanol | to 100 | | | | | | | | | |

| | | | | | | | | | | |
|---|---|---|---|---|---|---|---|---|---|---|
| *¹* Wt% is relative to the total weight of the composition. *²* Can be any one of the single fragrance materials examples: Indocolore, Dimethyl Benzyl Carbinol, Eugenol or Phenethyl alcohol. *³* Can be any one of the substantially non-odorous modulators examples: 3-((2-ethylhexyl)oxy)propane-1,2-diol; 3-((2-propylheptyl)oxy)propane-1,2-diol; or 1-amino-3-((2-ethylhexyl)oxy)propan-2-ol. | | | | | | | | | | |

### Example 4 - Olfactive Tests

Compositions disclosed in Tables 18-21 are applied to glass slides in accordance with the protocol described in the Method Section and a panel of 10 experienced panelists evaluate the perceived fragrance profile at initial time 0, then at various time points, typically 1 hour, 2 hours, 3 hours, 4 hours and 6 hours post application. Panellists are asked to score the compositions for the longevity and/or fidelity of the fragrance profile on a scale of 0 to 5, wherein 0 represents a no fragrance is detected and 5 represents a very strong fragrance intensity is detected. The results of the panellists are then averaged and discussed below.

Figure 2 shows the effect of Composition R, which comprises the substantially non-odorous fragrance modulator Sucrose Myristate and the single fragrance material, Ethyl safranate. Addition of the modulator maintains the intensity of the fragrance material from 0 hour up to 6 hours whilst the control, Composition S, in the absence of the substantially non-odorous fragrance modulator, drops in the fragrance profile intensity over the 6 hours. The substantially non-odorous fragrance modulator acts to maintain the continued evaporation over time of the fragrance material. Statistical analysis using the Tukey correction for multiple comparisons confirms the statistically significant difference at 0 hours (p = 0.0015), 1hour (p = 0.0033), 3hours (p = 0.0015) and 6 hours ((p = 0.0152) all at 95% significance level (*i.e.,* p < 0.05).

Figure 3 shows the effect of Composition P, which comprises the substantially non-odorous fragrance modulator Sucrose Myristate and the single fragrance material Dimethylbenzyl carbinol. Addition of the substantially non-odorous fragrance modulator maintains the intensity of the fragrance material from 0 hour up to 6 hours whilst the control, Composition Q, in the absence of the substantially non-odorous fragrance modulator, drops in fragrance profile intensity over the 6 hours. The substantially non-odorous fragrance modulator acts to maintain the continued evaporation over time of the fragrance material. Statistical analysis using the Tukey correction for multiple comparisons confirms the statistically significant difference at 1 hour (p = 0.0001), 3 hours (p = 0.0016) and 6 hours (p= 0.0003) all at 95% significance level (*i.e., p* < 0.05).

Figure 4 shows the effect of Composition T, which comprises the substantially non-odorous fragrance modulator Sucrose Myristate and the single fragrance material Phenethyl alcohol. Addition of the substantially non-odorous fragrance modulator maintains the intensity of the fragrance material from 0 hour up to 6 hours whilst the control, Composition U, in the absence of the substantially non-odorous fragrance modulator, drops in fragrance profile intensity over the 6 hours. The substantially non-odorous fragrance modulator acts to maintain the continued evaporation over time of the fragrance material. Statistical analysis using the Tukey correction for multiple comparisons confirms the statistically significant difference at 1hour (p = 0.0581) at 90% significance level (*i.e.,* p < 0.1), 3hours (p = 0.0311) at 95% significance level (*i.e., p* < 0.05).

Figure 5 shows the effect of Composition PP, which comprises the substantially non-odorous fragrance modulator Propylene Glycol Propyl Ether (PGPE) and the single fragrance material, Eugenol. Addition of the substantially non-odorous fragrance modulator maintains the intensity of the fragrance material from 1 hours up to 6 hours whilst the control, Composition QQ, in the absence of the substantially non-odorous fragrance modulator (PGPE), drops in fragrance profile intensity over the 6 hours. The substantially non-odorous fragrance modulator acts to maintain the continued evaporation over time of the fragrance material. Statistical analysis using the Tukey correction for multiple comparisons confirms the statistically significant difference at 1hour (p < 0.0001) and 6 hours (p = 0.0235) both at 95% significance level (*i.e.,* p < 0.05) and at 3 hours (p = 0.0676) at 90% significance level (i.e., p < 0.1)

Figure 6 shows the effect of Composition RR, which comprises the substantially non-odorous fragrance modulator Diisobutyl Adipate and single fragrance material Dimethyl Benzyl Carbinyl Acetate. Addition of the substantially non-odorous fragrance modulator (Diisobutyl Adipate) maintains the intensity of the fragrance material initially and up to 1 hour whilst the control, Composition SS, in the absence of the substantially non-odorous fragrance modulator, drops in fragrance profile intensity over this time. The substantially non-odorous fragrance modulator acts to maintain the continued initial evaporation over time of the fragrance material. Statistical analysis using the Tukey correction for multiple comparisons confirms the statistically significant difference at 0 hours (p = 0.0558) at 90% significance level (*i.e.,* p < 0.1) and at 1 hour (p = 0.0163) at 95% significance level (*i.e.,* p < 0.05).

Figure 7 shows the effect of Composition AAA, which comprises the substantially non-odorous fragrance modulator Expert Gel56 and the single fragrance material Indocolore. Addition of the substantially non-odorous fragrance modulator (Expert Gel56) maintains the intensity of the fragrance material from 1 hour to at least 6 hours whilst the control, Composition BBB, in the absence of the substantially non-odorous fragrance modulator, drops in fragrance profile intensity much more over this time. The substantially non-odorous fragrance modulator acts to maintain the continued initial evaporation over time of the fragrance material. Statistical analysis using the Tukey correction for multiple comparisons confirms the statistically significant difference at 1, 3 and 6 hours (p < 0.0001) at 95% significance level (*i.e.,* p < 0.05) at all these time points

Figure 8 shows the effect of Composition CCC, which comprises the substantially non-odorous fragrance modulator Kolliphor^{®} EL and the single fragrance material Dimethyl Benzyl Carbinol. Addition of the substantially non-odorous fragrance modulator (Kolliphor^{®} EL) maintains the intensity of the fragrance material from 1 hour to at least 6 hours whilst the control, Composition DDD, in the absence of the substantially non-odorous fragrance modulator, drops in fragrance profile intensity much more over this time. The substantially non-odorous fragrance modulator acts to maintain the reduce the rate of evaporation over time of the fragrance material. Statistical analysis using the Tukey correction for multiple comparisons confirms the statistically significant difference at 1 hour (p < 0.0001), 3 hours (p = 0.0265) and 6 hours (p = 0.0388) at 95% significance level (*i.e.,* p < 0.05) at all these time points.

Figure 9 shows the effect of Composition EEE, which comprises the substantially non-odorous fragrance modulator Kolliphor^{®} EL and the single fragrance material Eugenol. Addition of the substantially non-odorous fragrance modulator (Kolliphor^{®} EL) maintains the intensity of the fragrance material from 1 hour to at least 6 hours whilst the control, Composition FFF, in the absence of the substantially non-odorous fragrance modulator, drops in fragrance profile intensity much more over this time. The substantially non-odorous fragrance modulator acts to supress the initial display of Eugenol and then maintains that continued initial evaporation over time. Statistical analysis using the Tukey correction for multiple comparisons confirms the statistically significant difference at 0 hours (p = 0.0025), 1 hour (p < 0.0001), 3 hours (p < 0.0001) and 6 hours (p < 0.0001) at 95% significance level (*i.e.,* p < 0.05) at all time points,

Figure 10 shows the effect of Composition GGG, which comprises the substantially non-odorous fragrance modulator Kolliphor^{®} EL and the single fragrance material Phenethyl alcohol. Addition of the substantially non-odorous fragrance modulator (Kolliphor^{®} EL) maintains the intensity of the fragrance material from 1 hour to 3 hours whilst the control, Composition HHH, in the absence of the substantially non-odorous fragrance modulator, drops in fragrance profile intensity over this time. The substantially non-odorous fragrance modulator acts to reduce the rate of evaporation over time of the fragrance material. Statistical analysis using the Tukey correction for multiple comparisons confirms the statistically significant difference at 1 hour (p < 0.0001) at 95% significance level (*i.e.,* p < 0.05) and at 3 hours (p = 0.0876) at 90% significance level (*i.e.,* p < 0.1).

Panellists are asked to score the compositions for the intensity of the fragrance on a scale of 0 to 5, wherein 0 represents no fragrance intensity is detected and 5 represents a very strong fragrance intensity is detected. The results of the panel test are then averaged. The results show the effect of the substantially non-odorous fragrance modulator and reduced levels of low volatile fragrance materials for Compositions A, D, G, J and M on fragrance profile longevity and fidelity. Fragrance profile longevity, particularly intensity of the aromas attributable to the volatile fragrance materials are maintained for up to at least 6 hours in the presence of the substantially non-odorous fragrance modulator whilst it drops in the absence of the substantially non-odorous fragrance modulator. Without wishing to be bound by theory, it is believed that the substantially non-odorous fragrance modulator acts to maintain the continued evaporation over time of the fragrance materials, particular the volatile fragrance materials. The effect of the improved fragrance profile longevity of the present invention are noticeable at 1, 3 and 6 hours. Statistical analysis for multiple comparisons confirm the statistically significant difference between the 2 products at various time points.

Panellists are are also asked to score the composition for the fragrance profile fidelity. In particular, the panelists are asked to score the dominance of the floral aromas attributable to the volatile fragrance materials on a scale of 0 to 3 wherein 0 represents not detectable and 3 represents it being the dominant aroma. The results of the panel test are then averaged. The results show that the effect of the substantially non-odorous fragrance modulator and reduced levels of low volatile fragrance materials for Compositions A, D, G, J and M on floral aromas dominance. The floral aromas attributable to the volatile fragrance materials are maintained by the substantially non-odorous fragrance modulator over time for up to 6 hours. Statistical analysis for multiple comparisons can confirm the statistically significant difference between the 2 products at various time points.

Panellists are asked to score the compositions on a scale of 1 to 5, wherein 1 represents the fragrance profile remains unchanged and 5 represents a total change in the fragrance profile. The results of the panel test are averaged and plotted together with the confidence intervals. The results show the effect of the substantially non-odorous fragrance modulator and reduced levels of low volatile fragrance materials for Compositions A, D, G, J and M. The presence of the substantially non-odorous fragrance modulator and the reduced levels of low volatile fragrance materials result in noticeable fidelity in fragrance aromas. Particularly, noticeable fidelity in the floral aroms attributable to the volatile fragrance materials (data not shown). Statistical analysis for multiple comparisons can confirm the statistically significant difference between the 2 products at various time points.

### (a) Effects of the Substantially Non-Odorous Fragrance Modulators on Compositions having High Levels of Low Volatile Fragrance Materials (> 30 wt% relative to the total weight of the fragrance component)

Panellists are asked to score the compositions for the intensity of the fragrance on a scale of 0 to 5, wherein 0 represents a no fragrance intensity is detected and 5 represents a very strong fragrance intensity is detected. The results of the panel test are then averaged. The results show the effect of the substantially non-odorous fragrance modulator and excessive levels of low volatile fragrance materials for Compositions B, E, H, K and N on fragrance profile longevity and fidelity. Two outcomes are observed: (i) either the fragrance profile longevity is unaffected by the addition of the substantially non-odorous fragrance modulator (There are no statistical differences between the 2 products. ) or (ii) the fragrance profile appears to be suppressed with a loss of strength (data not shown). Panellists are also asked to score the Compositions B, E, H, K and N for the dominance of the floral aromas on a scale of 0 to 3 wherein 0 represents not detectable and 3 represents it being the dominant character. The results of the panel test are then averaged. The results show the effect of the substantially non-odorous fragrance modulator and excessive levels of low volatile fragrance materials for Compositions B, E, H, K and N on fidelity of the floral aromas attributable to the volatile fragrance materials. It is observed that the floral aromas is perceived initially but then drops quickly over time. Addition of the substantially non-odorous fragrance modulator does not result in improved fidelity of the floral aromas as seen in Compositions B, E, H, K and N. There are no statistical differences between the 2 products.

## Claims

1. An absorbent article comprising a fragrance composition wherein said fragrance composition comprises:
(i) a fragrance component present in an amount of from 0.04 wt% to 30 wt%, relative to the total weight of the composition, and wherein the fragrance component comprises:
(a) at least one low volatile fragrance material having a vapor pressure < 0.133 Pa (0.001 Torr) at 25 °C, said low volatile fragrance material being present in an amount of from 10 wt% to 30 wt%, relative to the total weight of the fragrance component;
(b) at least one moderate volatile fragrance material having a vapor pressure in the range of 13.33 Pa (0.1 Torr) to 0.133 Pa (0.001 Torr) at 25 °C, said moderate volatile fragrance material being present in an amount of from 40 wt% to 80 wt%, relative to the total weight of the fragrance component; and
(c) at least one high volatile fragrance material having a vapor pressure > 13.33 Pa (0.1 Torr) at 25 °C, said high volatile fragrance material being present in an amount of from 1 wt% to 30 wt%, relative to the total weight of the fragrance component;
(ii) at least one substantially non-odorous fixative (fragrance modulator) present in the amount of from 0.1 wt% to 20 wt%, relative to the total weight of the composition;
(iii)a volatile solvent present in an amount of from 50 wt% to 80 wt%, relative to the total weight of the composition; and
(iv)optionally water;
wherein the vapor pressure is determined by the method for determining the vapor pressue as defined in the test methods section herein.

2. The absorbent article of claim 1, wherein:
(i) the fragrance component is present in an amount from 1 wt% to 30 wt%, preferably less than 25 wt%, more preferably less than 20 wt%, yet even more preferably less than 15 wt%, yet even more preferably less than 10 wt% or yet even more preferably less than 8 wt%, relative to the total weight of the composition; and wherein the fragrance component comprises:
(a) the at least one low volatile fragrance material present in an amount less than 30 wt%, or less than 28 wt%, or less than 25 wt%, relative to the total weight of the fragrance component;
(b) the at least one moderate volatile fragrance material present in an amount of at least 45 wt%, or preferably at least 50 wt%, relative to the total weight of the fragrance component; and
(c) the at least one high volatile fragrance material present in an amount of less than 25 wt%, preferably less than 22 wt% or more preferably less than 20 wt%, relative to the total weight of the fragrance component;
(ii) the at least one substantially non-odorous fixative present in the amount of from 0.5 wt% to 18 wt%, or preferably from 2.5 wt% to 15 wt%, relative to the total weight of the composition;
(iii)the volatile solvent present in an amount of from 55 wt% to 75 wt%, relative to the total weight of the composition; and
(iv)the water present in an amount of from 0 wt% to 20 wt%, relative to the total weight of the composition

3. The absorbent article of any one of the preceding claims, wherein:
(i)(a) the low volatile fragrance material is selected from at least 1 material, or at least 2 materials, or at least 3 materials from the group of Table 1 Low Volatile Fragrance Materials 1-113, and mixtures thereof.

4. The absorbent article of claim 3, wherein:
(i)(a) the low volatile fragrance material is selected from the group of Table 1 Low Volatile Fragrance Materials 1, 4-6, 8, 12-16, 18, 22-25, 27-28, 31, 34-37, 41, 45, 47, 53-56, 58, 61, 62, 64, 66, 69, 70-74, 76, 79, 81, 84-85, 90, 95, 100, 103, 105, 107-109, and mixtures thereof; and
(ii) the substantially non-odorous fixative is selected from the group of Table 4 Substantially Non-Odorous Fragrance Modulators 1-4, and mixtures thereof.

5. The absorbent article of claim 3, wherein:
(i)(a) the low volatile fragrance material is selected from the group of Table 1 Low Volatile Fragrance Materials 1-6, 8-9, 12-14, 16, 18-19, 23, 25-28, 31, 34-35, 37, 41-42, 45, 47-49, 54-56, 58-61, 64, 66, 70, 72-74, 76, 79-80, 82, 85-86, 96, 101, 104, 106, 108, 110, and mixtures thereof; and
(ii) the substantially non-odorous fixative is selected from the group of Table 4 Substantially Non-Odorous Fragrance Modulators 5-7, and mixtures thereof.

6. The absorbent article of any one of the preceding claims, wherein:
(i)(b) the moderate volatile fragrance material is selected from at least 1 material, or at least 2 materials, or at least 3 materials from the group of Table 2 Moderate Volatile Fragrance Materials 1-320, and mixtures thereof.

7. The absorbent article of claim 6, wherein:
(i)(b) the moderate volatile fragrance material is selected from the group of Table 2 Moderate Volatile Fragrance Materials 1-9, 11-12, 14-15, 17-18, 20-25, 27-35, 37-38, 40-44, 46-47, 49-54, 56-62, 64, 66, 68-72, 74-78, 80, 82-85, 87-92, 94-123, 125-126, 131-132, 134-136, 138, 140-146, 148-150, 152, 154-156, 158, 162-163, 165-170, 172-192, 194, 196-199, 201-204, 206-216, 219-220, 222, 224-242, 244, 246-251, 253-256, 258-263, 265-266, 268-269, 272, 274-277, 280-301, 303-305, 307, 309-311, 313-320, and mixtures thereof; and
(ii) the substantially non-odorous fixative is selected from the group of Table 4 Substantially Non-Odorous Fragrance Modulators 1-4, and mixtures thereof.

8. The absorbent article of claim 6, wherein:
(i)(b) the moderate volatile fragrance material is selected from the group of Table 2 Moderate Volatile Fragrance Materials 1-9, 11-12, 14-15, 17-18, 20-25, 27-35, 37-38, 40-44, 46-47, 49-54, 56-62, 64, 66, 68-72, 74-78, 80, 82-85, 87-92, 94-123, 125-126, 131-132, 134-136, 138, 140-146, 148-150, 152, 154-156, 158, 162-163, 165-170, 172-192, 194, 196-199, 201-204, 206-216, 219-220, 222, 224-242, 244, 246-251, 253-256, 258-263, 265-266, 268-269, 272, 274-277, 280-301, 303-305, 307, 309-311, 313-320, and mixtures thereof; and
(ii) the substantially non-odorous fixative is selected from the group of Table 4 Substantially Non-Odorous Fragrance Modulators 5-7, and mixtures thereof.

9. The absorbent article of any one of the preceding claims, wherein:
(i)(c) the high volatile fragrance material is selected from at least 1 material, or at least 2 materials, or at least 3 materials from the group of Table 3 High Volatile Fragrance Materials 1-130, and mixtures thereof.

10. The absorbent article of claim 9, wherein:
(i)(c) the high volatile fragrance material is selected from the group of Table 3 High Volatile Fragrance Materials 1, 2, 6, 8, 9, 12, 14, 19, 36, 39, 46, 47, 56, 57, 58, 60, 62, 74, 78, 93, 94, 96, 100, 106, 111, 117, 119, 120, 128, 129, 131-135, and mixtures thereof; and
(ii) the substantially non-odorous fixative is selected from the group of Table 4 Substantially Non-Odorous Fragrance Modulators 1-4, and mixtures thereof.

11. The absorbent article of claim 9, wherein:
(i)(c) the high volatile fragrance material is selected from the group of Table 3 High Volatile Fragrance Materials 1, 2, 6, 8, 9, 12, 14, 19, 36, 39, 46, 47, 56, 57, 58, 60, 62, 74, 78, 93, 94, 96, 100, 106, 111, 117, 119, 120, 128, 129, 131-135, and mixtures thereof; and
(ii) the substantially non-odorous fixative is selected from the group of Table 4 Substantially Non-Odorous Fragrance Modulators 5-7, and mixtures thereof.

12. The absorbent article of any one of the preceding claims, wherein the substantially non-odorous fixative is selected from the group consisting of:
(a) Methyl Glucoside Polyol; Ethyl Glucoside Polyol; Propyl Glucoside Polyol; and their mixtures;
(b) Isocetyl Alcohol;
(c) PPG-3 Myristyl Ether; Neopentyl Glycol Diethylhexanoate; and their mixtures;
(d) Sucrose Laurate, Sucrose Myristate, Sucrose Palmitate and Sucrose Stearate and their mixtures;
(e) Cycloalkyl Acetal derivatives having the formula (I): 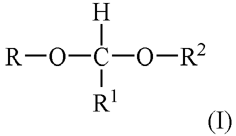 wherein:
R is C₁-C₈ cycloalkyl;
R¹ is hydrogen or methyl; and
R² is methyl, ethyl, propyl, isopropyl, allyl or propargyl;
(f) Trimethylcyclohexane derivatives having the formula (II): 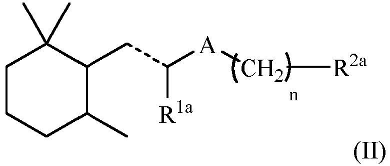 wherein:
n is 0, 1 or 2;
A is C=O or CH-OH;
R^{1a} is hydrogen or methyl;
R^{2a} is a C₂-C₁₀ hydrocarbon group; and
------ is a saturated or unsaturated carbon-carbon bond;
(g) 2-(1-methoxy)ethane-1-ol; 1-(1-menthoxy)propane-2-ol; 3-(1-menthoxy)propane-1-ol; 3-(1-menthoxy)propane-1,2-diol; 2-methyl-3-(1-menthoxy)propane-1,2-diol; 4-(1-menthoxy) butane-1-ol; and their mixtures;
(h) L-menthoxy ether derivatives having the formula (III): 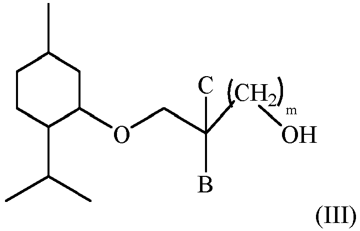 wherein:
m is 0, 1 or 2;
B is hydrogen or OH; and
C is hydrogen or methyl;
(i) Tetra-hydronaphthalene derivatives having the formula (IV): 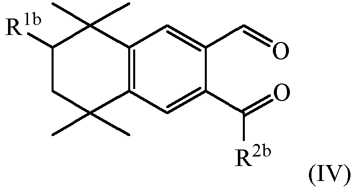 wherein:
R^{1b} is hydrogen or methyl; and
R^{2b} is alkyl;
(j) Hyaluronic acid disaccharide sodium salt, sodium hyaluronate and their mixtures;
(k) neopentyl glycol diisononanoate, cetearyl ethylhexanoate and their mixtures;
(l) *N*-hexadecyl n-nonanoate, *N*-octadecyl n-nonanoate and their mixtures;
(m)Ether derivatives having the formula (V) or formula (VI):
C₅H*ₗ*Oₘ-(OR^{1c})ₙ (V)
wherein:
C₅H*ₗ*Oₘ is a pentose residue, wherein *l* is an integer from 6 to 9, and m is an integer from 1 to 4;
n is an integer from 1 to 4; and
R^{1c} is C₄-C₂₀ hydrocarbon group; and
C₆HₓO_{y}-(O_{R}^{1d})_{z} (VI)
wherein:
C₆HₓO_{y} is a hexose residue, wherein x is an integer from 7 to 11, and y is an integer from 1 to 5;
z is an integer from 1 to 5; and
R^{1d} is C₄-C₂₀ hydrocarbon group; and
(n) Diethylene Glycol Ether derivatives having the formula (VII) or formula (VIII):
C₃H_{c}O_{d}-(OCH₂CH₂-O-CH₂CH₂-O-R^{1e})ₑ (VII)
wherein:
C₅H_{c}O_{d} is a pentose residue, wherein c is an integer from 6 to 8, and d is an integer from 1 to 3;
e is an integer from 2 to 4; and
R^{1e} is C₁-C₆ alkyl group; and
C₆H_{f}O_{g}-(OCH₂CH₂-O-CH₂CH₂-O-R^{1f})ₕ (VIII)
wherein:
C₆H_{f}O_{g} is a hexose residue, wherein f is an integer from 7 to 10, and g is an integer from 1 to 4;
h is an integer from 2 to 5; and
R^{1f} is C₁-C₆ alkyl group;
(o) Hydroquinone Glycoside derivatives having the formula (IX): 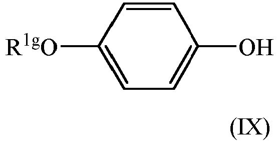 wherein:
R^{1g} is selected from the group consisting of: (i) pentose residue, hexose residue, aminosaccharide residue, uronic acid residue and their mixtures; (ii) methylated versions of group (i); and (iii) mixtures of groups (i) and (ii); and
(p) Propylene Glycol Propyl Ether; Dicetyl Ether; Polyglycerin-4 Ethers; Isoceteth-5; Isoceteth-7, Isoceteth-10; Isocetteth-12; Isoceteth-15; Isoceteth-20; Isoceteth-25; Isoceteth-30; Disodium Lauroamphodipropionate; Hexaethylene glycol monododecyl ether; and their mixtures;
(q) Neopentyl Glycol Diisononanoate; Cetearyl Ethylhexanoate; and their mixtures;
(r) Glyceryl Ether derivatives having the formula (X): 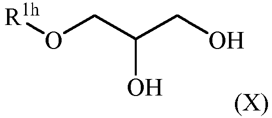 wherein:
R^{1h} is C₄-C₁₂ aliphatic hydrocarbon group;
(s) Panthenol Ethyl Ether, DL-Panthenol and their mixtures;
(t) Aliphatic Dibasic Acid Diester derivatives having the formula (XI):
R¹ⁱOCO^{R2i}COOR³ⁱ (XI)
wherein:
R¹ⁱ is C₄-C₅ alkyl;
R²ⁱ is C₄ alkylene; and
R³ⁱ is C₄-C₅ alkyl; and
(u) Tricyclodecane Amide derivatives selected from the group consisting of:
(i) the compounds of formula (XII): 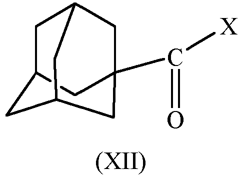 wherein:
X is selected from: 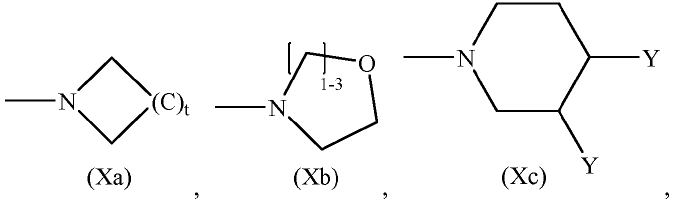 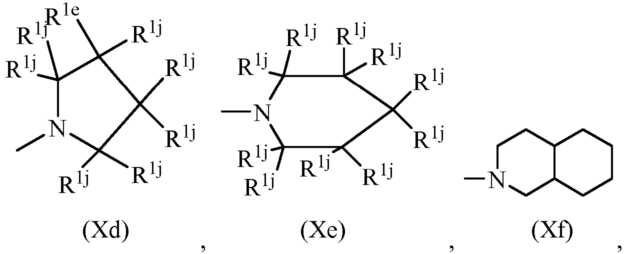 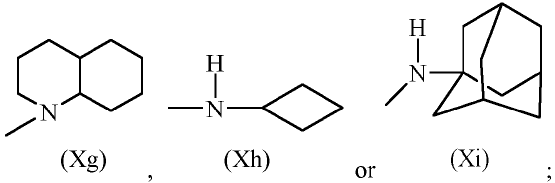
t is 1 to 8;
Y is hydrogen, 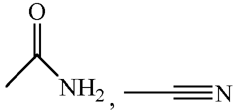 or a halogen; and
each R^{1j} is independently selected from a hydrogen, or C₁-C₄ alkyl;
(ii) the compounds of formula (XIII): 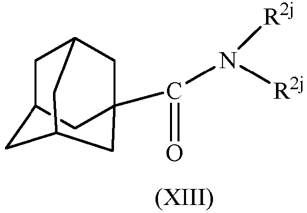 wherein:
each R^{2j} is independently selected from a hydrogen, methyl, ethyl or C₃-C₁₈ alkyl, cycloalkyl or cycloheteroalkyl, with the proviso that both R^{2e} groups are not hydrogen; and
(iii) mixtures of the compounds of formulae (XII) and (XIII);
(v) Aliphatic ether derivatives having the formula (XIV):
R^{1k}-O-(CH(CH₃)-CH₂O)ₚ-(CH₂-CH₂O)_{q} H (XIV)
wherein:
p and q are integers such that the sum of p and q is from 1 to 4; and
R^{1k} is an aliphatic chain comprising from 8 to 18 carbons; and
(w)propyl {4-[2-(diethylamino)-2-oxoethoxy]-3-methoxyphenyl}acetate;
(x) Glycerol alkoxylate derivatives selected from the group consisting of:
(i) the compounds of formula (XV): 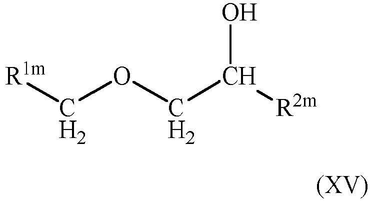 wherein:
R^{1m} is C₆-C₁₂ alkyl; and
R^{2m} is amino or hydroxyl group; and
(ii) the compounds of formula (XVI): 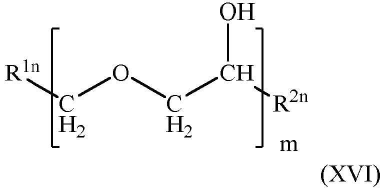 wherein:
m is 1 or 5;
R¹ⁿ is C₆-C₁₂ alkyl;
R²ⁿ is C₁-C₆alkylene-R³ⁿ; and
R³ⁿ is hydroxyl or amino group;
(y) Bis-methoxy PEG-13 PEG-438/PPG-110 SMDI Copolymer; and
(z) mixtures thereof.

13. The absorbent article of any one of the preceding claims, wherein the substantially non-odorous fixative is selected from the group of Table 4 Substantially Non-Odorous Fragrance Modulators 1-97, and mixtures thereof.

14. The absorbent article of any one of the preceding claims, wherein the volatile solvent is a branched or unbranched C₁ to C₁₀ alkanyl, alkenyl or alkynyl having at least one alcohol moiety, preferably ethanol, isopropanol, or glycol.

15. The absorbent article of any one of the preceding claims, wherein the composition maintains the perceived fragrance profile, particularly the aromas attributable to the moderate volatile fragrance material and/or high volatile fragrance material, to remain substantively unchanged from application up to 2 hrs, 3 hrs, 4 hrs, 5 hrs, 6 hrs or 8 hrs after application as determined by Fragrance Intensity according to the Olfactory Test 2(a) as described herein.

16. The absorbent article of any one of the preceding claims, wherein the substantially non-odorous fixativedoes not comprise:
(i) isocetyl alcohol, PPG-3 myristyl ether, neopentyl glycol diethylhexanoate or their mixtures; and
(ii) n-hexadecyl n-nonanoate, n-octadecyl n-nonanoate or their mixtures.

## Patentansprüche

1. Absorptionsartikel, umfassend eine Duftstoffzusammensetzung, wobei die Duftstoffzusammensetzung umfasst:
(i) einen Duftstoffbestandteil, der in einer Menge von 0,04 Gew.-% bis 30 Gew.-%, bezogen auf das Gesamtgewicht der Zusammensetzung, vorhanden ist, und wobei der Duftstoffbestandteil umfasst:
(a) mindestens ein schwerflüchtiges Duftstoffmaterial mit einem Dampfdruck von < 0,133 Pa (0,001 Torr) bei 25 °C, wobei das schwerflüchtige Duftstoffmaterial in einer Menge von 10 Gew.-% bis 30 Gew.-%, bezogen auf das Gesamtgewicht des Duftstoffbestandteils, vorhanden ist;
(b) mindestens ein mäßig flüchtiges Duftstoffmaterial, das einen Dampfdruck in dem Bereich von 13,33 Pa (0,1 Torr) bis 0,133 Pa (0,001 Torr) bei 25 °C aufweist, das in einer Gesamtmenge von mäßig flüchtigem Duftstoffmaterial von etwa 40 Gew.-% bis etwa 80 Gew.-%, bezogen auf das Gesamtgewicht des Duftstoffbestandteils, vorhanden ist; und
(c) mindestens ein hochflüchtiges Duftstoffmaterial mit einem Dampfdruck von > 13,33 Pa (0,1 Torr) bei 25 °C, wobei das hochflüchtige Duftstoffmaterial in einer Menge von 1 Gew.-% bis 30 Gew.-%, bezogen auf das Gesamtgewicht der Duftkomponente, vorhanden ist;
(ii) mindestens ein im Wesentlichen nicht riechendes Fixativ (Duftstoffmodulator), das in der Menge von 0,1 Gew.-% bis 20 Gew.-%, bezogen auf das Gesamtgewicht der Zusammensetzung, vorhanden ist;
(iii) ein flüchtiges Lösungsmittel, das in einer Menge von 50 Gew.-% bis 80 Gew.-%, bezogen auf das Gesamtgewicht der Zusammensetzung, vorhanden ist; und
(iv) wahlweise Wasser;
wobei der Dampfdruck durch das Verfahren zum Bestimmen des Dampfdrucks bestimmt wird, wie es hier im Abschnitt "Prüfverfahren" definiert ist.

2. Absorptionsartikel nach Anspruch 1, wobei:
(i) der Duftstoffbestandteil in einer Menge von 1 Gew.-% bis 30 Gew.-%, vorzugsweise weniger als 25 Gew.-%, mehr bevorzugt weniger als 20 Gew.-%, jedoch noch mehr bevorzugt weniger als 15 Gew.-%, jedoch noch mehr bevorzugt weniger als 10 Gew.-% oder jedoch noch mehr bevorzugt weniger als 8 Gew.-%, bezogen auf das Gesamtgewicht der Zusammensetzung, vorhanden ist; und wobei der Duftstoffbestandteil umfasst:
(a) das mindestens eine schwerflüchtige Duftstoffmaterial, das in einer Menge von weniger als 30 Gew.-% oder weniger als 28 Gew.-% oder weniger als 25 Gew.-%, bezogen auf das Gesamtgewicht des Duftstoffbestandteils, vorhanden ist;
(b) das mindestens eine mäßig flüchtige Duftstoffmaterial, das in einer Menge von mindestens etwa 45 Gew.-% oder vorzugsweise mindestens etwa 50 Gew.-%, bezogen auf das Gesamtgewicht des Duftstoffbestandteils, vorhanden ist; und
(c) das mindestens eine hochflüchtige Duftstoffmaterial, das in einer Menge von weniger als etwa 25 Gew.-%, vorzugsweise weniger als etwa 22 Gew.-% oder mehr bevorzugt weniger als etwa 20 Gew.-%, bezogen auf das Gesamtgewicht des Duftstoffbestandteils, vorhanden ist;
(ii) das mindestens eine im Wesentlichen nicht riechende Fixativ in der Menge von 0,5 Gew.-% bis 18 Gew.-% oder vorzugsweise von 2,5 Gew.-% bis 15 Gew.-%, bezogen auf das Gesamtgewicht der Zusammensetzung, vorhanden ist;
(iii) das flüchtige Lösungsmittel in einer Menge von 55 Gew.-% bis 75 Gew.-%, bezogen auf das Gesamtgewicht der Zusammensetzung, vorhanden ist; und
(iv) das Wasser in einer Menge von 0 Gew.-% bis 20 Gew.-%, bezogen auf das Gesamtgewicht der Zusammensetzung, vorhanden ist.

3. Absorptionsartikel nach einem der vorstehenden Ansprüche, wobei:
(i)(a) das schwerflüchtige Duftstoffmaterial aus mindestens 1 Material oder mindestens 2 Materialien oder mindestens 3 Materialien aus der Gruppe von Tabelle 1 Schwerflüchtige Duftstoffmaterialien 1-113 und Mischungen davon ausgewählt ist.

4. Absorptionsartikel nach Anspruch 3, wobei:
(i)(a) das schwerflüchtige Duftstoffmaterial aus der Gruppe von Tabelle 1 Schwerflüchtige Duftstoffmaterialien 1, 4-6, 8, 12-16, 18, 22-25, 27-28, 31, 34-37, 41, 45, 47, 53-56, 58, 61, 62, 64, 66, 69, 70-74, 76, 79, 81, 84-85, 90, 95, 100, 103, 105, 107-109 und Mischungen davon ausgewählt ist; und
(ii) das im Wesentlichen nicht riechende Fixativ aus der Gruppe der Tabelle 4 Im Wesentlichen nicht riechende Duftstoffmodulatoren 1-4 und Mischungen davon ausgewählt ist.

5. Absorptionsartikel nach Anspruch 3, wobei:
(i)(a) das schwerflüchtige Duftstoffmaterial aus der Gruppe von Tabelle 1 Schwerflüchtige Duftstoffmaterialien 1-6, 8-9, 12-14, 16, 18-19, 23, 25-28, 31, 34-35, 37, 41-42, 45, 47-49, 54-56, 58-61, 64, 66, 70, 72-74, 76, 79-80, 82, 85-86, 96, 101, 104, 106, 108, 110 und Mischungen davon ausgewählt ist; und
(ii) das im Wesentlichen nicht riechende Fixativ aus der Gruppe von Tabelle 4 Im Wesentlichen nicht riechende Duftstoffmodulatoren 5-7 und Mischungen davon ausgewählt ist.

6. Absorptionsartikel nach einem der vorstehenden Ansprüche, wobei:
(i)(b) das mäßig flüchtige Duftstoffmaterial aus mindestens 1 Material oder mindestens 2 Materialien oder mindestens 3 Materialien aus der Gruppe von Tabelle 2 Mäßig flüchtige Duftstoffmaterialien 1-320 und Mischungen davon ausgewählt ist.

7. Absorptionsartikel nach Anspruch 6, wobei:
(i)(b) das mäßig flüchtige Duftstoffmaterial aus der Gruppe von Tabelle 2 Mäßig flüchtige Duftstoffmaterialien 1-9, 11-12, 14-15, 17-18, 20-25, 27-35, 37-38, 40-44, 46-47, 49-54, 56-62, 64, 66, 68-72, 74-78, 80, 82-85, 87-92, 94-123, 125-126, 131-132, 134-136, 138, 140-146, 148-150, 152, 154-156, 158, 162-163, 165-170, 172-192, 194, 196-199, 201-204, 206-216, 219-220, 222, 224-242, 244, 246-251, 253-256, 258-263, 265-266, 268-269, 272, 274-277, 280-301, 303-305, 307, 309-311, 313-320 und Mischungen davon ausgewählt ist; und
(ii) das im Wesentlichen nicht riechende Fixativ aus der Gruppe der Tabelle 4 Im Wesentlichen nicht riechende Duftstoffmodulatoren 1-4 und Mischungen davon ausgewählt ist.

8. Absorptionsartikel nach Anspruch 6, wobei:
(i)(b) das mäßig flüchtige Duftstoffmaterial aus der Gruppe von Tabelle 2 Mäßig flüchtige Duftstoffmaterialien 1-9, 11-12, 14-15, 17-18, 20-25, 27-35, 37-38, 40-44, 46-47, 49-54, 56-62, 64, 66, 68-72, 74-78, 80, 82-85, 87-92, 94-123, 125-126, 131-132, 134-136, 138, 140-146, 148-150, 152, 154-156, 158, 162-163, 165-170, 172-192, 194, 196-199, 201-204, 206-216, 219-220, 222, 224-242, 244, 246-251, 253-256, 258-263, 265-266, 268-269, 272, 274-277, 280-301, 303-305, 307, 309-311, 313-320 und Mischungen davon ausgewählt ist; und
(ii) das im Wesentlichen nicht riechende Fixativ aus der Gruppe von Tabelle 4 Im Wesentlichen nicht riechende Duftstoffmodulatoren 5-7 und Mischungen davon ausgewählt ist.

9. Absorptionsartikel nach einem der vorstehenden Ansprüche, wobei:
(i)(c) das hochflüchtige Duftstoffmaterial aus mindestens 1 Material oder mindestens 2 Materialien oder mindestens 3 Materialien aus der Gruppe von Tabelle 3 Hoch flüchtige Duftstoffmaterialien 1-130 und Mischungen davon ausgewählt ist.

10. Absorptionsartikel nach Anspruch 9, wobei:
(i)(c) das hoch flüchtige Duftstoffmaterial aus der Gruppe von Tabelle 3 Hoch flüchtige Duftstoffmaterialien 1, 2, 6, 8, 9, 12, 14, 19, 36, 39, 46, 47, 56, 57, 58, 60, 62, 74, 78, 93, 94, 96, 100, 106, 111, 117, 119, 120, 128, 129, 131-135 und Mischungen davon ausgewählt ist; und
(ii) das im Wesentlichen nicht riechende Fixativ aus der Gruppe der Tabelle 4 Im Wesentlichen nicht riechende Duftstoffmodulatoren 1-4 und Mischungen davon ausgewählt ist.

11. Absorptionsartikel nach Anspruch 9, wobei:
(i)(c) das hoch flüchtige Duftstoffmaterial aus der Gruppe von Tabelle 3 Hoch flüchtige Duftstoffmaterialien 1, 2, 6, 8, 9, 12, 14, 19, 36, 39, 46, 47, 56, 57, 58, 60, 62, 74, 78, 93, 94, 96, 100, 106, 111, 117, 119, 120, 128, 129, 131-135 und Mischungen davon ausgewählt ist; und
(ii) das im Wesentlichen nicht riechende Fixativ aus der Gruppe von Tabelle 4 Im Wesentlichen nicht riechende Duftstoffmodulatoren 5-7 und Mischungen davon ausgewählt ist.

12. Absorptionsartikel nach einem der vorstehenden Ansprüche, wobei das im Wesentlichen nicht riechende Fixativ ausgewählt ist aus der Gruppe, bestehend aus:
(a) Methylglucosidpolyol; Ethylglucosidpolyol; Propylglucosidpolyol; und ihre Mischungen;
(b) Isocetyl-Alkohol;
(c) PPG-3-Myristylether; Neopentylglykoldiethylhexanoat; und ihre Mischungen;
(d) Saccharoselaurat, Saccharosemyristat, Saccharosepalmitat und Saccharosestearat sowie deren Mischungen;
(e) Cycloalkylacetalderivative, die die Formel (I) aufweisen: 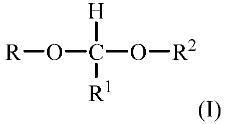 wobei:
R C₁-C₈-Cycloalkyl ist;
R¹ Wasserstoff oder Methyl ist; und
R² Methyl, Ethyl, Propyl, Isopropyl, Allyl oder Propargyl ist;
(f) Trimethylcyclohexanderivative, die die Formel (II) aufweisen: 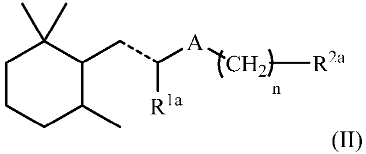 wobei:
n 0, 1 oder 2 ist;
A C=O oder CH-OH ist;
R^{1a} Wasserstoff oder Methyl ist;
R^{2a} eine C₂-C₁₀-Kohlenwasserstoffgruppe ist; und
- - - - - - eine gesättigte oder ungesättigte Kohlenstoff-Kohlenstoff-Bindung ist;
(g) 2-(1-Methoxy)ethan-1-ol; 1-(1-Menthoxy)propan-2-ol; 3-(1-Menthoxy)propan-1-ol; 3-(1-Menthoxy)propan-1,2-diol; 2-Methyl-3-(1-menthoxy)propan-1,2-diol; 4-(1-Menthoxy)butan-1-ol; und ihre Mischungen;
(h) L-Menthoxyetherderivative, die die Formel (III) aufweisen: 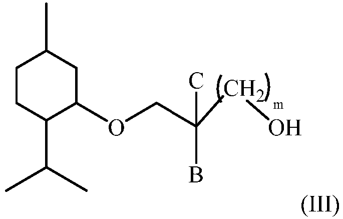 wobei:
m 0, 1 oder 2 ist;
B Wasserstoff oder OH ist; und
C Wasserstoff oder Methyl ist;
(i) Tetrahydronaphthalenderivative, die die Formel (IV) aufweisen: 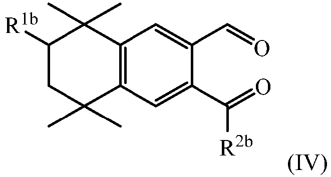 wobei:
R^{1b} Wasserstoff oder Methyl ist; und
R^{2b} Alkyl ist;
(j) Hyaluronsäuredisaccharidnatriumsalz, Natriumhyaluronat und ihre Mischungen;
(k) Neopentylglykoldiisononanoat, Cetearylethylhexanoat und ihre Mischungen;
(l) *N*-Hexadecyl-n-nonanoat, *N*-Octadecyl-n-nonanoat oder ihre Mischungen;
(m) Etherderivative, die die Formel (V) oder Formel (VI) aufweisen:
C₃H*ₗ*Oₘ-(OR^{1c})ₙ (V)
wobei:
C₅H*ₗ*Dₘ ein Pentoserückstand ist, wobei *l* eine ganze Zahl von 6 bis 9 ist, und m eine ganze Zahl von 1 bis 4 ist;
n eine ganze Zahl von 1 bis 4 ist; und
R^{1c} eine C₄-C₂₀-Kohlenwasserstoffgruppe ist; und
C₆HₓO_{y}-(O^{1d})_{z} (VI)
wobei:
C₆HₓO_{y} ein Hexoserückstand ist, wobei x eine ganze Zahl von 7 bis 11 ist, und y eine ganze Zahl von 1 bis 5 ist;
z eine ganze Zahl von 1 bis 5 ist; und
R^{1d} eine C₄-C₂₀-Kohlenwasserstoffgruppe ist; und
(n) Diethylenglykoletherderivative, die die Formel (VII) oder Formel (VIII) aufweisen:
C₅H_{c}O_{d}-(OCH₂CH₂-O-CH₂CH₂-O-R^{1e})ₑ (VII)
wobei:
C₅H_{c}O_{d} ein Pentoserückstand ist, wobei c eine ganze Zahl von 6 bis 8 ist, und d eine ganze Zahl von 1 bis 3 ist;
e eine ganze Zahl von 2 bis 4 ist; und
R^{1e} eine C₁-C₆-Alkylgruppe ist; und
C₆H_{f}O_{g}-(OCH₂CH₂-O-CH₂CH₂-O-R^{1f})ₕ (VIII)
wobei:
C₆H_{f}O_{g} ein Hexoserückstand ist, wobei f eine ganze Zahl von 7 bis 10 ist, und g eine ganze Zahl von 1 bis 4 ist;
h eine ganze Zahl von 2 bis 5 ist; und
R^{1f} eine C₁-C₆-Alkylgruppe ist;
(o) Hydroxychinonglykosidderivative, die die Formel (IX) aufweisen: 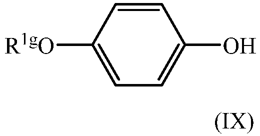 wobei:
R¹⁹ aus der Gruppe ausgewählt ist, bestehend aus: (i) Pentoserückstand, Hexoserückstand, Aminsaccharidrückstand, Uronsäurerückstand und ihren Mischungen; (ii) methylierten Versionen von Gruppe (i); und (iii) Mischungen von Gruppen (i) und (ii); und
(p) Propylenglycolpropylether; Dicetylether; Polyglycerin-4-Ethern; Isoceteth-5; Isoceteth-7, Isoceteth-10; Isocetteth-12; Isoceteth-15; Isoceteth-20; Isoceteth-25; Isoceteth-30; Dinatriumlauroamphodipropionat; Hexaethylenglycolmonododecylether; und ihre Mischungen;
(q) Neopentylglycoldiisononanoat; Cetearylethylhexanoat; und ihre Mischungen;
(r) Glyceryletherderivative, die die Formel (X) aufweisen: 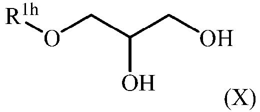 wobei:
R^{1h} eine aliphatische C₄-C₁₂-Kohlenwasserstoffgruppe ist;
(s) Panthenolethylether, DL-Panthenol und ihre Mischungen;
(t) aliphatische zweibasige Säurediesterderivative, die die Formel (XI) aufweisen:
R¹ⁱOCO^{R2i}COOR³ⁱ (XI)
wobei:
R¹ⁱ ein C₄-C₅-Alkyl ist;
R²ⁱ ein C₄-Alkylen ist; und
R³ⁱ ein C₄-C₅-Alkyl ist; und
(u) Tricyclodecanamidderivative, die aus der Gruppe ausgewählt sind, bestehend aus:
(i) den Verbindungen von Formel (XII): 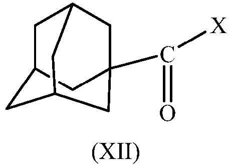 wobei:
X ausgewählt ist aus: 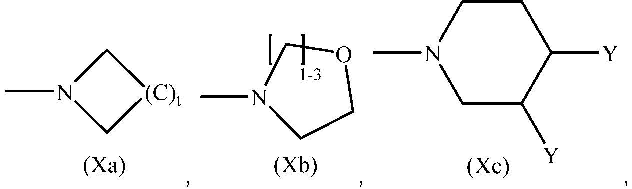 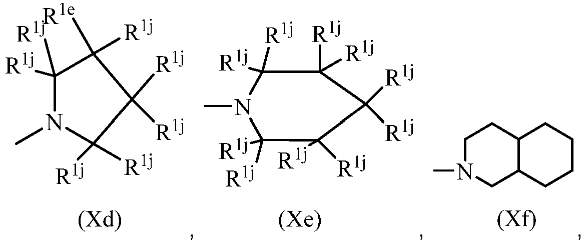 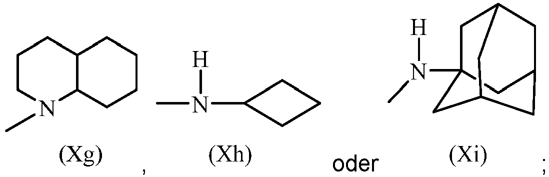
t 1 bis 8 ist;
Y Wasserstoff, 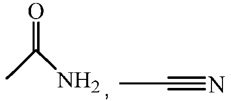 oder ein Halogen ist; und
jedes R¹ⁱ unabhängig aus einem Wasserstoff oder C₁-C₄-Alkyl ausgewählt ist;
(ii) den Verbindungen von Formel (XIII): 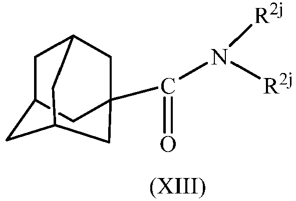 wobei:
jedes R^{2j} unabhängig aus einem Wasserstoff, Methyl, Ethyl oder C₃-C₁₈-Alkyl, Cycloalkyl oder Cycloheteroalkyl ausgewählt ist, vorausgesetzt, beide R^{2e}. Gruppen sind nicht Wasserstoff; und
(iii) Mischungen der Verbindungen von Formeln (XII) und (XIII);
(v) aliphatische Etherderivative, die die Formel (XIV) aufweisen:
R^{1k}-O-(CH(CH₃)-CH₂O)ₚ-(CH₂-CH₂O)_{q}-H (XIV)
wobei:
p und q ganze Zahlen sind, sodass die Summe von p und q von 1 bis 4 ist; und
R^{1k} eine aliphatische Kette ist, umfassend von 8 bis 18 Kohlenstoffe; und
(w) Propyl-{4-[2-(Diethylamino)-2-oxoethoxy]-3-methoxyphenyl}acetat;
(x) Glycerolalkoxylatderivative, die aus der Gruppe ausgewählt sind, bestehend aus:
(i) den Verbindungen von Formel (XV): 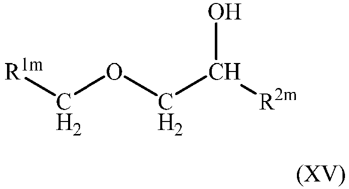 wobei:
R^{1m} C₆-C₁₂-Alkyl ist; und
R^{2m} eine Amino- oder Hydroxygruppe ist; und
(ii) den Verbindungen von Formel (XVII): 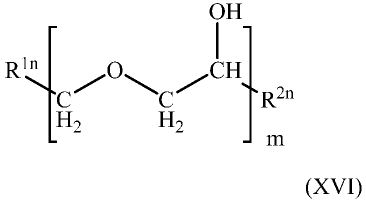 wobei:
m 1 oder 5 ist;
R¹ⁿ ein C₆-C₁₂-Alkyl ist;
R²ⁿ ein C₁-C₆-Alkylen-R³ⁿ ist; und
R³ⁿ eine Hydroxy- oder Aminogruppe ist;
(y) Bis-Methoxy-PEG-13-PEG-438/PPG-110-SMDI-Copolymer; und
(z) Mischungen davon.

13. Absorptionsartikel nach einem der vorstehenden Ansprüche, wobei das im Wesentlichen nicht riechende Fixativ aus der Gruppe von Tabelle 4 Im Wesentlichen nicht riechende Duftstoffmodulatoren 1-97 und Mischungen davon ausgewählt ist.

14. Absorptionsartikel nach einem der vorstehenden Ansprüche, wobei das flüchtige Lösungsmittel ein verzweigtes oder unverzweigtes C₁-C₁₀-Alkanyl-, Alkenyl- oder Alkinyl ist, die mindestens eine Alkoholeinheit, vorzugsweise Ethanol, Isopropanol oder Glycol aufweist.

15. Absorptionsartikel nach einem der vorstehenden Ansprüche, wobei die Zusammensetzung das wahrgenommene Duftstoffprofil beibehält, insbesondere die Aromen, die dem mäßig flüchtigen Duftstoff und/oder dem hochflüchtigen Duftstoff zuzuschreiben sind, sodass sie von der Anwendung bis zu 2 Stunden, 3 Stunden, 4 Stunden, 5 Stunden, 6 Stunden oder 8 Stunden nach der Anwendung im Wesentlichen unverändert bleiben, wie durch die Duftstoffintensität gemäß dem hierin beschriebenen Geruchstest 2(a) bestimmt.

16. Absorptionsartikel nach einem der vorstehenden Ansprüche, wobei der im Wesentlichen nicht riechende Duftstoffmodulator nicht umfasst:
(i) Isocetylalkohol, PPG-3-Myristylether, Neopentylglycoldiethylhexanoat oder deren Mischungen; und
(ii) n-Hexadecyl-n-nonanoat, n-Octadecyl-n-nonanoat oder deren Mischungen.

## Revendications

1. Article absorbant comprenant une composition parfumée, dans lequel ladite composition parfumée comprend :
(i) un constituant de parfum présent en une quantité de 0,04 % en poids à 30 % en poids, par rapport au poids total de la composition, dans lequel le constituant de parfum comprend :
(a) au moins un matériau de parfum peu volatil ayant une pression de vapeur < 0,133 Pa (0,001 Torr) à 25 °C, ledit matériau de parfum peu volatil étant présent en une quantité de 10 % en poids à 30 % en poids, par rapport au poids total du constituant de parfum ;
(b) au moins un matériau de parfum modérément volatil ayant une pression de vapeur dans la plage de 13,33 Pa (0,1 Torr) à 0,133 Pa (0,001 Torr) à 25 °C, ledit matériau de parfum modérément volatil étant présent en une quantité d'environ 40 % en poids à environ 80 % en poids, par rapport au poids total du constituant de parfum ; et
(c) au moins un matériau de parfum très volatil ayant une pression de vapeur > 13,33 Pa (0,1 Torr) à 25 °C, ledit matériau de parfum très volatil étant présent en une quantité de 1 % en poids à 30 % en poids par rapport au poids total du constituant de parfum ;
(ii) au moins un fixateur sensiblement non odorant (modulateur de parfum) présent en une quantité de 0,1 % en poids à 20 % en poids, par rapport au poids total de la composition ;
(iii) un solvant volatil présent en une quantité de 50 % en poids à 80 % en poids, par rapport au poids total de la composition ; et
(iv) éventuellement de l'eau ;
dans lequel la pression de vapeur est déterminée par le procédé de détermination de la pression de vapeur telle que définie dans la section des procédés d'essai du présent document.

2. Article absorbant selon la revendication 1, dans lequel :
(i) le constituant de parfum est présent en une quantité de 1 % en poids à 30 % en poids, de préférence inférieure à 25 % en poids, plus préférablement inférieure à 20 % en poids, encore plus préférablement inférieure à 15 % en poids, encore plus préférablement inférieure à 10 % en poids ou encore plus préférablement inférieure à 8 % en poids, par rapport au poids total de la composition ; et dans lequel le constituant de parfum comprend :
(a) l'au moins un matériau de parfum faiblement volatil présent en une quantité inférieure à 30 % en poids, ou inférieure à 28 % en poids, ou inférieure à 25 % en poids, par rapport au poids total du constituant de parfum ;
(b) l'au moins un matériau de parfum modérément volatil présent en une quantité d'au moins 45 % en poids, ou de préférence d'au moins 50 % en poids, par rapport au poids total du constituant de parfum ; et
(c) l'au moins un matériau de parfum fortement volatil présent en une quantité inférieure à 25 % en poids, de préférence inférieure à 22 % en poids ou plus préférablement inférieure à 20 % en poids, par rapport au poids total du constituant de parfum ;
(ii) l'au moins un fixateur de parfum sensiblement non odorant présent en une quantité de 0,5 % en poids à 18 % en poids, ou de préférence de 2,5 % en poids à 15 % en poids, par rapport au poids total de la composition ;
(iii) le solvant volatil présent en une quantité de 55 % en poids à 75 % en poids par rapport au poids total de la composition ; et
(iv) de l'eau présente en une quantité de 0 % en poids à 20 % en poids, par rapport au poids total de la composition.

3. Article absorbant selon l'une quelconque des revendications précédentes, dans lequel :
(i)(a) le matériau de parfum faiblement volatil est choisi parmi au moins 1 matériau, ou au moins 2 matériaux, ou au moins 3 matériaux du groupe des matériaux de parfum faiblement volatils 1 à 113 du tableau 1, et des mélanges de ceux-ci.

4. Article absorbant selon la revendication 3, dans lequel :
(i)(a) le matériau de parfum faiblement volatil est choisi dans le groupe des matériaux de parfum faiblement
volatils 1, 4 à 6, 8, 12 à 16, 18, 22 à 25, 27 à 28, 31, 34 à 37, 41, 45, 47, 53 à 56, 58, 61, 62, 64, 66, 69, 70 à 74, 76, 79, 81, 84 à 85, 90, 95, 100, 103, 105, 107 à 1 09 du tableau 1, et des mélanges de ceux-ci ; et
(ii) le fixateur substantiellement non odorant est choisi dans le groupe des modulateurs de parfum substantiellement non odorants 1 à 4 du tableau 4, et des mélanges de ceux-ci.

5. Article absorbant selon la revendication 3, dans lequel :
(i)(a) le matériau de parfum faiblement volatil est choisi dans le groupe des matériaux de parfum faiblement
volatils 1 à 6, 8 à 9, 12 à 14, 16, 18 à 19, 23, 25 à 28, 31, 34 à 35, 37, 41 à 42, 45, 47 à 49, 54 à 56, 58 à 61, 64, 66, 70, 72 à 74, 76, 79 à 80, 82, 85 à 86, 96, 101, 1 04, 106, 108, 110 du tableau 1, et des mélanges de ceux-ci ; et
(ii) le fixateur substantiellement non odorant est choisi dans le groupe des modulateurs de parfum substantiellement non odorants 5 à 7 du tableau 4, et des mélanges de ceux-ci.

6. Article absorbant selon l'une quelconque des revendications précédentes, dans lequel :
(i)(b) le matériau de parfum modérément volatil est choisi parmi au moins 1 matériau, ou au moins 2 matériaux, ou au moins 3 matériaux du groupe des matériaux de parfum modérément volatils 1 à 320 du tableau 2, et des mélanges de ceux-ci.

7. Article absorbant selon la revendication 6, dans lequel :
(i)(b) le matériau de parfum modérément volatil est choisi dans le groupe des matériaux de parfum modérément
volatils 1 à 9, 11 à 12, 14 à 15, 17 à 18, 20 à 25, 27 à 35, 37 à 38, 40 à 44, 46 à 4 7, 49 à 54, 56 à 62, 64, 66, 68 à 72, 74 à 78, 80, 82 à 85, 87 à 92, 94 à 123, 125 à 126, 131 à 132, 134 à 136, 138, 140 à 146, 148 à 150, 152, 154 à 156, 158, 162 à 163, 165 à 170, 172 à 192, 194, 196 à 199, 201 à 204, 206 à 216, 219 à 220, 22 2, 224 à 242, 244, 246 à 251, 253 à 256, 258 à 263, 265 à 266, 268 à 269, 272, 2 74 à 277, 280 à 301, 303 à 305, 307, 309 à 311, 313 à 320 du tableau 2, et des mélanges de ceux-ci ; et
(ii) le fixateur substantiellement non odorant est choisi dans le groupe des modulateurs de parfum substantiellement non odorants 1 à 4 du tableau 4, et des mélanges de ceux-ci.

8. Article absorbant selon la revendication 6, dans lequel :
(i)(b) le matériau de parfum modérément volatil est choisi dans le groupe des matériaux de parfum modérément
volatils 1 à 9, 11 à 12, 14 à 15, 17 à 18, 20 à 25, 27 à 35, 37 à 38, 40 à 44, 46 à 4 7, 49 à 54, 56 à 62, 64, 66, 68 à 72, 74 à 78, 80, 82 à 85, 87 à 92, 94 à 123, 125 à 126, 131 à 132, 134 à 136, 138, 140 à 146, 148 à 150, 152, 154 à 156, 158, 162 à 163, 165 à 170, 172 à 192, 194, 196 à 199, 201 à 204, 206 à 216, 219 à 220, 22 2, 224 à 242, 244, 246 à 251, 253 à 256, 258 à 263, 265 à 266, 268 à 269, 272, 2 74 à 277, 280 à 301, 303 à 305, 307, 309 à 311, 313 à 320 du tableau 2, et des mélanges de ceux-ci ; et
(ii) le fixateur substantiellement non odorant est choisi dans le groupe des modulateurs de parfum substantiellement non odorants 5 à 7 du tableau 4, et des mélanges de ceux-ci.

9. Article absorbant selon l'une quelconque des revendications précédentes, dans lequel :
(i)(c) le matériau de parfum fortement volatil est choisi parmi au moins 1 matériau, ou au moins 2 matériaux, ou au moins 3 matériaux du groupe des matériaux de parfum hautement volatils 1 à 130 du tableau 3, et des mélanges de ceux-ci.

10. Article absorbant selon la revendication 9, dans lequel :
(i)(c) le matériau de parfum fortement volatil est choisi dans le groupe des matériaux de parfum fortement
volatils 1, 2, 6, 8, 9, 12, 14, 19, 36, 39, 46, 47, 56, 57, 58, 60, 62, 74, 78, 93, 94, 9 6, 100, 106, 111, 117, 119, 120, 128, 129, 131 à 135 du tableau 3, et des mélanges de ceux-ci ; et
(ii) le fixateur substantiellement non odorant est choisi dans le groupe des modulateurs de parfum substantiellement non odorants 1 à 4 du tableau 4, et des mélanges de ceux-ci.

11. Article absorbant selon la revendication 9, dans lequel :
(i)(c) le matériau de parfum fortement volatil est choisi dans le groupe des matériaux de parfum fortement
volatils 1, 2, 6, 8, 9, 12, 14, 19, 36, 39, 46, 47, 56, 57, 58, 60, 62, 74, 78, 93, 94, 9 6, 100, 106, 111, 117, 119, 120, 128, 129, 131 à 135 du tableau 3, et des mélanges de ceux-ci ; et
(ii) le fixateur substantiellement non odorant est choisi dans le groupe des modulateurs de parfum substantiellement non odorants 5 à 7 du tableau 4, et des mélanges de ceux-ci.

12. Article absorbant selon l'une quelconque des revendications précédentes, dans lequel le fixateur substantiellement non odorant est choisi dans le groupe constitué de :
(a) Polyol de méthylglucoside ; Éthyle de méthylglucoside ; Propyl de méthylglucoside ; et des mélanges de ceux-ci ;
(b) Alcool isocétylique ;
(c) PPG-3 myristyléther , Diéthylhexanoate de néopentylglycol ; et des mélanges de ceux-ci ;
(d) le laurate de saccharose, le myristate de saccharose, le palmitate de saccharose et le stéarate de saccharose et des mélanges de ceux-ci ;
(e) dérivés d'acétal cycloalkylique ayant la formule (I) : 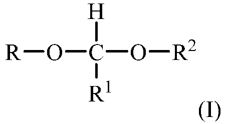 dans lequel :
R est un cycloalkyle en C₁ à C₈ ;
R¹ est hydrogène ou méthyle ; et
R² est un méthyle, un éthyle, un propyle, un isopropyle, un allyle ou un propargyle ;
(f) dérivés de triméthylcyclohexyle ayant la formule (II) : 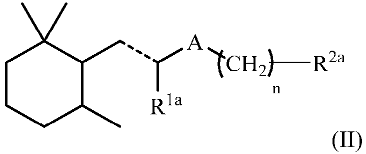 dans lequel :
n vaut 0, 1 ou 2 ;
A est C=O ou CH-OH ;
R^{1a} est de l'hydrogène ou du méthyle ;
R^{2a} est un groupe hydrocarboné en C₂ à C₁₀ ; et
- - - - - - est une liaison carbone-carbone saturée ou insaturée ;
(g) 2-(1-méthoxy)éthane-1-ol ; 1-(1-menthoxy)propane-2-ol ; 3-(1-menthoxy)propane-1-ol ; 3-(1-menthoxy)propane-1,2-diol ; 2-méthyl-3-(1-menthoxy)propane-1,2-diol ; 4-(1-menthoxy) butane-1-ol ; et des mélanges de ceux-ci ;
(h) dérivés d'éther L-menthoxylique ayant la formule (III) : 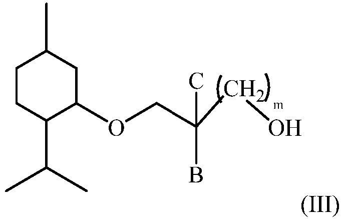 dans lequel :
m vaut 0, 1 ou 2 ;
B est de l'hydrogène ou de l'OH ; et
C est de l'hydrogène ou du méthyle ;
(i) dérivés de tétra-hydronaphtalène ayant la formule (IV) : 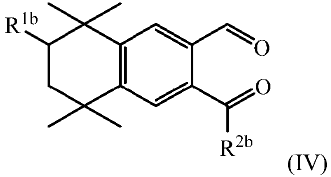 dans lequel :
R^{1b} est de l'hydrogène ou du méthyle ; et
R^{2b} est de l'alkyle ;
(j) sel de sodium disaccharide d'acide hyaluronique, hyaluronate de sodium et des mélanges de ceux-ci ;
(k) le diisononanoate de néopentyle glycol, l'éthylhexanoate de cétéaryle et des mélanges de ceux-ci ;
(l) n-nonanoate de *N*-hexadécyle, n-nonanoate de N-octadécyle et des mélanges de ceux-ci ;
(m) dérivés d'éther ayant la formule (V) et la formule (VI) :
C₅H*ₗ*Oₘ-(OR^{1c})ₙ (V)
dans lequel :
C₅H*ₗ*Oₘest un résidu de pentose, dans lequel*l* est un entier de 6 à 9, et m est un entier de 1 à 4 ;
n est un nombre entier allant de 1 à 4 ; et
R^{1c} est un groupe hydrocarboné en C₄ à C₂₀ ; et
C₆HₓO_{y}-(OR^{1d})_{z} (VI)
dans lequel :
C₆HₓO_{y} est un résidu d'hexose, dans lequel x est un nombre entier allant de 7 à 11, et y est un nombre entier allant de 1 à 5 ;
z est un nombre entier allant de 1 à 5 ; et
R^{1d} est un groupe hydrocarboné en C₄ à C₂₀ ; et
(n) des dérivés d'éther de diéthylène glycol ayant la formule (VII) ou la formule (VIII) :
C₃H_{c}O_{d}-(OCH₂CH₂-O-CH₂CH₂-O-R^{1e})ₑ (VII)
dans lequel :
C₅H_{c}O_{d} est un résidu de pentose, dans lequel c est un nombre entier allant de 6 à 8, et d est un nombre entier allant de 1 à 3 ;
n est un nombre entier de 2 à 4 ; et
R^{1e} est un groupe alkyle en C₁ à C₆ ; et
C₆H_{f}O_{g}-(OCH₂CH₂-O-CH₂CH₂-O-R^{1f})ₕ (VIII)
dans lequel :
C₆H_{f}O_{g} est un résidu de hexose, dans lequel f est un nombre entier allant de 7 à 10, et g est un nombre entier allant de 1 à 4 ;
h est un nombre entier allant de 2 à 5 ; et
R^{1f} est un groupe alkyle en C₁ à C₆ ;
(o) des dérivés de glycoside d'hydroquinone ayant la formule (IX) : 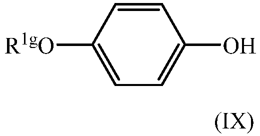 dans lequel :
R^{1g} est choisi dans le groupe constitué : (i) d'un résidu de pentose, un résidu d'hexose, un résidu d'aminosaccharide, un résidu d'acide uronique et leurs mélanges ; (ii) des versions méthylées du groupe (i) ; et (iii) des mélanges des groupes (i) et (ii) ; et
(p) éther propylique de propylène glycol ; éther dicétylique ; éthers de polyglycérine-4 ; Isoceteth-5 ; Isoceteth-7, Isoceteth-10 ; Isocetteth-12 ; Isoceteth-15 ; Isoceteth-20 ; Isoceteth-25 ; Isoceteth-30 ; Lauroamphodipropionate de disodium ; Ether monododécylique d'hexaéthylène glycol ; et des mélanges de ceux-ci ;
(q) Diisononanoate de néopentyle glycol ; Ethylhexanoate de cétéaryle ; et des mélanges de ceux-ci ;
(r) dérivés d'éther de glycéryle ayant la formule (X) : 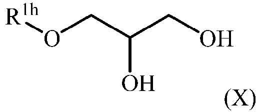 dans lequel :
R^{1h} est un groupe hydrocarboné aliphatique en C₄ à C₁₂ ;
(s) éther éthylique de panthényle, DL-panthénol et des mélanges de ceux-ci ;
(t) dérivés de diester d'acide dibasique aliphatique ayant la formule (XI) :
R¹ⁱOCO^{R2i}COOR³ⁱ (XI)
dans lequel :
R¹ⁱ est un alkyle en C₄ à C₅ ;
R²ⁱ est un alkylène en C₄ ; et
R³ⁱ est un alkyle en C₄ à C₅ ; et
(u) dérivés d'amide de tricyclodécane choisis dans le groupe constitué :
(i) les composés de formule (XII) : 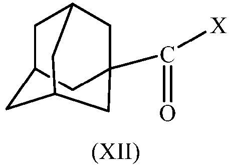 dans lequel :
X est choisi parmi : 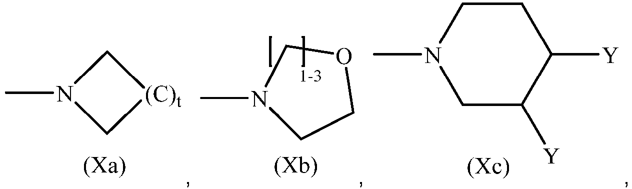 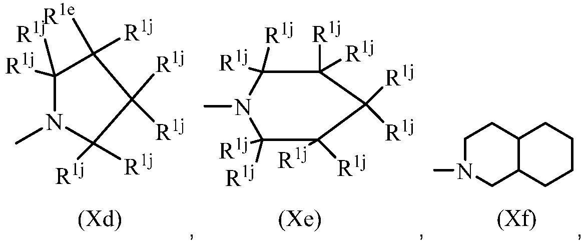 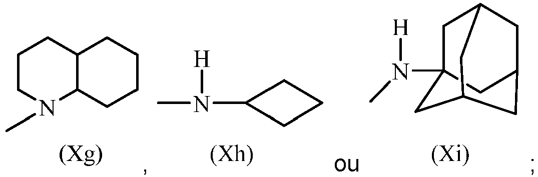
t vaut 1 ou 8 ;
Y est de l'hydrogène, 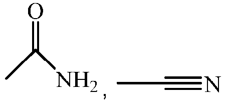 ou un halogène ; et
chaque R¹ⁱ est indépendamment choisi parmi un hydrogène, ou alkyle en C₁ à C₄ ;
(ii) les composés de formule (XIII) : 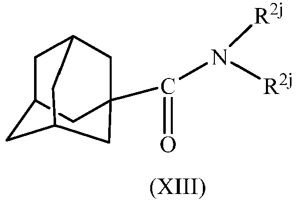 dans lequel :
chaque R^{2j} est indépendamment choisi parmi un hydrogène, méthyle, éthyle ou alkyle en C₃ à C₁₈, cycloalkyle ou cyclohétéroalkyle, à condition que les deux groupes R^{2e} ne soient pas de l'hydrogène ; et
(iii) des mélanges des composés de formules (XII) et (XIII) ;
(v) dérivés d'éther aliphatique ayant la formule (XIV) :
R^{1k}-O-(CH(CH₃)-CH₂O)ₚ-(CH₂-CH₂-CH₂O)_{q}-H (XIV)
dans lequel :
p et q sont des nombres entiers de telle sorte que la somme de p et q de 1 à 4 ; et
R^{1k} est une chaîne aliphatique comprenant de 8 à 18 carbones ; et
(w) acétate de propyle {4-[2-(diéthylamino)-2-oxoéthoxy]-3-méthoxyphényl} ;
(x) dérivés d'amide de tricyclodécane choisis dans le groupe constitué :
(i) des composés de formule (XV) : 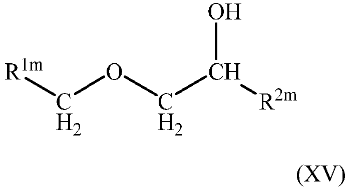 dans lequel :
R^{1m} est un alkyle en C₆ à C₁₂ ; et
R^{2m} est un groupe amino ou hydroxyle ; et
(ii) des composés de formule (XVI) : 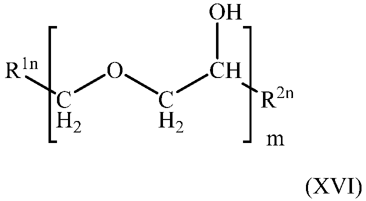 dans lequel :
m est 1 ou 5 ;
R¹ⁿ est un alkyle en C₆ à C₁₂ ;
R²ⁿ est un alkylène R³ⁿ en C₁ à C₆ ; et
R³ⁿ est un groupe hydroxyle ou aminé ;
(y) Copolymère SMDI Bis-méthoxy PEG-13 PEG-438/PPG-110 ; et
(z) des mélanges de ceux-ci.

13. Article absorbant selon l'une quelconque des revendications précédentes, dans lequel le fixateur substantiellement non odorant est choisi dans le groupe des modulateurs de parfum substantiellement non odorants 1 à 97 du tableau 4, et des mélanges de ceux-ci.

14. Article absorbant selon l'une quelconque des revendications précédentes, dans lequel le solvant volatil est un groupe alkyle, alcényle ou alcynyle en C₁ à C₁₀ ramifié ou non ramifié ayant au moins un fragment alcool, de préférence éthanol, isopropanol ou glycol.

15. Article absorbant selon l'une quelconque des revendications précédentes, dans lequel la composition maintient le profil de parfum perçu, en particulier les arômes attribuables à la substance parfumée modérément volatile et/ou à la substance parfumée très volatile, pour rester substantiellement inchangé depuis l'application jusqu'à 2 heures, 3 heures, 4 heures, 5 heures, 6 heures ou 8 heures après l'application, comme déterminé par l'intensité du parfum selon le test olfactif 2(a) tel que décrit dans le présent document.

16. Article absorbant selon l'une quelconque des revendications précédentes, dans lequel le fixateur sensiblement non odorant ne comprend pas :
(i) d'alcool isocétylique, d'éther myristylique de PPG-3, de diéthylhexanoate de néopentyle glycol ou de leurs mélanges ; et
(ii) de n-nonanoate de n-hexadécyle, de n-nonanoate de n-octadécyle ou des mélanges de ceux-ci.
